# EUROPEAN PATENT SPECIFICATION

(11) **EP 2 414 842 B1**
(45) Date of publication and mention of the grant of the patent: **16.05.2018**
(21) Application number: 10721197.1
(22) Date of filing: 01.04.2010
(51) Int. Cl.: C07K 16/40, G01N 33/573

(54) **DIAGNOSIS AND TREATMENT OF GLUTEN-INDUCED AUTOIMMUNE DISEASES**
DIAGNOSE UND BEHANDLUNG GLUTEN-INDUZIERTER AUTOIMMUNKRANKHEITEN
DIAGNOSTIC ET TRAITEMENT DES MALADIES AUTO-IMMUNES INDUITES PAR LE GLUTEN

(30) Priority: 01.04.2009 HU 0900195
(43) Date of publication of application: 08.02.2012
(73) Proprietor: University of Debrecen, 4032 Debrecen (HU)
(72) Inventor: KORPONAY-SZABÓ, Ilma, HU-1054 Budapest (HU); FÉSÜS, László, HU-4032 Debrecen (HU); BAGOSSI, Péter, HU-4032 Debrecen (HU); CSÖSZ, Éva, H-4225 Debrecen (HU); KIRÁLY, Róbert, HU-4032 Debrecen (HU); SIMON-VECSEI, Zsófia, HU-2083 Solymár (HU); MÄKI, Markku, FI-33180 Tampere (FI)
(74) Representative: Svingor, Adam
(86) International application number: PCT/HU2010/000036
(87) International publication number: WO 2010/116196

(56) References cited:
- EP-A1- 1 978 364
- WO-A2-2006/100678
- US-A1- 2006 240 475
- BEGG G E ET AL: "Mutation of a critical arginine in the GTP-binding site of transglutaminase 2 disinhibits intracellular cross-linking activity" JOURNAL OF BIOLOGICAL CHEMISTRY 20060505 AMERICAN SOCIETY FOR BIOCHEMISTRY AND MOLECULAR BIOLOGY INC. US, vol. 281, no. 18, 5 May 2006 (2006-05-05), pages 12603-12609, XP002599935 DOI: DOI:10.1074/JBC.M600146200
- SBLATTERO DANIELE ET AL: "The analysis of the fine specificity of celiac disease antibodies using tissue transglutaminase fragments." EUROPEAN JOURNAL OF BIOCHEMISTRY, vol. 269, no. 21, November 2002 (2002-11), pages 5175-5181, XP002599936 ISSN: 0014-2956
- NAKACHI KEN ET AL: "Epitopes recognised by tissue transglutaminase antibodies in coeliac disease." JOURNAL OF AUTOIMMUNITY, vol. 22, no. 1, February 2004 (2004-02), pages 53-63, XP002600134 ISSN: 0896-8411

### SEQUENCE LISTING

<110> Universty of Debrecen
<120> DIAGNOSIS AND TREATMENT OF GLUTEN-INDUCED AUTOIMMUNE DISEASES
<130> P107216
<160> 20
<170> PatentIn version 3.5
<210> 1
   <211> 43
   <212> DNA
   <213> Artificial Sequence
<220>
   <223> Primer to obtain His-tagged human TG2
<400> 1
   gacgacgaca agatgagaat tcagaccatg gccgaggagc tgg 43
<210> 2
   <211> 45
   <212> DNA
   <213> Artificial Sequence
<220>
   <223> Primer to obtain His-tagged human TG2
<400> 2
   gaggagaagc ccggttgaat tcggttaggc ggggccaatg atgac 45
<210> 3
   <211> 35
   <212> DNA
   <213> Artificial sequence
<220>
   <223> primer for mutagenesis of TG2
<400> 3
   gctggagacc aatggcagcg accaccacac ggccg 35
<210> 4
   <211> 35
   <212> DNA
   <213> Artificial Sequence
<220>
   <223> primer for mutagenesis of TG2
<400> 4
   cggccgtgtg gtggtcgctg ccattggtct ccagc 35
<210> 5
   <211> 34
   <212> DNA
   <213> Artificial Sequence
<220>
   <223> primer for mutagenesis of TG2
<400> 5
   gctgtgtacc tggactcgag cgaggagcgg cagg 34
<210> 6
   <211> 34
   <212> DNA
   <213> Artificial Sequence
<220>
   <223> primer for mutagenesis of TG2
<400> 6
   cctgccgctc ctcgctcgag tccaggtaca cagc 34
<210> 7
   <211> 35
   <212> DNA
   <213> Artificial Sequence
<220>
   <223> primer for mutagenesis of TG2
<400> 7
   cctgctgccg ctccacagcg gcctccacaa gctgg 35
<210> 8
   <211> 35
   <212> DNA
   <213> Artificial Sequence
<220>
   <223> primer for mutagenesis of TG2
<400> 8
   ccagcttgtg gaggccgctg tggagcggca gcagg 35
<210> 9
   <211> 32
   <212> DNA
   <213> Artificial Sequence
<220>
   <223> primer for mutagenesis of TG2
<400> 9
   ggaagaggag cggcagctgt atgtcctcac cc 32
<210> 10
   <211> 32
   <212> DNA
   <213> Artificial Sequence
<220>
   <223> primer for mutagenesis of TG2
<400> 10
   gggtgaggac atacagctgc cgctcctctt cc 32
<210> 11
   <211> 31
   <212> DNA
   <213> Artificial Sequence
<220>
   <223> primer for mutagenesis of TG2
<400> 11
   ggaagaggag cggcagcagt atgtcctcac c 31
<210> 12
   <211> 31
   <212> DNA
   <213> Artificial Sequence
<220>
   <223> primer for mutagenesis of TG2
<400> 12
   ggtgaggaca tactgctgcc gctcctcttc c 31
<210> 13
   <211> 26
   <212> DNA
   <213> Artificial sequence
<220>
   <223> primer for mutagenesis of TG2
<400> 13
   cctggactcg gaaaaggagc ggcagg 26
<210> 14
   <211> 26
   <212> DNA
   <213> Artificial Sequence
<220>
   <223> primer for mutagenesis of TG2
<400> 14
   cctgccgctc cttttccgag tccagg 26
<210> 15
   <211> 49
   <212> DNA
   <213> Artificial Sequence
<220>
   <223> primer for mutagenesis of TG2
<400> 15
   gggtgaggac atactgctgc cgctgctgct gcgagttcag gtacacagc 49
<210> 16
   <211> 49
   <212> DNA
   <213> Artificial Sequence
<220>
   <223> primer for mutagenesis of TG2
<400> 16
   gctgtgtacc tgaactcgca gcagcagcgg cagcagtatg tcctcaccc 49
<210> 17
   <211> 8
   <212> PRT
   <213> Artificial sequence
<220>
   <223> mutated residues 151 to 158 of Site 4
<400> 17
   Asn Ser Gln Gln Gln Arg Gln Gln
   1               5
<210> 18
   <211> 5
   <212> PRT
   <213> Artificial sequence
<220>
   <223> mutated residues 434 to 438 of Site 5
<400> 18
   Asn Gln Arg Gln Asn
   1               5
<210> 19
   <211> 8
   <212> PRT
   <213> Artificial sequence
<220>
   <223> human TG2 site 4 wild type sequence
<400> 19
   Asp Ser Glu Glu Glu Arg Gln Glu
   1               5
<210> 20
   <211> 5
   <212> PRT
   <213> Artificial sequence
<220>
   <223> human TG2 site 5 wild type sequence
<400> 20
   Asp Glu Arg Glu Asp
   1               5

## Description

### FIELD OF THE INVENTION

The invention relates to selective diagnosis of gluten-induced autoimmune diseases by binding assays utilizing the main celiac epitope present on proteins of the transglutaminase family.

The invention also relates to products for the treatment of gluten-induced autoimmune diseases by inhibiting binding of celiac autoantibodies by therapeutic compounds.

### BACKGROUND ART

Celiac disease (also known as celiac disease, non-tropical sprue or gluten-sensitive enteropathy) is a unique autoimmune disorder for which the environmental trigger of the disease is known. The disorder develops among genetically predisposed persons with the human leucocyte antigen (HLA) DQ2 or DQ8 background and is characterized by chronic bowel inflammation, malabsorption and production of autoantibodies as a result of gluten consumption. Gluten also can induce in addition to the enteropathy an itchy and blistering skin rash, and in this case, the disease is called dermatitis herpetiformis. The disease can successfully be treated by elimination of gluten from the patients' diet, but for a sustained response this treatment should be followed life-long.

Celiac disease is precipitated by the ingestion of the gluten fraction of wheat, rye and barley. Gliadin is the alcohol-soluble part of gluten and contains a mixture of peptides that were shown to be toxic for the patients. A 33-mer, α-gliadin-derived peptide was identified as the most immunogenic part of gliadin, which is resistant to gastric, pancreatic and brush-border membrane protease-digestion and can pass bowel epithelium to provoke immune response in the lamina propria [Shan L et al, Science. 2002;297:2275-9.] Chronic inflammatory cell infiltration, villous atrophy and crypt hyperplasia in the upper small intestine are characteristic features of the disease, while it can also cause extraintestinal symptoms such as anemia, neurological disorders or the skin disease called dermatitis herpetiformis. The disease primarily occurs in Western populations where wheat is the basic food (Europe, USA, Australia), but recently it has appeared in the Middle East, South America, North Africa and Asia too. The disease was found in 1.0 % of Finnish schoolchildren [Maki M et al, N Engl J Med. 2003;348(25):2517-24.], in 0.75% of the USA "not-at-risk" groups [Fasano A et al, Arch Intern Med. 2003;163(3):286-92.] and in 1.4% of Hungarian 6 years old children by screening [Korponay-Szabo IR et al, BMJ. 2007;335(7632):1244-7.].

The disease has a wide range of various clinical manifestations such as diarrhea, abdominal distension, anemia, weight loss, retarded growth, skin or neurologic symptoms and can be diagnosed at any age. As a result of the lack of proper absorption, deficiencies of vitamins and minerals, vitamin B₁₂, vitamin K, folic acid, iron and calcium, can occur. Many patients may have only subtle symptoms and in such cases the proper diagnosis is often delayed. Untreated celiac disease has serious complications like adenocarcinoma (with a risk almost twice compared to the general population), T-cell lymphoma (with approximately fifty-fold risk) and osteoporosis.

The current diagnostic criteria of celiac disease were established by the European Society for Pediatric Gastroenterology and Nutrition and require histological examination of a biopsy sample from the upper part of the small bowel (duodenal biopsy) featuring intraepithelial lymphocytosis, crypt hyperplasia and villous atrophy plus a favourable response to a gluten-free diet also should be observed. It also supports the diagnosis of celiac disease if the patient displays in blood certain autoantibodies that can bind to normal tissue sections along the connective tissue sheets surrounding smooth muscle cells (endomysial antibody) or along reticulin fibers (R₁-reticulin antibody binding pattern) [Green PH, Cellier C, N Engl J Med. 2007;357(17):1731-43.].

The disease is heavily underdiagnosed and only two of ten patients are clinically recognized. The main explanation for this is that not all of the patients develop clinical manifestations (silent disease) and some may even have normal mucosal morphology for a long time [Maki M, Collin P, Lancet. 1997;349(9067):1755-9.]. However, they are often positive for reticulin and endomysial tissue autoantibodies with a high density of intraepithelial lymphocytes, or with positive celiac-like intestinal antibody pattern. This subgroup of the disease called latent or potential celiac disease is a diagnostic challenge and these patients may present villous atrophy and crypt hyperplasia in later years or may develop severe - sometimes irreversible - damage in other organs (gluten ataxia, cardiomyopathy, diabetes mellitus, hypothyreosis). The diagnosis in patients with a latent phase is difficult, as they initially often do not fulfill conventional diagnostic criteria and they need a long follow-up time. Antibody levels may fluctuate over time, and presently there are no good tools to exactly predict which subjects will deteriorate in the near future [Simell S et al, Scand J Gastroenterol. 2005;40(10):1182-91.].

Current diagnostic criteria based on the established villous damage in the small intestine are thus insufficient and need reformulation [Kaukinen K et al, Dig Dis Sci. 2001;46:879-87.] In addition, small bowel biopsy is an unpleasant and invasive diagnostic procedure and substitution of biopsy-based diagnostic criteria by antibody positivity would have several practical advantages, and also the possibility to include subjects with early disease and formulate treatment indication in time.

The need for less complicated and better tolerable initial diagnostic tools has long been recognized. Serologic tests based on the detection of circulating antibodies in the patients' blood against gliadin, reticulin, endomysium and type-2 transglutaminase (TG2) are increasingly used. They are also useful for finding patients with mild symptoms or atypical clinical manifestations and for the screening of first degree relatives or risk groups. The main risk groups consist of patients with diabetes mellitus, Down's syndrome, selective IgA deficiency and various autoimmune disorders like autoimmune thyroid disease, Sjögren's syndrome, lupus erythematosus, autoimme liver disease, alopecia, glomerular disease and heart disease which often co-exist with celiac disease [Green PH, Cellier C, N Engl J Med. 2007;357(17):1731-43.].

Antigliadin antibodies do not show enough sensitivity or specificity for celiac disease, despite this, a test based on deamidated gliadin peptides that have structural similarity with TG2 is relatively efficient [Volta U et al, Dig Dis Sci. 2008;53(6):1582-8.; Korponay-Szabo IR et al, J Pediatr Gastroenterol Nutr. 2008;46(3):253-61.]. Monitoring endomysial IgA antibodies using indirect immunfluorescence assays can provide a high accuracy, since these antibodies are highly specific markers of the disease. However, evaluation of the endomysial antibody test is largely observer-dependent and requires highly skilled and trained personnel and thus it is not widely available in each medical setting. Selective IgA deficiency is a common feature in celiac patients (1 in 40 cases) which makes very difficult to recognize patients by the conventional endomysial antibody test. In these cases, IgG class anti-TG2 autoantibodies should be detected [Korponay-Szabo I et al, Gut. 2003;52(11):1567-71]. Further, the endomysial test cannot diffentiate between the overt and latent forms of the disease [Green PH, Cellier C, N Engl J Med. 2007;357(17):1731-43.]. Thus, there was a need in the art for reliable and more easy-to-use tests.

TG2 (also known as tissue, erythrocyte or cellular transglutaminase) was found to be the main self autoantigen and a target for endomysial and reticulin antibodies in celiac disease. The enzyme is a member of the transglutaminase superfamily, which enzymes catalyze acyl-transfer reaction between γ-carboxamide group of a glutamine and the ε-amino group of a lysine residue -covalent protein crosslinking- in a Ca²⁺-dependent manner (EC 2.3.2.13). Besides this TG2 can catalyze incorporation of amines into proteins, site-specific deamidation, it has isopeptidase activity and takes part in transmembrane signaling via its GTPase activity. TG2 can externalize from the cells where it is complexed with integrins and fibronectin, promotes the remodelling and assembly of the extracellular matrix and mediates cell-matrix interactions. In this way TG2 takes part in wound healing and angiogenesis. The enzyme can translocate to the nucleus and with crosslinking of histones and retinoblastoma proteins it also can regulate gene expression. TG2 is induced and activated during apoptosis; moreover the lack of the enzyme affects the engulfment of apoptotic cells by macrophages [Fesus L, Piacentini M, Trends Biochem Sci. 2002;27(10):534-9.].

TG2 is a ubiquitous enzyme, it can be found all over the body, intra- and also extracellularly. After the identification of the main autoantigen TG2, numerous enzyme-linked immunoassays (ELISA) were developed with guinea pig liver TG2 or recombinant human TG2 as antigen [Schuppan D et al, 1998; WO 98/03872.; Powell M et al, 2002; WO 02/068616 A2.]. Further, extracellular matrix rich in TG2 produced by cell lines can also be used as antigen in diagnostic tests. These assays are less expensive than endomysial antibody tests and their sensitivity and specificity is greater than 90% in the case of human TG2 antigen. Moreover, efficient rapid antibody detection was described by utilizing the natural self TG2 in the red blood cells of patient blood samples [Maki M et al, 2002; WO 02/086509].

In some patients, and particularly in those with dermatitis herpetiformis, antibodies against a homologous skin transglutaminase protein (TG3) were also described, that could be used as well for diagnostic purposes (Paulsson M. et al., 2001, WO 01/001133).

Hadjivassiliou et al. and Aeschlimann, D. et al. [Hadjivassiliou et al, Ann Neurol. 2008;64:332-43 and Aeschlimann, D. et al. EP1978364A1, respectively] suggested that antibodies against transglutaminase 6 (TG6) can serve as a marker of a gluten induced autoimmune disease, in addition to human leukocyte antigen type and detection of anti-gliadin and anti-transglutaminase 2 antibodies, to identify a subgroup of patients with gluten sensitivity who may be at risk for development of neurological disease. They observed that TG6 IgG and IgA response is prevalent in gluten ataxia, independent of intestinal involvement.

In fact, several studies based on TG2-based tests of the art described false negative IgA TG2 results with IgA endomysium antibody positivity and false positive IgA TG2 results in the absence of IgA endomysium positivity [Wong RC et al, J Clin Pathol. 2002;55(7):488-94.]. False positive TG2 antibody positive results are relatively common in the clinical settings [Lock RJ et al, Eur J Gastroenterol Hepatol. 2004;16(5):467-70.; Green PH, Cellier C, N Engl J Med. 2007;357(17):1731-43.] and this severely restricts the use of TG2 antibody positivity as the sole diagnostic test. Particularly, patients with other autoimmune diseases, tumors, cardiac failure, neurological disorders, psoriasis and liver diseases may exhibit low levels of antibodies reacting with TG2. This is not surprising, because these tissues contain relatively high amounts of TG2 which can be liberated by any kind of tissue damage that can induce non-specific autoantibodies against TG2 and this antibody positivity may be irrelated to celiac disease.

In contrast, celiac-specific antibodies are induced in response to gluten probably by a hapten-carrier mechanism [Sollid LM et al, Gut. 1997;41(6):851-2.], and recognize TG2 in the form as it is bound to the surface of fibronectin in muscular tissue section substrates used for the endomysial antibody detection by immunofluorescence. However, even the endomysial antibody test cannot distinguish between anti-TG2 antibodies of celiac patients and other TG2 antibodies experimentally induced in mice [Korponay-Szabo IR et al, J Pediatr Gastroenterol Nutr. 2008;46(3):253-61.]. Further, in an attempt to produce more celiac-specific diagnostic tests, TG2 complexed with gliadin peptides was also used as antigen, but this gave even less reliable results than TG2 alone [Rajadhyaksha M et al, WO 01/29090 A1].

Koshla C. et al. in US2006/240475 have found that the multivalent gluten oligopeptides are resistant to cleavage by gastric and pancreatic enzymes and the presence of these pepetides is responsible to toxic effects mediated by antibodies and T-cell proliferation in the celiac disease. Based on their finding Koshla et al. describe methods for diagnosing celiac sprue by detecting certain multivalent toxic gluten oligopeptides or T cell proliferation elicited by such oligopeptides in a patient.

A verification immunoassay differently recognizing antibodies directed against the celiac and other TG2 epitopes could be exquisitely important for the simplification of the diagnostic process in future. Thus, there is still a need to develop a reliable and relatively convenient diagnostic method which is specific for a gluten-induced autoimmune disease or preferably, celiac disease. In particular, there is still a need in the art to develop a diagnostic assay which could identify latent form of the disease, and/or which is applicable in children or in cases when symptoms do not provide a decisive answer. Moreover, replacing diagnosis based on jejunal biopsy would also be desirable in terms of cost and patient comfort.

In fact, several efforts have been made in the art to identify the main binding epitopes of the celiac autoantibodies. However, no such unique epitope has been found so far and it is also not clear whether only one or several such epitopes exist. Protein engineering methods provide a well-known tool to explore binding properties of both ligands and antibodies to TG2. For example, Begg G E et al. have found that Arg579 is particularly important for GTP binding, as isothermal titration calorimetry demonstrated a 100-fold reduction in GTPbinding affinity by the R579A mutant. Previous studies [Seissler J et al, Clin Exp Immunol. 2001;125(2):216-21.; Sblattero D et al, Eur J Biochem. 2002;269(21):5175-81.; Nakachi K et al, J Autoimmun. 2004;22(1):53-63.], in which TG2 fragments were applied, indicated that the C-terminal domain may harbor important binding sites. However, some patient samples also recognized the N-terminal or the core domain, so that it was concluded that the antibody response may be dispersed and variable according to the subjects. Further, a difference was noted between young female celiac patients and those with other clinical manifestations [Tiberti C et al, Clin Immunol. 2003;109(3):318-24.]. In another study [Byrne G et al, Gut. 2007;56(3):336-41.], the catalytic triad was suggested as the main binding site, as mutation of these three amino acids resulted in a protein to which patient antibodies bound poorly. Again in this study, the specificity of IgA and IgG class celiac antibodies seemed to be different. However, these studies did not take into account the three-dimensional structure of the protein which might severely be deranged by gross deletions, particularly by those affecting the core domain or the catalytic triad. Further studies conducted by TG2 fragments displayed on the surface of phages also indicated that the conformation of the protein might have a high importance to form a functional binding site for the celiac antibodies, as it was not possible to identify the epitopes of celiac patient derived monoclonal antibodies by these means either [Di Niro R et al, Biochem J. 2005;388(3):889-94.].

TG2 is not only a main autoantigen in celiac diease and thus an important target molecule in the diagnosis, but it is also involved in the pathogenesis of the disease.

Increased TG2 expression and activity were detected in the duodenal biopsies of celiac patients compared to healthy donors and gliadin peptides were found to be good substrates for TG2. According to the present hypothesis, gliadin-TG2 complexes can bind to TG2-specific B cells, where both gliadin and TG2 fragments can be presented on DQ2. The presented gliadin-DQ2 complexes can activate the gliadin-specific T-cells, which can provide help for the TG2-specific B cells to produce anti-TG2 antibodies by a hapten-carrier mechanism [Sollid LM et al, Gut. 1997;41(6):851-2.]. Celiac disease is conventionally regarded as a mainly T-cell mediated disorder where the role of celiac antibodies is debated.

Interestingly, celiac antibodies do not block the cross-linking activity of TG2 [Dieterich W et al, Gut. 2003;52(11):1562-6.; Roth EB et al, Clin Exp Rheumatol. 2006;24(1):12-8.], instead, they may even enhance the transamidation and deamidation processes presumably by stabilising the enzyme's conformation [Kiraly R et al, J Autoimmun. 2006;26(4):278-87.]. In this way, TG2 will process more gliadin, which could enhance the immune response ending in more and more antibody production and driving the disease process further. Proposals in the art that the epitope is conformational and multiple domains are involved apparently contradict this finding.

Several lines of new evidence suggest that autoantibodies against TG2 also do have pathogenic role in the development of the disease. Presence of these antibodies is a uniform feature in all patients and if not detectable in the circulation, these antibodies can be found deposited in various tissues. Tissues damage has been related to the *in vivo* binding of antibodies and antibodies bound to extracellular TG2 were found in situ in diseased organs like small bowel, liver, muscles, kidney, brain etc also in the absence of positivity for the antibodies in serum samples [Korponay-Szabo IR et al, Gut. 2004;53(5):641-8.]. These antibodies are functional as they can bind externally added recombinant TG2 and they appear in tissues already before the villous atrophy develops [Salmi TT et al, Gut. 2006;55(12):1746-53.; Salmi TT et al, Aliment Pharmacol Ther. 2006;24(3):541-52.].

Further, celiac antibodies were shown to disturb epithelial cell differentiation and angiogenesis in cellular models [Myrsky E et al, Clin Exp Immunol. 2008;152(1):111-9.], as well as induced apoptosis via mitochondrial pathways [Cervio E et al, Gastroenterology. 2007;133(1):195-206.]. Antibodies purified with a TG2 homologue peptide increased epithelial cell permeability and induced monocyte activation [Zanoni G et al, PLoS Med. 2006;3(9):e358.].

Identification of an important celiac epitope, if any, in transglutaminases which are autoantigens in celiac disease could also be important to see whether specific blocking of the antibody binding affects the pathological processes during the disease development, and thus interference with patient antibody binding at the level of epitopes would offer a new therapeutic approach. Prevention of antibody-antigen binding might alleviate both the effects based on direct protein-protein interactions and also those related to the alterations in enzyme activity.

The present inventors unexpectedly found that a main celiac epitope can be localized on transglutaminases which function as autoantigens in gluten induced autoimmune diseases.

Surprisingly, improved diagnostic methods can be provided based on engineered transglutaminases having a folded beta-sandwich and core domains, by utilizing binding pattern of celiac autoantibodies and thereby eliminating false positive results. Moreover, further improved diagnostic method can be provided by using compounds specifically binding to the main celiac epitope and being capable of displacing or displaced by celiac patient autoantibodies.

The present inventors also found that it is possible to specifically inhibit the binding of autoantibodies to TG2 and its downstream biological effects allowing promising diagnosis and treatment options as well as enabling the skilled person to design interfering compounds in a targeted way.

### BRIEF DESCRIPTION OF THE INVENTION

The invention is defined by the claims. According to a first aspect the invention relates to a diagnostic method for diagnosis of a gluten-induced autoimmune disease in a subject comprising the steps of
i) providing a biological sample taken from a subject, said sample containing autoantibodies of said subject, and optionally isolating autoantibodies from said sample,
ii) contacting the autoantibodies of said sample with
   - a reference protein belonging to the transglutaminase family (a TG family protein) and having
      a) a part of the molecular surface of a TG family protein to which Mab885 is capable of binding, said epitope including a surface area within 7 Å from the area covered by Mab885 when bound, or
      b) a part of the molecular surface of a TG family protein, to which a celiac autoantibody of a subject with celiac disease is capable of binding, in a manner that said autoantibody can be displaced in a competitive assay by Mab 885, or
      c) a part of the molecular surface of a TG family protein which may be an autoantigen in celiac disease, at least partially formed by amino acid residue corresponding/equivalent to Glu153 and/or Arg19 numbered according to the amino acid numbering of the full length human TG2 based on multiple sequence alignment
      d) a part of a molecular surface of a TG family protein, which is capable of binding a surface recognition molecule, wherein said surface recognition molecule is capable of binding to a part of the molecular surface as defined in a) to c)
      said part forming an intact main celiac epitope,
   - at least one test protein belonging to the transglutaminase family, in which the side chain and/or spatial position of at least one surface amino acid residue contributing to the main celiac epitope is altered as compared to the reference protein in which the main celiac epitope is intact,
   wherein the said at least one surface amino acid residue the side chain and/or spatial position of which is altered is selected from
   an amino acid residue corresponding to or equivalent to Glu153 numbered according to the amino acid numbering of the full length human TG2 based on amino acid sequence alignment, and/or
   an amino acid residue corresponding to or equivalent to Arg19 numbered according to the amino acid numbering of the full length human TG2 based on amino acid sequence alignment,
   and wherein the core domain has a folded three dimensional structure, preferably a native folded three dimensional structure, and the beta-sandwich domain has a folded three dimensional structure, preferably a native folded three dimensional structure, preferably the fold of these domains is essentially integral or is maintained,
iii) assessing a binding property of the autoantibodies to the reference protein and to the at least one test protein,
wherein if the binding of autoantibodies to the test protein is impaired as compared to the reference protein, this fact is considered as indicative of a gluten-induced autoimmune disease in said subject. Preferably, when a side chain is altered, the side chain geometry, side chain size, side chain functional group or charge thereof is altered.

Preferably the binding property is assessed by assessing the binding level or by assessing binding kinetic rates or parameters, e.g. those of association and/or dissociation.

In a preferred embodiment in step iii) of the diagnostic method the binding is assessed by measuring binding level of the autoantibodies to the test protein and to the reference protein, wherein if the binding level of the autoantibodies to the reference protein exceeds a predetermined threshold value and if the binding level of the autoantibodies to the test protein is significantly lower than to the reference protein or preferably reduced by at least 30%, preferably at least 40%, more preferably at least 50% as compared to the reference protein, this fact is considered as indicative of a gluten-induced autoimmune disease in said subject.

Setting a predetermined threshold (or cut-off) is a routine optimisation process and is well within the skills of a person skilled in the art of setting assay conditions. A suitable cut-off for declaring binding molecules, e.g. antibodies as reactive with a TG family protein, e.g. a reference TG family protein can be established for example by a receiver operating characteristic curve (ROC) performed with known celiac and non-celiac samples, as well known in the art.

In an embodiment, the predetermined threshold is set to 1%, 2%, 3%, 4%, 5%, 6%, 7%, 8%, 9%, 10%, 11%, 12%, 13%, 15%,16%, 17%, 18%, 19% or 20% of the maximal reference value, e.g. of the level of 100% binding.

In a preferred embodiment, any one of the test protein and the reference protein is a wild type protein or is prepared by protein engineering from an appropriate scaffold which is selected from an animal TG1, TG2, TG3, TG4, TG5, TG6, TG7, factor XIII, or EBP42, wherein preferably a) the test protein is a mutant transglutaminase (TG), preferably a mutant TG2, TG3 or TG6, and b) the reference protein is a wild type TG, preferably a wild type TG2, TG3 or TG6.

It is disclosed herein that the first alpha helix of the beta-sandwich domain spans from an amino acid residue which is in eight amino acid distance in the N-terminal direction from the first His of the HisHisThr motif of the beta sandwich domain to said first His of the HisHisThr motif of the beta sandwich domain.

It is also disclosed herein that the last five amino acids of the first alpha helix of the beta-sandwich domain spans from the conserved Asn of the first alpha helix of the beta-sandwich domain to the first His of the conserved HisHisThr motif of the beta-sandwich domain and preferably the last six, five, four or three amino acid residues of said helix are calculated accordingly.

In a preferred embodiment the sixth amino acid residue of said helix is the second amino acid in amino terminal direction from the conserved HisHisThr motif of the beta-sandwich domain, or the second amino acid in carboxy terminal direction from the conserved Asn of the first alpha helix of the b-sandwich domain.

In a preferred embodiment the sixth amino acid of the first alpha helix of the beta-sandwich domain is Arg 19 in human TG2 or a corresponding amino acid in the sequence of a protein of the transglutaminsase family having a folded beta-sandwich domain and a folded core domain; and the first His of the HisHisThr motif is His22 in human TG2 or a corresponding amino acid in the sequence of a protein of the transglutaminsase family having a folded beta-sandwich domain and a folded core domain.

In a preferred embodiment the first alpha helix of the core domain spans
- from the fifth amino acid in amino terminal direction from the conserved GluTyrXxx motif (wherein Xxx is an apolar amino acid residue, preferably Val or Ile or Leu) of the first alpha helix of the core domain or
- from the third amino acid in amino terminal direction from the conserved Arg of the first alpha helix of the core domain of human transglutaminases
- to at least the Glu or Tyr of said GluTyrXxx motif.

Thus, the first or the first two, three or four amino acid residues of the first alpha helix of the core domain is/are calculated accordingly.

In a preferred embodiment, the first amino acid residue of said alpha helix is the fifth amino acid in amino terminal direction from the conserved GluTyrXxx motif of the first alpha helix of the core domain or the third amino acid in amino terminal direction from the conserved Arg of the first alpha helix of the core domain of the majority of human transglutaminases.

In a preferred embodiment the spatial position or the side chain geometry of
a) at least a surface amino acid residue of the first alpha helix of the core domain, preferably a surface amino acid residue selected from the first four, three, or two amino acid residues of said alpha helix, more preferably the first amino acid residue of said alpha helix, and
b) at least a surface amino acid residue of the first alpha helix of the beta-sandwich domain and the conserved HisHisThr motif of the beta sandwich domain, preferably a surface amino acid residue selected from the last six, five, four or three amino acid residues and the HisHisThr motif, more preferably the sixth amino acid residue of said helix,
are altered relative to each other.

In a further embodiment a mutation is carried out in a part of the protein of the transglutaminase family which results in the dislocation of or an altered spatial position of any of the amino acids defined herein as an amino acid contributing to the main celiac epitope or an SSE carrying said protein. In certain embodiments larger parts of the alpha helices are dislocated, e.g. the alpha helices are disrupted or tilted.

In a preferred embodiment of the diagnostic method of the present invention preferably
the first amino acid residue of said first alpha helix of the core domain is an amino acid residue corresponding to or equivalent to Glu153 numbered according to the amino acid numbering of the full length human TG2 based on amino acid sequence alignment, and/or
the sixth amino acid residue(s) of said first alpha helix of the beta-sandwich domain is an amino acid residue corresponding to or equivalent to Arg19 numbered according to the amino acid numbering of the full length human TG2 based on amino acid sequence alignment, and/or
the first amino acid of the HisHisThr motif said first alpha helix of the beta-sandwich domain is an amino acid residue corresponding to or equivalent to His22 numbered according to the amino acid numbering of the full length human TG2 based on amino acid sequence alignment.

In a preferred embodiment of the diagnostic method according to the present invention in said TG family test protein the side chain or spatial position of at least one further amino acid is altered, e.g. one or more further amino acid is mutated, as compared to the reference protein in which the main celiac epitope is intact,
wherein preferably said further amino acid is selected from the group of amino acid residues corresponding to or equivalent to Arg 151, Glu 153, Glu 154, Arg156, Arg 19, His22, Val431, Arg433, Glu435, Met659, Leu661 numbered according to the amino acid numbering of the full length human TG2 based on multiple sequence alignment and wherein preferably said alteration effects celiac antibody binding.

In a further preferred embodiment the spatial position of any of the following amino acid residues is altered due to the alteration of one or more further amino acid as compared to the reference protein:
a surface amino acid residue selected from the first four, three, or two amino acid residues of said alpha helix, more preferably the first amino acid residue of said alpha helix, and/or
a surface amino acid residue selected from the last six, five, four or three amino acid residues of said first alpha helix of the beta-sandwich domain and amino acid residues of the HisHisThr motif, more preferably the sixth amino acid residue of said helix and/or the first amino acid of said HisHisThr motif.

In a preferred variant of this embodiment the said further alteration is a mutation as compared to the reference protein in one or more amino acid residues selected from the group of amino acid residues corresponding to or equivalent to the following amino acids of the full length human TG2 based on multiple sequence alignment: Glu158, Tyr160, Val161, His23, Thr24, or an alteration in the N-terminal portion of the beta-sandwich domain, e.g. a deletion or substitution of at least 1, 2, 3, 4, 5, 6, 7, 8, 9, 10, 11, 12, 13, 14, 15, 16, amino acid residues calculated from the sixth residuee of the first alpha helix of the beta-sandwich domain.

Preferably, the test protein is a mutant TG2 and the alteration of said one or more amino acids is a mutation selected from the following group:
a mutation of
Glu153, e.g. E153S, E153T, E153Y, E153K, E153R, E153Q, E153N,
Arg19, e.g. R19S, R19K, R19Q
Glu154, e.g. E154S, E154T, E154Y, E154K, E154R, E154Q, E154N,
Glu158, e.g. E158S, E158Q, E158L,
His22, e.g. H22S
and as further mutations:
   Ala24, Arg 151, Arg156, Val431, Val432, Arg433, Glu435, Met659, Leu661

A skilled person will understand that if the effect of the mutation or alteration in the side chain is small, e.g. if a conservative substitution is made, it may be expedient to have more than one amino acid residues mutated.

In a further embodiment of the present invention the autoantibodies or a panel of autoantibodies are(is) isolated before contacting them with the test protein and, if desired, with the reference protein. In a further aspect the invention relates to a diagnostic kit for the diagnosis of a gluten-induced autoimmune disease in a subject comprising
a) a test protein belonging to the transglutaminase family, in which the side chain and/or spatial position of at least one surface amino acid residue contributing to the main celiac epitope is altered as compared to the reference protein in which the main celiac epitope is intact,
   wherein the said at least one surface amino acid residue the side chain and/or spatial position of which is altered is selected from
   an amino acid residue corresponding to or equivalent to Glu153 numbered according to the amino acid numbering of the full length human TG2 based on amino acid sequence alignment, and/or
   an amino acid residue corresponding to or equivalent to Arg19 numbered according to the amino acid numbering of the full length human TG2 based on amino acid sequence alignment,
   and wherein the core domain has a folded three dimensional structure, preferably a native folded three dimensional structure, and
b) a reference protein belonging to the transglutaminase family, said protein having an intact main celiac epitope or at least a medium carrying instructions for providing and using said reference protein belonging to the transglutaminase family and having
   a) a part of the molecular surface of a TG family protein to which Mab885 is capable of binding, said epitope including a surface area within 7 Å from the area covered by Mab885 when bound, or
   b) a part of the molecular surface of a TG family protein, to which a celiac autoantibody of a subject with celiac disease is capable of binding, in a manner that said autoantibody can be displaced in a competitive assay by Mab 885, or
   c) a part of the molecular surface of a TG family protein which may be an autoantigen in celiac disease, at least partially formed by amino acid residue corresponding/equivalent to Glu153 and/or Arg19 numbered according to the amino acid numbering of the full length human TG2 based on multiple sequence alignment
   d) a part of a molecular surface of a TG family protein, which is capable of binding a surface recognition molecule, wherein said surface recognition molecule is capable of binding to a part of the molecular surface as defined in a) to c),
   said part forming an intact main celiac epitope, wherein the core domain of said reference protein has a folded three dimensional structure, and optionally
   means for taking a biological sample from a subject, said sample containing autoantibodies of said subject and/or means for isolating autoantibodies from said sample, and/or means for assessing the level of binding and/or kinetics of the autoantibodies to the proteins.

In the diagnostic kit of the invention any test protein and/or reference protein as defined herein can be applied.

In a preferred embodiment
a) the test protein is a mutant transglutaminase (TG), preferably a mutant TG2, TG3 or TG6, and
b) the reference protein is a wild type TG, preferably a wild type TG2, TG3 or TG6.

In a preferred embodiment the means for assessing the level of binding comprises a plate e.g. a microtiter plate having wells wherein a first part of the wells are coated with the reference protein and a second part of the wells are coated with the at least one test protein.

In an embodiment the wells of the plate are also coated with fibronectin.

In a further aspect the invention relates to a diagnostic method for the diagnosis of a gluten-induced autoimmune disease in a subject comprising the steps of
i) providing a biological sample taken from said subject, said sample containing autoantibodies of said subject and optionally isolating autoantibodies from said sample,
ii) contacting autoantibodies of said sample with the reference protein belonging to the transglutaminase family, said protein having an intact main celiac epitope as defined above and
iii) assessing the level of binding of the autoantibodies to the reference protein both in the absence of and in the presence of a test compound, said test compound being a test antibody or fragment, variant, derivative or analogue thereof known to be capable of binding to the main celiac epitope as defined above,
wherein if the binding level of the autoantibodies in the absence of the test compound exceeds a predetermined threshold value and if the binding level of the autoantibodies to the reference protein in the presence of the test compound is significantly lower as compared to the binding level of the autoantibodies in the absence of said test antibody, this fact is considered as indicative of a gluten-induced autoimmune diseases in said subject. Preferably the binding level of the autoantibodies to the reference protein in the presence of the test compound is reduced by at least a predetermined ratio, preferably by at least at least 40%, preferably at least 50%, more preferably at least 60%, 70% or 80%, corresponding to a binding level wherein the remaining binding is not higher than 60%, preferably not higher than 50%, more preferably not higher than 40%, 30% or 20%, respectively.

The test compound, therefore, shall be a compound which is capable of binding to the main celiac epitope with an affinity or avidity high enough to replace, at least partly, patient celiac antibodies or vice versa. The test compound should bind the main celiac epitope with a binding affinity or avidity sufficiently high to compete with patient autoantibodies, preferably with a binding affinity or avidity higher than that of the patient antibodies. A skilled person will understand that even if the binding affinity or avidity of the test compound is comparable with or lower than that of the patient autoantibodies, a sufficient degree of replacement can be achieved by using a higher concentration of the test compound.

In an alternative embodiment of the diagnostic method displacement of the test compound by celiac patient antibodies is detected.

Specifically, the invention relates to a diagnostic method for the diagnosis of a gluten-induced autoimmune disease in a subject comprising the steps of
i) providing a biological sample taken from a subject,
ii) contacting a test compound with a reference protein belonging to the transglutaminase family as defined in claim 1, said protein having an intact main celiac epitope as defined in claim 1 and said test compound being a test antibody or fragment, variant, derivative or analogue known to be capable of binding to the main celiac epitope,
iii) assessing the level of binding of the test compound to the reference protein both in the absence of and in the presence of said biological sample,
wherein if the mean binding level of the test compound to the reference protein in the presence of the sample is lower than the binding level in the absence of the sample, this fact is indicative of the presence of autoantibodies capable of binding to the main celiac epitope and of a gluten-induced autoimmune disease in said subject.

Setting the concentration to achieve an appropriate replacement of the autoantibodies by the test compound is well within the skills of a person skilled in the art.

A preferred test compound is an antibody or antibody fragment highly specific for the main celiac epitope. A particularly preferred test compound is a monoclonal antibody raised against said epitope. A preferred example is Mab885 of Phadia deposited on March 25, 2010 according to the Budapest Treaty by Phadia AB (P.O.Box 6460 751 37 UPPSALA, Sweden, Visiting address: Rapsgatan 7P 754 50 Uppsala) under Accession number DSM ACC3053 at DSMZ - Deutsche Sammlung von Miroorganismen un Zellkulturen GmbH, Inhoffenstr. 7 B, D-38124 Braunschweig, Germany as indicated in the appropriate international form.

In the preferred embodiment the test compound is a test antibody or fragment, variant, derivative or analogue thereof known to be capable of binding to the main celiac epitope of a protein belonging to the transglutaminase family, said protein being an autoantigen in celiac disease, preferably TG2, TG3 or TG6, more preferably TG2 or TG6, highly preferably TG2.

In the preferred embodiment the test compound is a fragment, variant, derivative or analogue of the Mab885 comprising a binding region having the amino acid sequence of a variable region of Mab885 or an amino acid sequence having at least 60%, 70%, 80%, 90% 95%, 98% or 99% sequence identity therewith, said a fragment, variant, derivative or analogue being capable of binding to the main celiac epitope of a protein belonging to the transglutaminase family.

Expediently, the biological sample taken from said subject is a sample of a tissue or body part, either in native or processed state, wherein celiac autoantibodies can reside. In a preferred embodiment of the invention the biological sample taken from said subject is a body fluid sample, e.g.in saliva, duodenal juice, stool or a blood sample, or a processed sample thereof, e.g. a serum sample or a sample containing appropriate inhibitors.

In further embodiments the biological sample is a solid tissue sample wherein celiac autoantibodies are deposited e.g. preferably a sample taken from small intestine, jejunum, placenta or liver [Korponay-Szabo, I.R. et al. Gut 53, 641-648 (2004)]. The sample can be taken from the skin wherein the presence of anti TG3 antibodies are reported.

The advantage of using a body fluid sample is that patient autoantibodies are present in the sample in a directly applicable form and no isolation thereof is necessary. Preferably, from solid tissue samples patient autoantibodies are to be obtained by at least partial isolation or elution of the autoantibodies from the sample as described in Salmi TT et al, Gut. 2006;55(12):1746-53.].

The invention also provides for the use of a test compound as disclosed herein, said compound specifically binding to the main celiac epitope of a reference protein belonging to the transglutaminase family as defined above, for diagnosis of celiac disease or in a method for diagnosis of celiac disease in a displacement assay, said test compound being a test antibody or antibody fragment, variant or analogue known to be capable of binding to the main celiac epitope said reference protein being an autoantigen in celiac disease.

In the use of the present invention patient antibodies and the test compound of the invention both bind to the main celiac epitope and thereby compete for the binding site. Assay conditions can be set either in such way that celiac patient autoantibodies replace the test compound or alternatively in such a way that the test compound replaces the celiac patient autoantibodies.

According to a further aspect of the invention a further diagnostic kit for the diagnosis of a gluten-induced autoimmune disease in a subject is provided, said kit comprising
- a reference protein belonging to the transglutaminase family and having
   a) a part of the molecular surface of a TG family protein to which Mab885 is capable of binding, said epitope including a surface area within 7 Å from the area covered by Mab885 when bound, or
   b) a part of the molecular surface of a TG family protein, to which a celiac autoantibody of a subject with celiac disease is capable of binding, in a manner that said autoantibody can be displaced in a competitive assay by Mab 885, or
   c) a part of the molecular surface of a TG family protein which may be an autoantigen sin celiac disease, at least partially formed by amino acid residue corresponding/equivalent to Glu153 and/or Arg19 numbered according to the amino acid numbering of the full length human TG2 based on multiple sequence alignment
   d) a part of a molecular surface of a TG family protein, which is capable of binding a surface recognition molecule, wherein said surface recognition molecule is capable of binding to a part of the molecular surface as defined in a) to c),
   said part forming an intact main celiac epitope,, wherein the core domain of said reference protein has a folded three dimensional structure, and
- a test compound known to be capable of binding to the main celiac epitope of said reference protein belonging to the transglutaminase family, said main celiac epitope wherein
   either the test compound is labelled or
   the kit also comprises a labelled compound specifically binding to the test compound
   to detect binding of the test compound to the reference protein,
   and optionally
   means for taking a biological sample from a subject, said sample containing autoantibodies of said subject and/or means for isolating autoantibodies from said sample.

Preferably the test compound is a test antibody or fragment, variant or analogue thereof known to be capable of binding to the main celiac epitope of a protein belonging to the transglutaminase family, said protein being an autoantigen in celiac disease. More preferably the test antibody is a celiac patient derived antibody or a fragment, a variant or an analogue thereof having the binding site and/or the recognition pattern typical of a patient derived antibody.

Preferably, the test antibody is a monoclonal antibody or fragment, variant or analogue thereof having a binding site of the same binding properties. In a highly preferred embodiment the monoclonal antibody is an antibody capable of binding to the surface part of the first one to four amino acids of the first alpha helix of the core domain. Preferably, the antibody is the Mab 885 monoclonal antibody. In a further aspect the invention relates to a use of a test protein in a method for diagnosis of a gluten induced autoimmune disease,
said test protein belonging to the transglutaminase family, in which the side chain and/or spatial position of at least one surface amino acid residue contributing to the main celiac epitope is altered as compared to the reference protein in which the main celiac epitope is intact, as defined above. Preferably, in the use of the present invention, the test protein and the reference protein is any test protein and reference protein, respectively, as defined herein.

Preferably, in the use of the present invention the method for diagnosis of celiac disease is any diagnostic method as described herein.

For example, in the use, diagnostic method or test kit according to the invention
the test protein is a mutant of a wild type protein belonging to the transglutaminase family and being an autoantigen in celiac disease, and
the reference protein is wild type protein belonging to the transglutaminase family which is an autoantigen in celiac disease.

Preferably, the reference protein is a TG2, TG3 or TG6 comprising wild type amino-terminal beta-sandwich and core domains, and the test protein is a TG2, TG3 or TG6 comprising mutant beta-sandwich and core domains.

The present inventors also disclose a preparation method for preparing a test protein belonging to the transglutaminase family useful in a diagnostic method for diagnosing celiac disease in a subject, comprising the steps of
i) selecting a reference protein belonging to the transglutaminase family in which the celiac epitope is intact,
ii) designing a mutant variant of said protein wherein the side chain and/or spatial position of at least one surface amino acid residue contributing to the main celiac epitope is altered as compared to the reference protein in which the main celiac epitope is intact,
   said at least one surface amino acid residue the side chain and/or spatial position of which is altered is selected from
   an amino acid residue corresponding to or equivalent to Glu153 numbered according to the amino acid numbering of the full length human TG2 based on amino acid sequence alignment, and/or
   an amino acid residue corresponding to or equivalent to Arg19 numbered according to the amino acid numbering of the full length human TG2 based on amino acid sequence alignment
   and wherein the core domain has a folded three dimensional structure, preferably a native folded three dimensional structure
iii) preparing a mutant recombinant nucleic acid coding for a mutant amino acid sequence comprising said mutation,
iv) expressing said mutant recombinant nucleic acid in a host,
v) obtaining said mutant protein from said host to provide a test protein which, upon contacting it with a celiac antibody shows reduced binding level (preferably at least 30% lower, more preferably at least 40% lower, even more preferably at least 50% lower level of binding) as compared to the corresponding wild type reference protein.

In a further embodiment the invention relates to a preparation method for preparing a reference protein belonging to the transglutaminase family useful in a diagnostic method for diagnosing celiac disease in a subject.

In a further aspect of the invention discloses a method for the preparation of a therapeutic compound which is an inhibitor of the binding of a celiac autoantibody to a celiac epitope present on a TG family protein as defined herein, preferably on TG2 and/or TG6 of a patient, said method comprising the steps of:
i) providing a set of compounds having a variable binding region and being capable of binding, via their binding region, to a celiac epitope,
ii) selecting a subset of compounds by a differential binding assay, said compounds in said subset being capable of binding only to a part of the celiac epitope as compared to patient celiac autoantibodies, and thereby being capable of displacing an thereby antagonizing said celiac autoantibodies
iii) selecting a compound from said subset of compounds which is lack of an adverse effect of a celiac autoantibody, preferably
   - which affects or alters the transglutaminase activity by less than ±50%, preferably less than ±40%, more preferably by less than ±30% or ±20% of the activity of said active protein.
   - which affects or alters the GTPase activity by less than ±50%, preferably less than ±40%, more preferably by less than ±30% or ±20% of the activity of said active protein.
   - which is capable of reversing or antagonizing an adverse effect of a celiac autoantibody on cells of an endothelial, epithelial, fibroblast, muscle or nerve cell culture or tissue culture, preferably on HUVEC cells.

Preferably, the compound does not increase significantly the activity of the transglutaminase family protein (as celiac antibodies do). Said activity is selected from transglutaminase activity and GTPase activity. Activity is significantly increased when said increase is large enough so as not to be attributed to usual variation in experimental conditions.

In an embodiment the therapeutic compound is a monoclonal antibody, including any fragment thereof comprising a binding region, e.g. single chain variable fragment (scFv) or an Fab fragment.

In an embodiment the set of monoclonal antibodies is prepared by the hybridoma technology.

In a further embodiment the set of monoclonal antibodies is prepared by a display methodology, e.g. the phage display methodology as dislosed hereinbelow. In a further preferred embodiment the antibody is of a non-human origin, e.g. a mammalian origin, e.g. a rodent origin, preferably a rabbit, mouse or rat origin, and said antibody is prepared by immunization and subsequent screening. In a preferred embodiment phage display methodology is performed on an antibody set of non-human origin using a TG family protein, preferably TG2, TG3 or TG6 as immunogen.

Expediently, in the differential binding assay of step ii) the reference and test TG family proteins as defined herein are used so that therapeutic compounds binding to only a definite part of the epitope as explained below are selected.

In a further aspect we also disclose a method for prevention of the onset or treatment of a gluten induced autoimmune disease in a patient comprising the administration of a therapeutic compound which is an inhibitor of the binding of a celiac antibody to a celiac epitope present on TG2 of the patient whereas said inhibitor is capable of reversing/interfering with an adverse effect of a celiac autoantibody in cell culture or tissue culture. We also disclose a method for treatment of or a method for preventing the onset of a gluten induced autoimmune disease in a patient comprising the administration of a therapeutic compound to a patient having a gluten induced autoimmune disease including a latent form thereof,
said compound being capable of binding to a part of the main celiac epitope only, said part is being at least partially formed or contributed by
one or more surface amino acid residue(s) of the first alpha helix of the core domain or
one or more surface amino acid residue(s) of the first alpha helix of the beta-sandwich domain
and of the conserved HisHisThr motif of the beta sandwich domain;
with a sufficiently high avidity of binding affinity to displace a celiac autoantibody said compound being capable of reversing or antagonizing an adverse effect of celiac autoantibodies on cells of a cell or tissue culture, optionally epithelial cell tissue or culture, and wherein said compound does not effect or alter by more than ±50%, preferably more than ±40%, more preferably more than ±30% or ±20% the transglutaminase activity of said active protein and/or said compound does not affect or alter by more than ±50%, preferably more than ±40%, more preferably more than ±30% or ±20% the GTPase activity of said protein.

In a further aspect the invention relates to a therapeutic compound
for use in the prevention of the onset or treatment of a gluten induced autoimmune disease,
said compound
being a monoclonal antibody or any fragment thereof comprising a binding region, and said compound being a specific inhibitor of the binding of a celiac antibody to a celiac epitope present on a protein belonging to the transglutaminase family, and
said compound being capable of binding
to a part of the main celiac epitope which is at least partially formed by an amino acid residue corresponding to or equivalent to Glu153 numbered according to the amino acid numbering of the full length human TG2 based on amino acid sequence alignment
and
said part being a part of the molecular surface of a TG family protein to which or to a part of which Mab 885 is capable of binding, including a surface area within 7 Å from the area covered by Mab885 when bound,
with a sufficiently high avidity of binding affinity to displace a celiac autoantibody,
said compound being capable of reversing or antagonizing an adverse effect of celiac autoantibodies on cells of an epithelial cell or tissue culture and
wherein said compound does not affect or alter by more than ±50% the transglutaminase activity of said active protein and/or said compound does not affect or alter by more than ±50% the GTPase activity of said protein.

In an embodiment the therapeutic compound is an inhibitor compound capable of binding to the binding site of the celiac antibody, preferably the inhibitor compound carries a mimotope of the celiac epitope. In this embodiment preferably the mimotope has a structure which is different from the amino acid sequence of a transglutaminase or parts thereof forming a main celiac epitope; thus the molecular surface of the mimotope is similar to a main celiac epitope only to the extent that celiac autoantibodies bind thereto. Expediently, the binding is a high affinity or avidity binding, preferably an irreversible binding. Thereby the inhibitor compound is capable of blocking the binding of a celiac autoantibody to the celiac epitope.

In a further, preferred embodiment therapeutic compound useful in the present invention is capable of binding to a part of the celiac epitope only, e.g. only to a part of the surface amino acids composing or forming the celiac epitope, whereby if contacted with a TG family protein useful in the present invention (in a competition assay) it is capable of displacing a celiac antibody and thereby to antagonize its effect.

Preferably, said therapeutic compound does not impair the functional and physiological opening, i.e. the open-close conformational transition of an active transglutaminase.

Preferably, the binding of said therapeutic compound does not block activity of an active protein belonging to the transglutaminase family.

Preferably, the therapeutic compound affects or alters the transglutaminase activity by less than ±50%, preferably less than ±40%, more preferably by less than ±30% of the activity of said active protein.

Preferably, said therapeutic compound affects or alters the GTPase activity by less than ±50%, preferably less than ±40%, more preferably by less than ±30% of the activity of said active protein. Preferably, said therapeutic compound is capable of reversing an adverse effect of a celiac autoantibody on cells of an endothelial cell culture, preferably on HUVEC cells.

In a preferred embodiment the therapeutic compound is capable of binding to a part of the celiac epitope only, whereby if contacted with a TG family protein in a competition assay it is capable of displacing the celiac antibody and thereby to antagonize its effect.

In a preferred embodiment, the therapeutic compound is capable of binding only to a region of the beta-sandwich domain and does not bind to the core domain. Preferably, it is capable of binding to one or more surface amino acid residues of the first alpha helix of the beta-sandwich domain and the conserved HisHisThr motif of the beta sandwich domain, preferably a surface amino acid residue selected from the last six, five, four or three amino acid residues of said first alpha helix of the beta-sandwich domain and amino acid residues of the HisHisThr motif, more preferably the sixth amino acid residue of said helix and/or the first amino acid of said HisHisThr motif.

In a further preferred embodiment the therapeutic compound is capable of binding to a region of the core domain and does not bind to the beta-sandwich domain only. Preferably, it is capable of binding to one or more surface amino acid residue of the first alpha helix of the core domain, preferably a surface amino acid residue selected from the first four, three, or two amino acid residues of said alpha helix, more preferably the first amino acid residue of said alpha helix.

In a preferred embodiment said therapeutic compound is capable of binding to an amino acid residue in a TG family protein corresponding to Glu153 of the human TG2 but incapable of binding to an amino acid residue in a TG family protein corresponding to Arg19 of the human TG2 protein and numbered accordingly.

Preferably, the therapeutic compound is an antibody, preferably a monoclonal antibody. In a highly preferred embodiment said antibody is the Mab 885 monoclonal antibody [Phadia, Uppsala, Sweden] deposited on March 25, 2010 according to the Budapest Treaty by Phadia AB (P.O.Box 6460 751 37 UPPSALA, Sweden, Visiting address: Rapsgatan 7P 754 50 Uppsala) under Accession number DSM ACC3053 at DSMZ - Deutsche Sammlung von Microorganismen un Zellkulturen GmbH, Inhoffenstr. 7 B, D-38124 Braunschweig, Germany.

In a preferred embodiment the therapeutic compound is a fragment, variant, derivative or analogue of the Mab885 comprising a binding region having the amino acid sequence of a variable region of Mab885 or an amino acid sequence having at least 60%, 70%, 80%, 90%, 95%, 98% or 99% sequence identity therewith and being capable of binding to the main celiac epitope of a protein belonging to the transglutaminase family.

### DEFINITIONS

The *fold* of a protein or domain structure is understood herein as the spatial (three dimensional) arrangement of its secondary structural elements (including *major structural elements*, e.g. helices, e.g. alpha-helices, and extended structures, e.g. beta structures, e.g. beta strands, parallel and antiparallel beta sheets, beta barrels, and *minor structural elements*, e.g. various turns, e.g. beta or gamma turns, loops etc.) relative to each other.

A *secondary structural element (SSE)* is a unit of secondary structure of a given part or segment of a protein having a defined conformation. SSEs can be assessed, predicted or calculated by various methods (see below). For example, according to an embodiment two or optionally three types of SSE-s are used are used: alpha-helix, extended structure and optionally coils or random structures (see below).

The fold of two proteins or two protein domains are considered herein to be analogous or similar if at least the majority of, preferably at least 60%, 70%, 80% or at least 90% of or each of the major SSEs of one of the proteins or domains can be corresponded to those of the other. In case its fold is at least 80%, preferably at least 90% identical they are called essentially identical. This is the case e.g. if to a given secondary structure element in a first protein or domain which is connected or adjacent to given further elements, a corresponding element of the same type can be found in a second protein or domain which is connected or adjacent to elements of the same type as the given further elements in the first protein or domain. The ratio of the corresponding SSEs can be calculated by number of amino acids or by number of the SSE.

The fold of a protein after any event or effect remains essentially intact or is maintained if any of the following criteria are fulfilled:
i) the spatial arrangement of at least the major SSEs (helices, e.g. alpha helices and extended structures, e.g. beta structures, like beta sheets and beta barrels) is maintained and/or if the fold remains analogous or similar or essentially identical,
ii) the ratio of the major structural elements is changed by at most 40% or 30%, preferably by at most 25% or 20%, more preferably by at most 15%, 10% or 5% and the ratio of the random structures is changed by at most 40% or 30%, preferably by at most 25% or 20%, more preferably by at most 15%, 10% or 5%,
iii) any activity of the protein remains detectable,
iv) any composite or conformational (non-continuous) epitope of the protein remains immunologically detectable.

It is to be understood that any art methods useful for studying said criteria can be applied in the present invention. The skilled person will know that all these methods have their limitations so the definition above is to be interpreted necessarily in view of these limitations.

A protein or protein domain, including any mutant, variant or homologue thereof has a *native fold* if its fold is similar or analogous to or essentially identical with a wild type protein. A *transglutaminase fold* of a protein or domain is understood herein as a fold analogous or similar or essentially identical to the fold of a TG family protein or a respective domain thereof (e.g. domain I, II, III or IV), preferably wherein said TG family protein may be an autoantigen in celiac disease and preferably wherein said TG family protein is a eukaryotic, an animal, a vertebrate or mammalian protein.

A *folded three dimensional structure* of a protein or domain is understood herein as a structure wherein spatially (three dimensionally) defined structural elements, e.g. SSEs (including major structural elements, e.g. helices, e.g. alpha-helices and extended or beta structures are arranged in a spatially defined albeit flexible tertiary structure which provides a certain extent of thermodynamic stability to the protein or domain under the conditions given. Alternatively, a folded protein or domain has a definite or definable fold. An unfolded protein or domain or a molten globule state is not considered as folded.

*A folded three dimensional structure* of a protein or domain is *native* if it has a native fold.

In preferred embodiments, in a folded or native folded three dimensional structure the fold of a protein or domain remains essentially intact or is maintained. As immediately apparent to a skilled person this can be experimentally shown by a number of structure analysis method. Any of these methods can be used to characterize the folded nature of the said protein or domain, with the proviso that the definition of folded nature in this case is limited or bound by the method applied.

As also apparent to a skilled person that if the protein remains active, the protein must be essentially or at least partly folded which is to be considered as folded herein. Furthermore, if composite or conformational (non-continuous) epitopes are present, the protein or domain is also to be considered as folded and this fact can be detected by an appropriate antibody.

A *mutant* of a given protein or protein domain is a protein or protein domain in the sequence of which one ore more amino acid residues has been deleted, substituted or inserted by genetic engineering or random mutagenesis method as compared to the sequence of the given protein or protein domain.

A *variant* of a given protein or protein domain is a protein or protein domain the sequence of which differs in one ore more amino acid residues as compared to the sequence of the given protein or protein domain, wherein the variant may comprise deletions, substitutions or insertions.

A *homologue* of a given protein or protein domain or a protein or domain homologous thereto has a sequence identity of at least 30%, 40%, 50%, 60%, 70%, 80%, 90% or 95 %, and its fold is analogous or similar. Preferably, at least 50%, 60%, 70%, 80%, 90% or 95 % of the conserved regions of the given protein family are the same in a protein or domain and its homologue.

A *mutant*, *variant* or *homologue* has preferably an analogous or essentially identical fold as the given protein or protein domain, and even more preferably the amino acid sequence identity between said mutant, variant or homologue, independently from each other, and the given protein or protein domain is at least 40%, preferably at least 50%, at least 60% or more preferably at least 70%, at least 80% or at least 90% or 95%.

*Altering the side chain* of an amino acid residue in a given protein, i.e. in the amino acid sequence or the three dimensional structure may comprise altering the side chain geometry, side chain size, side chain functional group, or side chain charge either by mutagenesis, including site directed or random mutagenesis or by chemical derivatization.

*Side chain geometry* of an amino acid present in a polypeptide or protein molecule can be characterized by any method or model appropriate for molecular geometry calculation. For example, side chain geometry can be understood for example as the shape of a three dimensional surface of the side-chain (enclosing the molecular volume), e.g. the van der Waals surface, or a spatial arrangement of atomic nuclei of a set of atoms, either with or without hydrogen atoms, forming the side chain relative to each other etc. Side chain geometry can be calculated by any suitable mathematical method as well-known to a person skilled in the art.

*Side chain size* of an amino acid present in a polypeptide or protein molecule relates to any geometrical measure related or characteristic to the size of said side chain, e.g. length (expressed e.g. in pm or Å), surface area (expressed e.g. in pm² or Å²) or molecular volume (expressed e.g. in pm³ or Å³) of a side-chain of said amino acid.

*Side chain functional group* is understood herein as a chemical functional group present in the side chain of an amino acid present in a polypeptide or protein molecule.

The *spatial position* of an amino acid residue in a polypeptide or protein molecule i.e. in a three dimensional protein structure, e.g. transglutaminase, is understood herein as a set or entirety of the positions of its atoms in the three dimensional structure of the protein, or a set or entirety of its atomic or position coordinates in a coordinate system taken for, defined by or fitted to the given protein three dimensional structure. Thus, *altering the spatial position of an amino acid residue* is to be understood as altering or mutating the protein structure so that these positions or coordinates are altered in a higher degree than those resulted from flexibility or conformational movement of that protein under the conditions given. For example, the alteration of a spatial position of an amino acid may result from a mutation in the vicinity or neighbourhood of said amino acid resulting a change in the side chain position or conformation or from a mutation effecting also the position of the alpha carbon atom or the peptide backbone related to that amino acid.

In particular, in proteins belonging to the transglutaminase family and having a folded beta-sandwich domain and a folded core domain such an alteration in spatial positions of a surface amino acid residue of the first alpha helix of the core domain, and a surface amino acid residue of the first alpha helix of the beta-sandwich domain may be characterized by a distance between these sets of amino acids i.e. their spatial positions relative to each other.

The expressions *transglutaminase superfamily* or *transglutaminase family* of proteins (TG family proteins) are used interchangeably and are understood herein as a class of proteins categorized as EC 2.3.2.13, said proteins having a domain structure comprising an N-terminal beta-sandwich domain, a core domain which is the catalytic domain in these proteins when they have activity, a first beta-barrel domain and a second beta-barrel domain (also called domains I, II, III and IV domain II being the core domain) or a mutant or fragment thereof comprising at least a folded N-terminal beta-sandwich domain and a folded core domain. As a review, see e.g. [Lorand, L., and Graham, R. M., Nat Rev Mol Cell Biol 2003, 4, 140-156]. A protein belonging to the transglutaminase family as defined above is referred herein in short as a *TG family protein.*

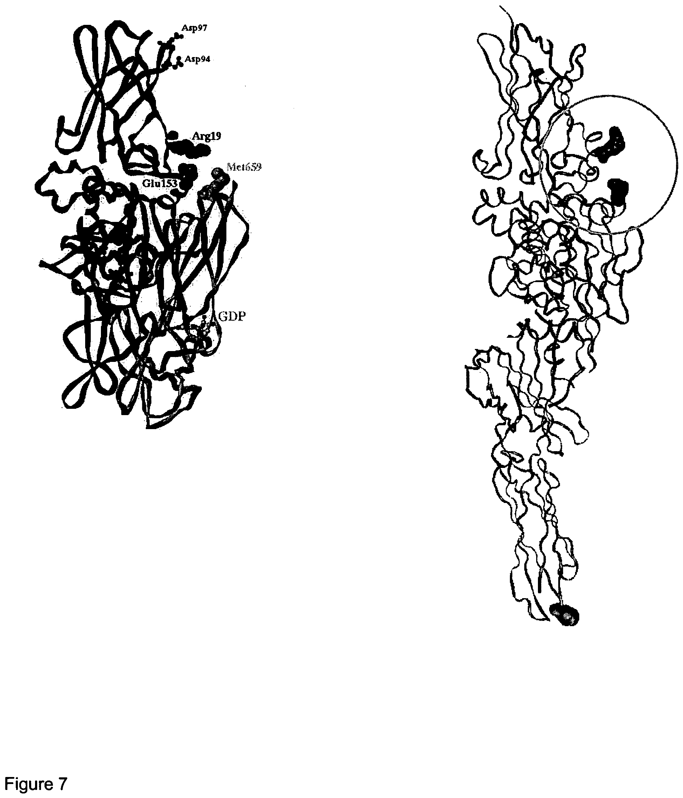

Preferably, the core domain has a fold analogous to or essentially identical with the core domain of any of the products of any of the following genes: F13A1, TGM1, TGM2, TGM3, TGM4, TGM5, TGM6, TGM7 or EPB42 as indicated for example on Table 1 and Figure 7 of Grenard et al, [J. Biol. Chem. (2001) vol. 276(35) pages 33056-33078], or any eukaryotic equivalent or homologue thereof provided that it has said core domain with said fold, or any mutant, variant or homologue thereof. Preferably, in said gene products the beta-sandwich domain has *a first alpha helix of the beta sandwich domain* and said core domain also has the *first alpha helix of the core domain* as defined below or in the Brief description of the invention.

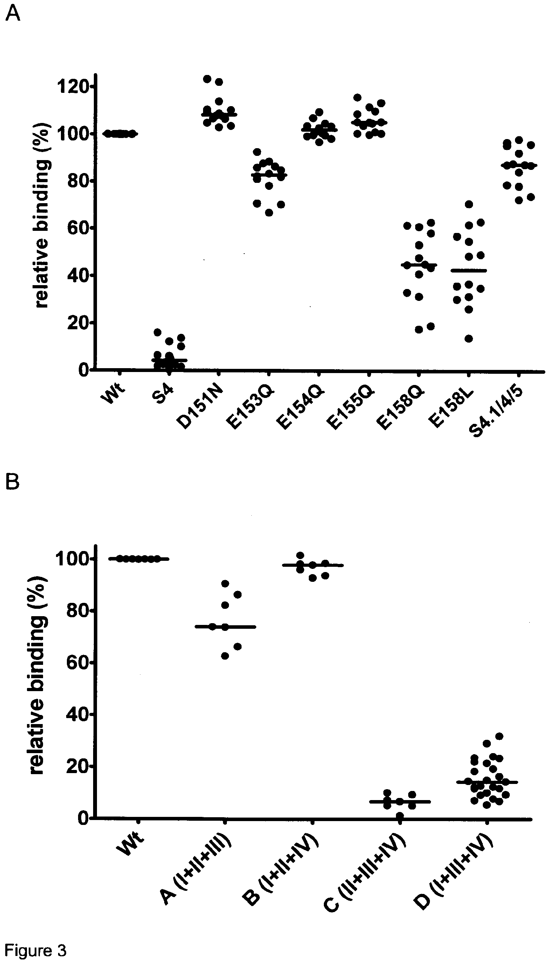

*The first alpha helix of the core domain* of a protein belonging to the transglutaminase family is the first alpha helix from the N-terminal of the core domain or domain II (which is the catalytic domain of active members of the transglutaminase family but being also present in the inactive members of the family in a catalytically inactive form). The first alpha helix of the core domain is adjacent to the first alpha helix of the beta-sandwich domain in the native transglutaminase fold. Thus, the *first alpha helix of the core domain* is an alpha helical SSE corresponding to any of the following alpha helical secondary structure elements (SSE):
an SSE spanning from Glu153 to at least Tyr159 or Val160 in human transglutaminase 2,
an SSE spanning from Glu198 to at least Tyr204 or Val205 in human factor XIII,
an SSE spanning from Glu153 to at least Tyr159 or Val160 in human transglutaminase 1,
an SSE spanning from His148 to at least Tyr154 or Val155 in human TG3,
as indicated e.g. in Loránd and Graham, Nature Reviews 2003, Vol. 4, page 140, figure 3.

The *first alpha helix of the beta sandwich domain* of a protein belonging to the transglutaminase family is the first alpha helix from the N-terminal of the N-terminal beta-sandwich domain or domain I. The first alpha helix of the beta-sandwich domain is adjacent to the first alpha helix of the core domain in the native transglutaminase fold. Thus, the *first alpha helix of the beta sandwich domain* is an alpha helical SSE corresponding to any of the following alpha helical secondary structure elements (SSE):
an SSE spanning from Ser14 to His 21 in human transglutaminase 1,
an SSE spanning from Leu14 to His21 in human transglutaminase 2,
an SSE spanning from Thr12 to His19 in human TG3,
an SSE spanning from Trp57 to His64 in human factor XIII,
[see Loránd and Graham, Nature Reviews Volume 4, page 140, 2003].

The *first*, *or the first n amino acid residue(s)* of an SSE is(are) the first or the first n amino acid residue(s) of the SSE from the N-terminal, i.e. the most amino-terminally located amino acid residue(s) of said SSE wherein n is a positive integer. The *last or the last n amino acid residue(s)* of an SSE is(are) the last or the last n amino acid residue(s) of the SSE from the C-terminal, i.e. the most carboxy-terminally located amino acid residue(s) of said SSE wherein n is a positive integer. The ascertion whether an amino acid residue belongs to a given SSE can be based on the three dimensional structure of the protein or homologous proteins if related by sequence alignment or homology modelling or by any other appropriate method and/or based on the torsion angle value(s) of the respective amide plane(s) and/or by any appropriate detection, calculation or prediction method. The position of an amino acid residue which is *in n amino acid distance* from a given amino acid in either C-terminal (carboxy-terminal) or N-terminal (amino-terminal) direction is to be calculated by counting each of n amino acid residues in the said direction starting from the first amino acid which is adjacent to the given amino acid in the said direction, wherein n is a positive integer.

A *gluten-induced autoimmune disease* involves any disease, either in latent or explicit (overt) form, the symptoms of which can be induced by gluten and during the course of the disease autoantibodies are elicited in the patient against a transglutaminase (TG), preferably against TG2, TG3 or TG6. Gluten-induced autoimmune diseases typically include e.g. celiac disease (also known as coeliac disease, non-tropical sprue or gluten-sensitive enteropathy) which typically develops among genetically predisposed persons with the human leucocyte antigen (HLA) DQ2 or DQ8 background, and dermatitis herpetiformis in which, in addition to disorders in the intestine, there are granular IgA depositions in the skin subpapillar areas.

An *epitope* is a patch or site on a protein molecule, preferably a part of its molecular surface, to which an antibody or the binding region of an antibody can bind.

An epitope can be defined e.g. by giving the amino acid residues contributing to this patch or site of the molecule or the formation of the given molecular surface, or coordinates thereof; by giving its location on the protein molecule in terms of structural elements contributing to the fold of said protein; or by defining the molecular surface part by mathematical calculation methods.

A *mimotope* of an epitope is a patch or site on a molecule, preferably a part of its molecular surface, to which the same antibody or the binding region can bind as to said epitope.

Epitopes as molecular surface parts of a molecule can by given and mimotopes can be prepared e.g. as described [Goede, Andrean et al.: BMC Bioinformatics 2005, 6:223].

*A main celiac epitope* is understood as an epitope for which at least one of the following features are true:
a) a part of the molecular surface of a TG family protein to which a disease specific autoantibody of a subject with celiac disease is capable of binding, wherein said autoantibody is capable of binding to said part of the molecular surface whereas autoantibodies of a subject with any other disease having autoantibodies capable of binding to TG2 usually or typically do not bind to said part of the molecular surface; or
   a part of the molecular surface of a TG family protein to which or to a part of which Mab 885 is capable of binding, optionally including a surface area within 2, 3, 4, 5, 6, or 7 Å from the area covered by Mab885 when bound, or
   a part of the molecular surface of a TG family protein, to which a celiac autoantibody of a subject with celiac disease is capable of binding as explained above, in a manner that said autoantibody can be displaced in a competitive assay by Mab 885;
b) a part of the molecular surface of a TG family protein which may be an autoantigen in celiac disease, said molecular surface part at least partially formed or contributed by
   one or more surface amino acid residue(s) of the first alpha helix of the core domain, preferably a surface amino acid residue selected from the first four, three, or two amino acid residues of said alpha helix, more preferably the first amino acid residue of said alpha helix, and/or
      one or more surface amino acid residue(s) of the first alpha helix of the beta-sandwich domain and the conserved HisHisThr motif of the beta sandwich domain, preferably a surface amino acid residue selected from the last six, five, four or three amino acid residues of said first alpha helix of the beta-sandwich domain and amino acid residues of the HisHisThr motif, more preferably the sixth amino acid residue of said helix and/or the first amino acid of said HisHisThr motif;
c) a part of the molecular surface of a TG family protein which may be an autoantigen in celiac disease, overlapping with or comprising a surface part within a sphere having a 6, 7 Å radii calculated from any atom of the Glu153 and/or within a sphere having a 6, 7 Å radii calculated from any atom of the Arg19 according to the amino acid numbering of the full length human TG2, or an amino acid residue corresponding/equivalent thereto in, respectively, in an animal (preferably vertebrate, more preferably mammalian) TG2 based on multiple sequence alignment;
d) a part of the molecular surface of a TG family protein which may be an autoantigen in celiac disease, at least partially formed by amino acid residue corresponding/equivalent to Glu153 and/or Arg19 numbered according to the amino acid numbering of the full length human TG2 based on multiple sequence alignment;
e) a patch of a molecular surface of a molecule, e.g. a part of the molecular surface in a protein, preferably a TG family protein, which is capable of binding a surface recognition molecule, preferably an antibody, wherein said surface recognition molecule is capable of binding to a main celiac epitope as defined in a) to d),
wherein preferably said TG family protein is
- a eucaryotic protein, preferably an animal protein, more preferably a vertebrate or mammalian or human protein,
- a TG2 and/or TG6 protein,
- an engineered TG family protein, preferably wherein a main celiac epitope is created by mutation or wherein a main celiac epitope is impared by mutation.

The main celiac epitope can be considered as *integral* if
it is intact, i.e. has a molecular surface essentially identical with the molecular surface of a wild type protein of the TG family which is autoantigen in celiac disease, and/or
a celiac antibody known to be capable of binding the main celiac epitope, e.g. to that epitope of TG2, is capable of detectably binding to it, and/or
the side chain geometry, side chain size, side chain functional group, charge and spatial position of at least the first N-terminal surface exposed amino acid residue of the N-terminal alpha helix of the core domain, as defined below, are essentially identical with or immunologically indistinguishable from the same values for a corresponding wild type protein of the transglutaminase family (TG family protein) which may be an autoantigen in celiac disease, and/or
it is altered as compared to the test protein in which the main celiac epitope is impaired to obtain a reference protein in which the main celiac epitope is integral or recognizable by celiac antibodies.

A *main celiac epitope is impaired or deficient* if in comparison with the main celiac epitope of a wild type transglutaminase, which is an autoantigen in celiac disease, it provides a different molecular surface and therefore a celiac antibody can be found which bind to the impaired main celiac epitope with a lower affinity or avidity, or it can not bind.

In an embodiment, binding of a celiac antibody to a test protein in which the celiac epitope is different from an intact celiac epitope is impaired if the binding affinity and/or avidity and/or rate of the binding reaction is reduced as compared to a reference protein, preferably the average or mean binding level is reduced by at least a predetermined ratio, preferably by at least 30%, more preferably by at least 40%, even more preferably by at least 50%).

A *celiac antibody* is an antibody capable of specifically recognizing and binding to the main celiac epitope.

A *celiac autoantibody* is a celiac antibody which is an autoantibody of a subject in celiac disease wherein said autoantibody is capable of binding to a wild type TG family protein, wherein said protein is an autoantigen in celiac disease, preferably TG2 and/or TG6 and optionally TG3, more preferably TG2, and/or it is capable of binding at the main celiac epitope as defined above. *Endomysial antibody* is a celiac antibody capable of binding to the connective tissue structure surrounding smooth muscle cells in human or primate esophagus or of other tissue sections containing smooth muscle cells and which antibody is detected on frozen tissue sections by indirect immunofluorescent method.

The *molecular surface* of a protein molecule, e.g. a TG family protein, is the surface area which describes the three dimensional surface of said protein molecule which is accessible to other chemical entities, e.g. to the solvent or other molecules, e.g. ions or protein molecules, e.g. antibodies. For the purposes of the present description the meaning of molecular surface also includes the "accessible surface area" or "Lee-Richards molecular surface", the solvent-excluded surface or Connolly surface or the Van der Waals surface. Thus the molecular surface can be calculated by any algorithm suitable for calculation of such three dimensional surfaces irrespective of variations between calculation methods as so far as the model used for calculation provides a scientifically correct estimation of the surface.

*Binding affinity*, as used herein is a thermodynamic expression of the strength of interaction between an antigen binding site and an antigenic determinant (and thus of the stereochemical compatibility between them). In a preferred meaning, the term is applied to interactions among antigenic determinants and antibody binding sites. Binding affinity can be characterized by a quantity or measure calculated from or correlating to the dissociation constant or a function thereof, e.g. the reciprocal of the dissociation constant 1/K_{d} (association constant) or the negative logarithm of the dissociation constant or a function thereof, such as -log₁₀[K_{d}/NA * (mol/l)⁻¹] which is also indicated as pK_{d}. For example, binding affinity can be characterized by the quantity or ratio of the bound molecules, e.g. ligands or antibodies, in or after an equilibrium state. Typically, owing to the heterogeneity of affinities in a population of antibody molecules of a given specificity, binding affinity (and e.g. the association constant or the pK_{d}) actually represents a kind of average value. *Avidity*, as used herein in an aspect relates to the combined strength of affinities in a protein-ligand complex. In an other aspect, avidity describes the binding intensity of multiple bond interactions between proteins. Preferably, avidity refers to the strength of antibody-antigen binding. Avidity is meant herein as a term including affinity but more broadly as avidity is also applicable to describe strength of a multiple bond. For instance, a single binding site of IgM may have low affinity but still IgM has high avidity due to its 10 weak binding sites contrary to the two strong binding sites of IgG. An *inhibitor of the binding of a celiac antibody to a celiac epitope* is a compound capable of binding to either a TG2 (transglutaminase) or a celiac antibody and upon binding of which the detectable level or affinity of binding is reduced as compared to the binding in absence of said inhibitor.

### BRIEF DESCRIPTION OF THE FIGURES

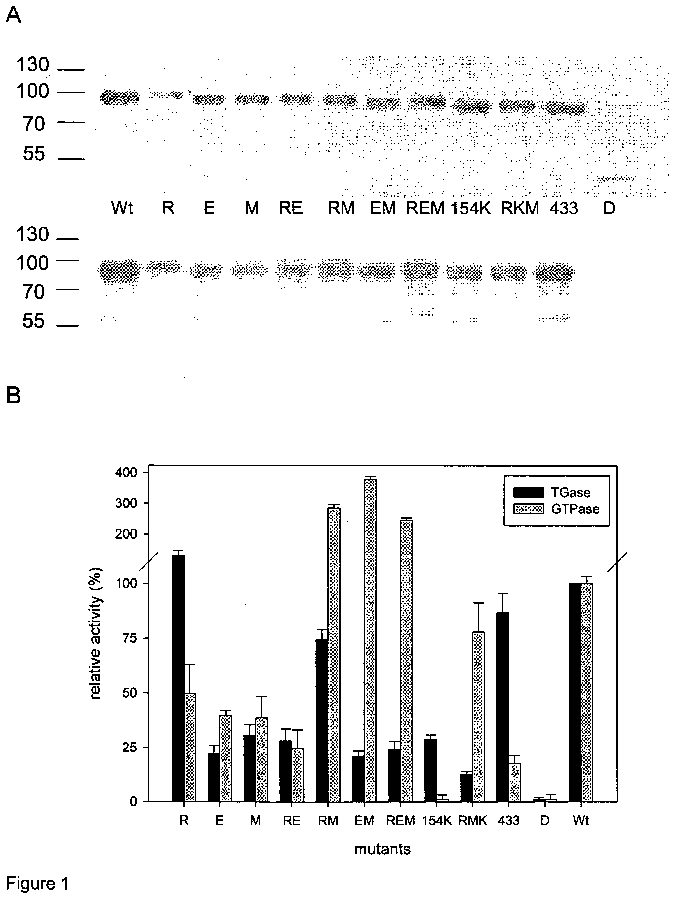

1. Characterization of the recombinant transglutaminase 2 (TG2) mutant proteins. (A) Western blot. Upper panel: primary antibody polyclonal goat anti-TG2; lower panel: primary antibody monoclonal mouse anti-transglutaminase 2 antibody TG100. Wt, wild-type TG2, R, R19S, E, E153S, M, M659S, RE, R19S-E153S double mutant, RM, R19S-M659S double mutant, EM, E153S-M659S double mutant, REM, R19S-E153S-M659S triple mutant, 154 K, E154K mutant, 433, R433S-E435S double mutant, D, domain mutant which contains only domains I-III-IV
   (B) Transglutaminase (TGase) and GTPase activities of the mutant proteins as percentages of respective values of wild type TG2. Values are presented as means from two separate measurements done in triplicate.
2. Fibronectin binding of TG2 mutants. Proteins directly coated to the plate (aTG-ELISA) or added to fibronectin were meaured by monoclonal anti-TG2 antibody TG100 recognizing the core domain (II). Results showed equal antigen amounts on the plate in the assays and relative to each other. These amounts were used in further measurements with celiac samples. Values are presented as means from two separate experiments done in duplicate. For the abbreviation of proteins see Figure 1.
3. Binding of serum IgA antibodies from celiac patients (n=14) to Site 4 mutants (A) and domain mutant TG2 proteins (B) in ELISA performed with direct coating of antigens to the plate. Results are shown as percentages of the binding relative to wild type TG2. S4, Site 4 mutant, S4.1/4/5, D151N-E154Q-E155Q combined mutant.
4. Binding of serum IgA antibodies from celiac children presenting with severe malabsorption (upper panel) and adult patients with malabsorption (lower panel) to mutant TG2 proteins. Anti-TG2 ELISA performed by direct coating of antigens to the plate. Binding results are shown as percentages of amounts bound to wild type TG2 in comparison to anti-TG2 monoclonal antibody TG100. All serum samples were examined in duplicates. Dashes indicate medians. For the abbreviations of mutants see Figure 1.
5. (A) Structure of the putative celiac epitope in TG2 and Factor XIII. (B) Binding of serum IgA antibodies of celiac children (n=20) and adults (n=17) to wild type TG2 and TG2 mutants mimicking the surface of Factor XIII. K, E154K, RKM, R19S-E154K-M659S triple mutant.
6. Binding of serum IgA antibodies from celiac children and adults to fibronectin-bound TG2 mutants in ELISA. Results are shown as percentages of amounts bound to wild type TG2 in comparison to anti-TG2 monoclonal antibody TG100.
7. Structure of the celiac epitope of TG2 in closed and open form of the protein.
8. Disease specificity of the celiac epitope targeting. Binding of anti-TG2 antibodies from celiac disease patients and from patients with other autoimmune diseases (n=11) to wild type and mutant TG2 proteins.
9. Celiac disease antibodies from different patients recognize the same epitope in competition assays. (A) Serum samples from 56 IgA deficient celiac patients containing IgG class anti-TG2 antibodies compete with the same celiac IgA in ELISA using wild type TG2 and the competition is proportional to their serum concentrations (A2). Three of the patients also have IgM class anti-TG2. Serum samples from non-celiac IgA deficient (n=23) and IgA competent subjects (n=22) do not show interference (A1). (B) Purified IgG celiac antibodies reacting with the N-terminal R19S mutant (G1) or not reacting with the R19S mutant (G5) but both equally non-reactive to R19S-E153S composite (RE) mutants compete in the same dose-dependent manner with purified celiac IgA not reacting to R19S, whereas total IgG fraction (K4) prepared from non-celiac subject does not show interference. IgA and IgG antibodies bound to ELISA plate were separately recognized.
10. Correlation of bindings of celiac antibodies to Site 4 and Site 5 mutants compared to wild type TG2
11. Stability of the celiac epitope targeting in celiac patients undergoing diagnostic gluten challenge after a period of diet and seronegativity (A-B) and in a celiac patient followed up for 5 years without diet (C).
12. Tissue deposited and passively transferred pathogenic anti-TG2 antibodies have same epitope specificity as the antibodies in the serum of celiac patients. (A) Maternal anti-TG2 IgA antibodies deposited on the surface of chorionic villous structures of the infant and in the maternal parts of the placenta in a celiac woman with active disease. Passively transferred maternal IgG deposited in umbilical cord tissues of the newborn as endomysial antibodies. (B) Antibodies eluted from these tissues show similar epitope specificity as serum antibodies using TG2 mutant proteins. (C) Human umbilical cord vein endothelial cells (HUVEC) from a newborn with maternal celiac antibodies show abnormal shape, spreading and decreased cell lengths compared to HUVEC prepared from a newborn of a celiac mother on diet and without anti-TG2 antibodies.
13. Measurement of binding kinetics of purified celiac IgA to the same amounts of wild type and Site 4 mutant TG2 using plasmon surface resonance. Decreased binding and faster dissociation are observed.
14. Similar epitope specificity of serum antibodies in subjects with latent (CDL, n=11) and overt (CD, n=11) celiac disease.
15. Characteristics of an anti-TG2 antibody clone with potential for use in immunotherapy. Clone 885 antibodies have epitope binding specificity partially overlapping with those of celiac disease antibodies (A) and compete with natural celiac patient IgA in ELISA (B), whereas anti-TG2 monoclonal antibodies TG100, CUB7402 and H23 do not show interference with celiac IgA binding. Values are shown as bound amounts of celiac IgA to wild type TG2 in the presence of antibodies compared to the values without antibodies. Representative values from three independently performed experiments. (C) 885 antibodies bind to placenta tissue sections of a celiac woman and displace tissue-deposited celiac IgA in vitro. Released patient IgA antibodies appear in the incubation solution and are measurable by ELISA (D). (E) 885 antibodies do not inhibit, alter or activate normal transglutaminase activity of recombinant wild type TG2. (F) 885 antibodies co-administered with celiac IgA reverse effects of celiac IgA on the length of HUVEC cells in culture.
16. Frozen small bowel section from a non-celiac subject incubated with serum antibodies from celiac patients and monoclonal mouse antibodies 885 (A) and CUB7402 (B). Bound human IgA was recognized with fluorescent isothiocyanate-conjugated green fluorescent secondary antibodies and mouse antibodies were labelled with rhodamine-conjugated red fluorescent secondary antibodies. Under these conditions, 885 was not able to bind to the tissue along the endomysial structures where patient antibodies bound. CUB bound to endomysial structures along the binding of patient IgA resulting in a merged yellow color.

### DETAILED DESCRIPTION OF THE INVENTION

The present inventors studied human TG2 applying a complex structural, biochemical and immunological approach.

The monomeric human TG2 consists of 687 amino acids with a molecular weight 76 kDa and is composed of four structural domains: N-terminal beta-sandwich domain with a fibronectin-binding region, catalytic core domain with the catalytic triad, and two C-terminal beta-barrel domains. The GDP-bound form of TG2 has been crystallized a few years ago, which is the "closed" conformation of the enzyme [Liu S et al, Proc Natl Acad Sci USA. 2002;99(5):2743-7.]. The inventors started the localisation of the potential binding sites of celiac antibodies with this model. In this conformation the GDP is bound in a cleft between the catalytic core and the first β-barrel, and the catalytic triad, which is responsible for the transamidation activity, is hidden inside the molecule and it is inhibited by two loops, so the enzyme can not operate as a transglutaminase. Recently, the enzyme's "open" form has been crystallized in a complex with a gluten peptide-derived inhibitor [Pinkas DM et al, PLoS Biol. 2007;5(12):e327.]. In this case the C-terminal β-barrel domains are displaced by 120 Å -compared to the GDP-bound form- and the active site residues become accessible to the substrates. This activated form of TG2 is "frozen" in this model with the active site inhibitor bound to the catalytic amino acids. Despite this finding, a Ca²⁺-bound form of TG2, without substrate or inhibitor, is still unknown. It is still a question, which form of TG2 appears extracellularly, where it is recognizable for the celiac autoantibodies. After a series of protein engineering experiment the present inventors found that two anchor sites in the TG2 structure are essential in forming an epitope for patient autoantibodies in gluten induced autoimmune diseases, preferably in celiac disease. It was found that neither the substrate binding site nor Ca²⁺ ions form part of the celiac epitope, however, Ca²⁺ binding site 4 overlaps therewith. The epitope is a conformational one but as the two anchor sites are located on the N-terminal beta-sandwich domain and the core domain, binding of the majority of patient autoantibodies do not block the open-closed conformational transition of the enzyme.

Surprisingly, it has also been found by the inventors that this surface patch of the molecule can distinguish between autoantibodies from celiac patients or patients with a gluten induced autoimmune disease and those from other autoimmune diseases. Thereby a diagnosis highly selective for these diseases can be provided.

As immediately obvious for a skilled person an epitope in such a complex disease can vary from patient to patient or depending on the disease manifestation type or progress of the disease. In light of this general knowledge, it is significant that the composite epitope region found by the inventors is highly characteristic i.e. selective for celiac autoimmunity and that according to measurements with serum samples from celiac adults, only insignificant spreading of the disease epitope occurs in later years.

Evidence is also provided herein that the epitope region found herein is already present in the early preclinical stage of the disease and has also predictive value for diagnosis of celiac disease in later life. However, it is to be realized that the exact celiac epitope may spread to further amino acids as the disease proceeds and this may be considered in certain embodiments of the tests of the invention. For example, without a limitation, the following amino acids in TG2 or amino acids corresponding thereto may be mutated in order to further reduce autoantibody binding: Arg 19, His22, Asp 151, Glu 153, Glu 154, Arg156, Glu157, Leu161, Val431, Arg433, Glu435, Met659, Leu661 numbered according to the amino acid numbering of the full length human TG2. Corresponding amino acids may be found based on multiple sequence alignment.

It is obvious that the exact area of the patch of molecular surface considered as an epitope may vary as mentioned above. Thus, it should be understood that amino acid replacements or other mutations outside the most central region of the epitope may effect antibody binding. Such regions, without completeness, can be found in certain regions on amino-terminal beta-barrel domain and on the core domain, e.g. in Ca²⁺ binding site 5. The skilled person, upon designing transglutaminase mutants for use in the present invention, will be able to consider these variations and optimise mutants for a given purpose based on the teaching provided herein.

The inventors at the first time identified, as a conformational epitope, the main celiac epitope sought but not found in the prior art. Several overlapping variants of the main celiac epitope may exists. The fact whether a given antibody binds to the celiac epitope or not can be clearly decided based on the teaching provided herein. The present inventors identified essential parts of the epitope, which provides the skilled person with the key to design diagnostic methods, mutant transglutaminases useful therein, diagnostic kits for that purpose, compounds capable of inhibiting binding of antibodies to the main celiac epitope and thereby use and design treatment methods for a gluten induced autoimmune disease. The invention is defined by the claim.

### Diagnostic Methods and Kits

Based on the teaching provided herein the skilled person will recognize that according to an embodiment of the invention the diagnostic method has two important features. On the one hand, a test TG family protein is to be provided in which the celiac epitope is impaired or deficient or lacking so that celiac autoantibodies can not bind to it or bind only at a reduced level. On the other hand, other epitopes are preferably present to ensure selectiveness of the method, therefore, at least the beta-sandwich domain and the core domain have a folded structure. Preferably any or both of the beta-barrel domains are folded as well. Thus, in the present diagnostic method a positive result very reliably indicates that the patient suffers from celiac disease.

The folded character of a domain can be characterized by several means.

A convenient way is to check activity. Thus, in a preferred embodiment a mutant TG2 has one or more of the following detectable functional activities: transglutaminase enzyme activity, a GTPase activity and/or fibronectin binding. If transglutaminase activity is decreased due to the mutation it may be due to the fact that the mutated site is involved in Ca²⁺ binding.

The fact that a non-celiac conformational antibody is capable of binding to the protein is also indicative of its folded character.

As immediately apparent to a skilled person correct protein folding can be experimentally shown by a number of structure analysis method, for example by CD (circular dichroism) spectroscopy, FT-IR (fourier-transformed infrared spectroscopy), DSC (differential scanning) microcalorimetry, NMR (nuclear magnetic resonance) spectroscopy, DPI (dual polarisation interferometry), atomic force microscope etc or predicted by the method of Chou-Fasman and GOR methods, neural network models or nearest-neighbor methodsj, as reviewed e.g. in [Simossis VA, and Heringa J, "Integrating protein secondary structure prediction and multiple sequence alignment", Curr Protein Pept Sci. 2004 5(4) 249-66; Rost B, "Review: protein secondary structure prediction continues to rise" J Struct Biol. 2001 134(2-3) 204-18.].

These methods can be used either as a fingerprint of said fold or for detection of any difference between folds of different proteins or domains, e.g. a wild type or a mutant thereof, or between folds of the same protein in different states.

In a real life diagnostic method a reference protein is to be provided which comprises an integral celiac epitope or, if a wild type transglutaminase is used, e.g. a TG2 or TG6, or in certain embodiments TG3, intact celiac epitope. It is to be noted that the molecular surface of the epitope region in TG6 is highly similar to that of TG2, thus, if desired, these epitope regions can be easily converted into each other by site directed mutagenesis. Moreover, provided that autoantibodies against TG6 in a variant of the disease are different from those against TG2, the epitope of TG6 is to be applied for diagnosis of that particular variant of disease. This is also true for further transglutaminases autoantigenic in a gluten induced autoimmune disease.

In a method differential binding of autoantibodies is to be shown. For example, if a value, preferably an average or mean value correlating to the binding affinity and/or avidity and/or rate of the binding reaction is reduced in the test protein as compared to a reference protein, preferably said value is reduced by at least a predetermined ratio, preferably by at least 20%, 30%, more preferably by at least 40%, even more preferably by at least 50% or 60% or 70% or 80%, or the corresponding remaining value is not more than 80%, 70%, more preferably not more than 60%, even more preferably not more than 50%, or 40% or 30% or 20%, respectively, this result is considered as an indication of the presence of the disease. It is also preferred if the mean binding level of the autoantibodies to the reference protein exceeds a predetermined threshold value. This threshold value can be determined by simply carrying out preliminary measurements with a reference transglutaminase using either a standard celiac sample or a sample obtained from a patient known to have celiac disease. A predetermined threshold value need not to be numerically defined in each cases, it may be sufficient if binding is safely detectable. As disclosed herein binding is usually given as a relative value in comparison with the binding of autoantibodies known to have binding properties to a transglutaminase with an integral celiac epitope.

Both the test protein and the reference protein may be a wild type protein or can be prepared by protein engineering from an appropriate scaffold which is sufficiently similar or homologous to a protein which is an autoantigen in celiac disease. For example, without completeness, as such a "scaffold" human or animal TG1, TG3, TG4, TG5, TG7, factor XIII, or EBP42 [see e.g. Lorand, L., and Graham, R. M., Nat Rev Mol Cell Biol 2003, 4, 140-156 and Grenard et al, J. Biol. Chem. (2001) vol. 276(35) pages 33056-33078].

So as to design an appropriate mutant in a transglutaminase or transglutaminase derived protein it is advisable to identify key amino acid residues. For that purpose, amino acid sequence alignment can be performed as well known in the art and includes means well known in the art, e.g. by method reviewed by Shyu et al. [Shyu et al. Genetic Programming and Evolvable Machines 2004 June; 5(2): 121-144] or by methods available e.g. on the home page of the European Bioinformatics Institute, e.g. ClustalW2, MAFFT, MUSCLE, T-Coffee, etc. and/or by using the Genetic Data Environment (GDE) software package [Smith SW et al. Comput Appl Biosci. 1994 Dec;10(6):671-5.].

At present it is also within the skills of a person skilled in the art to prepare mutations which comprise a patch of molecular surface forming an appropriate epitope. Methods for computation of molecular surfaces of proteins are available in the art and can be performed e.g. by the WHATIF program [G.Vriend, J. Mol. Graph. (1990) 8, 52-56], the Molecular Surface Package of Michael L. Connolly, Version 3.9.3 [1259 El Camino Real, #184 Menlo Park, CA 94025, U.S.A.]; Molecular Surfaces Computation (MSMS) [M.F. Sanner et al. Biopolymers, 1996 Vol. 38, (3), 305-320.]; SURFNET [R.A. Laskowski J. Mol. Graph., (1995) 13, 323-330.]; The Analytic Surface Calculation Package (ASC) [F. Eisenhaber et al. J. Comp. Chem. 1995 16(3): 273-284.]; SUPERFICIAL [Goede, Andrean et al. BMC Bioinformatics 2005, 6:223] and the method of Xiang and Hu [Xiang and Hu, BioMedical Engineering and Informatics, 2008. BMEI 2008. International Conference on; Volume 1, Issue , 27-30 Page(s):52 - 56].

Both the test protein and a reference protein can be a protein fragment provided that it meets the requirements defined herein. In a preferred embodiment, nevertheless, the test and reference proteins also comprise a folded N-terminal beta-barrel domain.

It will also be understood that any protein, which is an appropriate scaffold, as explained above, can be used in the present invention. Thus, the protein belonging to the transglutaminase family can be a eukaryotic, more preferably an animal, even more preferably a vertebrate, optionally amphibian, reptilian, fish, avian or highly preferably a mammalian or human protein.

In certain preferred embodiment of the invention the test proteins of the invention are different from mutant proteins of the art, preferably mutants as specifically described in any of the following publications [Sblattero et al. Eur J Biochem. 2002 Nov;269(21):5175-81, Nakachi K et al. J Autoimmun. 2004 Feb;22(1):53-63. Seissler et al. Clin Exp Immunol. 2001 Aug;125(2):216-21.]. Typically these mutants are deletion mutants which, provided that they are impaired or deficient in a celiac epitope, do not comprise both a folded beta-sandwich and core domains. In case the opposite would be true and certain methodology would show in these epitope deficient proteins a folded structure for at least these domains, the mutants are preferably excluded from the claimed scope.

In a certain specific embodiment, if appropriate, the TG2 E153S (E), R19S (R), M659S (M) triple mutant and the R19S (R), M659S (M) double mutant, wherein preferably the sequence comprising no further mutation, is excluded from the claimed scope. In a further specific embodiment, if appropriate, mutants comprising a TG2 Met659 mutation or an M659S mutation, wherein preferably the sequence comprising no further mutation, preferably are excluded. In a further embodiment mutant transglutaminases disclosed in Király et al. FEBS J. 2009 Dec;276(23):7083-96] are excluded from the scope of the present invention, In an embodiment Met659 is unmutated in TG2. In a further embodiment every mutant disclosed in one or all or any combination of the above publications are excluded from the scope of the present invention. In a further embodiment the diagnostic use of any or all or any combination of the above prior art mutants are excluded from the scope of the present invention.

It is well within the skills of a person skilled in the art to detect autoantibody binding. It is immediately apparent for a skilled person that both kinetic methods as well as method characterizing binding affinity or avidity are applicable. For example, any of the methods below, without any limitation, are applicable:
an immunoassay, e.g. ELISA, RIA, lateral flow, immunoprecipitation
a binding assay, e.g. Biacore, fluorescence quenching,
a spectrophotometric method, e.g. FT-IR, circular dichroism, NMR,
a physico-chemical method, e.g. calorimetry, ultracentrifugation etc.

In a preferred embodiment of the diagnostic method is carried out in the form of an immunoassay, like RIA or DELPHIA or preferably in an immunosorbent assay, like ELISA.

In a preferred embodiment of the diagnostic method of the invention the transglutaminase is fibronectin bound. Fibronectin binding provides a preliminary orientation of the transglutaminase protein thereby exposing its celiac epitope to antibodies. Thereby, the diagnostic test becomes more sensitive.

In certain embodiments, however, the object is to provide an assay which selectively differentiates among patient celiac autoantibodies and other antibodies which are not celiac antibodies, i.e. are not typical of celiac disease. In such cases it may be advisable to omit fibronectin to allow access to the whole surface of both the test protein and the reference protein. In this case preferably a non-celiac autoantibody of the celiac patient will bind both to the test protein and the reference protein with nearly equal binding affinity. In a variant of this embodiment the assay is carried out in liquid phase. In both methods the presentation of conformational epitopes are believed to be better to some extent. In a preferred variant IgA or IgG are measured as autoantibodies, provided that the patients are not deficient in any of these types.

The invention also relates to kits for performing the diagnostic methods as outlined above. These kits may comprise the parts as mentioned above or as useful in carrying out the methods outlined above. A kit necessarily comprises a test protein and at least instructions for obtaining a reference protein or the reference protein as well.

Preferably, the kit comprises a human TG family protein and means for detection of antibody binding thereto. Thus, the kit is advisably an immunological kit, and means for detection include the use of direct markers and/or secondary markers. Direct markers can be fluorescent markers or radioactive markers, secondary markers can be secondary antibodies, optionally conjugated, e.g. marked or enzymel linked antibodies. Thus the kit may work on ELISA, EMA, DELFIA, RIA etc. principles.

In a preferred embodiment of the invention the test protein of the present invention is added to an assay kit useful as a Tissue Transglutaminase Antibody Assay useful in the detection of celiac disease. Such assay kits, so called second-generation human tissue transglutaminase antibody assays are reviewed by van Meensel et al [Clinical Chemistry 50:11 2125-2135 (2004)]. The assay can be performed essentially as described with the exception of assessing binding of patient autoantibodies also to the test protein and a difference in binding is evaluated in comparison with a TG family protein with an integral main celiac epitope mentioned as a reference protein herein.

It will be understood by a skilled person that taking a calibration curve indicating the signal, e.g. OD in ELISA, in the function of antibody level is preferred to using the signal values themselves, as disclosed by van Meensel et al, above. For example, calibration curves can be taken by using an appropriately diluted antibody with known binding properties, like a TG100 antibody.

### Diagnostic Methods, Uses and Kits based on Antibody Displacement

In these embodiments, as defined in the brief description of the invention, if a patient autoantibody is displaced by a preselected test compound specifically capable of binding to the main celiac epitope, than this is indicative of the fact that said patient autoantibody is a celiac autoantibody and therefore of the disease itself, i.e. that said patient suffers from or is susceptible for a gluten induced autoimmune disease or preferably celiac disease.

Preferably, the test compound is a test antibody or fragment, variant or analogue thereof known to be capable of binding to the main celiac epitope of a protein belonging to the transglutaminase family, said protein being an autoantigen in celiac disease, preferably selected from TG2, TG3 or TG6.

In principle any antibody binding to the celiac epitope with a sufficient binding affinity or avidity is applicable in this embodiment. Such an antibody can be e.g. an isolated patient autoantibody as in Example 7.

More preferably monoclonal antibodies are prepared against the celiac epitope. Such antibodies can be prepared by known techniques, e.g. the hybridoma method. Techniques for the hybridoma method are well known and are disclosed e.g. in Kontermann, Roland; Dübel, Stefan (Eds.) Antibody Engineering Series: Springer Lab Manuals 2001, XII, 792 p. ISBN: 978-3-540-41354-7; Gary C. Howard, Matthew R. Kaser (Eds) Making and Using Antibodies: A Practical Handbook, CRC Press, 2006, ISBN: 9780849335280. Moreover, preparation of monoclonal antibody producing hybridomas can be ordered from a number of companies like GL Biochem Ltd. (Shanghai, CH), LC Sciences (Houston, TX, USA) or LAMPIRE Biological Laboratories (Pipersville, PA, USA), FusionAntibodies (Pembroke Loop Rd. BT17 0QL Northern Ireland).

In the present invention antibody selection involves selection for the epitope. In one embodiment, this can be done by selecting clones which secrete antibodies that bind to an intact TG2 or other protein of the transglutaminase protein but do not bind to a mutant which has an impaired celiac epitope. Alternatively, clones are selected which secrete antibodies which can be displaced by an antibody known to be capable of binding to the celiac epitope as disclosed herein. This can be done by routine immunological testing, e.g. by ELISA as described in the Examples.

Once monoclonal antibodies are obtained in the form of hybridomas they can be sequenced easily. Sequencing services can be ordered e.g. from FusionAntibodies (Pembroke Loop Rd. BT17 0QL Northern Ireland) based on cDNA sequence.

In an alternative approach the antibody sequence can be obtained by amino acid sequencing, e.g by the Edman method. This, while sometimes cumbersome, is a routine method and such service can be ordered from various firms e.g. from Proteome Factory AG Magnusstr. 11 D-12489 Berlin Germany. Alternatively, a relatively recent method, shotgun sequencing can be applied [Nuno Bandeira et al. Nature Biotechnology, December 2008, v26, n12, pp1336-1338].

Usually it may be sufficient to sequence only the variable domains or variable domains plus leader sequence. In certain cases full antibody sequence can be obtained. Based on the sequence the gene of the antibody or its fragments can be cloned and tailored to the given use, e.g. can be humanized [e.g. a HAMA (human anti-mouse antibody) can be prepared; a service also offered by FusionAntibodies], binding affinity can be increased by directed evolution methods etc.

Antibody fragments, like single chain antibody fragments (scFv) and Fab fragments carrying the variable region can be obtained and have several advantages due to their size and reliability. Fab fragments differ from scFv's in that as well as containing variable domains, constant regions and are a dimer linked via two cysteine residues.

Fab's and scFv's offer several advantages over monoclonal antibodies as carriers and lower sensitivity due to their small size and a lower negative response by the human immune system. These fragments can be prepared both from a synthetic DNA or from a monoclonal cell line. Such fragments can even be prepared when only the sequence of the variable region is known.

The skilled person may use the phage display method disclosed e.g. in [Marzari, R. et al. J. Immunol. 166, 4170-4176 (2001)], by which scFv's can be prepared, too. Examplary methods for the preparation of diagnostic or therapeutic antibodies based on the phage display method are described in the Examples.

Based on the teaching provided herein it is well within the skills of a person skilled in the art to identify further test compounds.

It is to be understood herein that instead of antibodies and antibody fragments further recognition molecules, e.g. other protein-based receptors of the art can be used. For example, applying the principles observed with IgG domains, successful attempts have been made to construct binding sites specific to a given target molecule on proteins that originally have no receptor characteristics [Xu, L. et al. Chemistry & Biology 2002, 9, 933-942; Skerra, A. Rev. Mol. Biotech. 2001, 74, 257-275; Nygren P. and Uhlen, M. Curr. Op. Struct. Biol. 1997, 7, 463-469]. Appropriate scaffolds are e.g. fibronectin type 3 and the lipocalin protein and formation of binding site can be achieved by a directed evolution technique combined e.g. by a display methodology. Surface of these molecules can be designed by the methods mentioned above.

Preferably, the test compound should be bound more strongly and/or it should be used in a high concentration to displace the autoantibodies.

In a preferred embodiment the binding of the test compound is detected. Binding can be detected by a labelled compound specifically binding to the test compound. Alternatively, the test compound itself can be labelled. In such case for example a signal without patient sample or antibodies can be defined as 0% inhibition (maximum binding of the test compound - no disease) and signal with blank as a 100% inhibition (corresponding to full replacement of the test compound by patient antibodies). Theoretically it might happen that if the test compound is very strongly bound disease is not recognized. However, binding levels can be influenced by concentration as well known in the art and thus it is within the skills of a skilled person to set the assay conditions such as shown in Example 7. In a preferred embodiment the bound antibodies are detected. In this case e.g. a conjugated secondary antibody can be used to detect antibody binding. The secondary antibody shall be specific to the test antibody and should not recognize patient antibodies. In a preferred embodiment the antibody type is different (e.g. IgA antibody deficient patients are diagnosed by an IgA antibody). Alternatively, the test antibody carries a specific sequence e.g. it is a non-human antibody. In case of engineered antibodies or antibody fragments (e.g. scFv or Fab) it is easy to add such sequences which provide specific recognition sites for secondary antibodies by genetic engineering (Kontermann et al., 2001, Howard and Kaser, 2006, see above). As known in the art, further to secondary antibodies, antibody fragments and derivatives other recognition molecules can be used, as well.

In an alternative method the binding of patient antibodies can by detected by a molecule specifically recognizing such antibodies, e.g. a secondary antibody. In this case specific binding of patient celiac autoantibodies to the celiac epitope is detected via the replacement of said antibodies by the test compound and thereby the reduction of the signal measured at the maximum binding.

The invention also relates to kits for performing the diagnostic methods as outlined above.

The kits of the present embodiment can comprise the constituents of the kits as described above with the difference that instead of or further to a test protein a test compound as defined herein is comprised in said kit.

In a preferred embodiment of the invention the test compound as defined in the present invention can be added to known assay kit useful as a Tissue Transglutaminase Antibody Assay useful in the detection of celiac disease. Thereby, such an assay can be used as a displacement assay, as taught herein which is useful to exclude false positive findings. The assay shall be performed in the presence of a test compound of the invention. Either displacement of the test compound by patient autoantibodies or displacement of patient autoantibodies by the test compound can be monitored depending on the method of detection. Such assay kits, so called second-generation human tissue transglutaminase antibody assays are reviewed by van Meensel et al [Clinical Chemistry 50:11 2125-2135 (2004)]. Assay conditions can be optimized starting from the manufacturers' guidelines, while the procedure basically can be performed as described.

The invention also relates to uses of the test compounds of the subject invention in the diagnosis of a gluten induced autoimmune disease as disclosed herein.

In a preferred embodiment the test compound of the present invention is different from an antibody, antibody fragment or derivative disclosed in any of the following documents: Sblattero et al. Eur J Biochem. 2002 Nov;269(21):5175-81, Nakachi K et al. J Autoimmun. 2004 Feb;22(1):53-63. Seissler et al. Clin Exp Immunol. 2001 Aug;125(2):216-21], provided that said antibody is capable of binding, preferably specific binding to the main celiac epitope. Alternatively every antibodies, antibody fragments or derivatives disclosed in one or all or any combination of the above publications or the diagnostic use thereof are excluded from the scope of the present invention.

### Treatment Options of a Gluten Induced Autoimmune Disease

With the relatively high prevalence and development of easier diagnostic tools for diagnosing celiac disease there are more and more people found to be affected and there is a growing demand for alternative therapies besides gluten-free diet. The diet is effective but may severely interfere with the individual's social activities and lifestyle. Since there is no mouse model for the disorder, several attempts were made to develop good animal models. At the level of symptoms gluten fed rabbits [March JB, Dig Dis Sci. 3;48(3):608-10], juvenile rhesus macaques [Bethune MT et al, PLoS ONE. 2008;3(2):e1614.], Irish setter dogs [Batt RM et al, Res Vet Sci. 1984;37(3):339-46.; Hall EJ, Batt RM, Gut. 1992;33(2):198-205.] and a mouse model for dermatitis herpetiformis [Marietta E et al, J Clin Invest. 2004 Oct;114(8):1090-7.] appeared to be promising. However, none of these models produced anti-TG2 antibodies apparently an essential hallmark of the human disease. That is why the testing of new potential therapeutics seems not to be easy and should be done on simplified biological systems, like cell cultures.

The present invention also provides methods for testing candidates for their positive effect. The identification of the main celiac epitope and the surprising finding that patient autoantibodies in each examined case bind to this epitope region raised the hope that an adverse effect of celiac autoantibodies can be treated by a compound specifically inhibiting their binding to the main celiac epitope.

The binding of the autoantibodies can be avoided by several mechanisms, like blocking peptide or antibody, which interfere with the celiac antibodies.

Thus, one option is masking of the epitope which also can be achieved for example with a peptide designed to the site of the epitope or to the specific binding part of the antibody. According to an embodiment a therapeutic compound is prepared, said compound carrying a mimotope, i.e. a highly similar patch of molecular surface than the main celiac epitope with the same or similar immunological properties preferably said compound having a structure being different from the amino acid sequence of tissue transglutaminase. Such a compound could bind to a patient autoantibody and block or inhibit its binding to the main celiac epitope.

Alternatively, the therapeutic compound of the present invention is a compound having the binding properties of Mab885. More specifically, said compound being capable of binding to essentially analogous or equivalent surface part of a TG family protein to which Mab885 binds.

Another option is to apply a compound, e.g. an antibody, the binding site of which should overlap with the epitope of celiac antibodies and after its binding it would hide the epitope from the celiac antibodies. This antibody should not be harmful, i.e. should not have the same effects as the binding of the disease antibodies (e.g. enhancing the activity of the enzyme). We can demonstrate that the binding of celiac serum IgA antibodies to human recombinant TG2 can be inhibited by purified IgG antibodies derived from patients with celiac disease and IgA deficiency. Successful examples of treating complex immune diseases by interfering monoclonal antibodies are numerous, like Chron's disease, rheumatoid arthritis, systemic lupus erythematosus etc. and targeting even very downstream players in the immune or inflammatory reaction (e.g. TNF alfa) is highly effective in a major proportion of patients. [Robak et al, Curr Drug Targets. 2009;10:26-37., Lees CW. Aliment Pharmacol Ther. 2009 ;29:286-97.]

Thus, in this embodiment, as compared to the compounds described in the chapter Diagnostic Methods and Kits based on Antibody Displacement a further requirement for this compound is that it should not have the adverse effect a celiac autoantibody has. A possible test for that is measurement of activity of a transglutaminase. If transglutaminase activity and/or GTPase activity and/or fibronectin binding is/are essentially maintained or left unaltered by the therapeutic compound, and if celiac autoantibodies are effectively displaced thereby, then the therapeutic compound can be considered as an effective candidate.

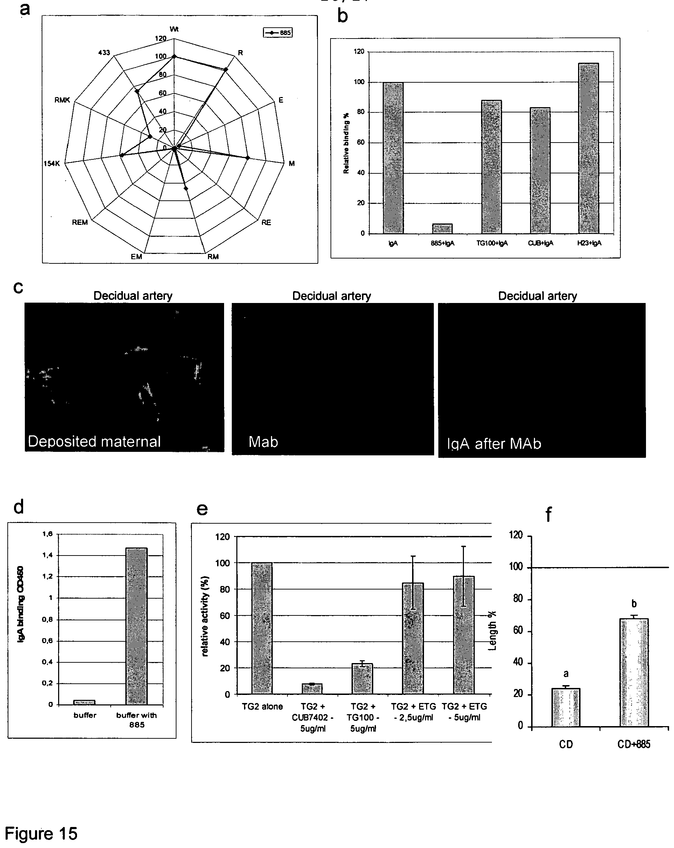

An alternative selection tool is to select for binding in endomysial pattern and displacement of patient antibodies in endomysium detected by immunofluorescence as described in the Examples. As will be understood any tissue samples like placenta, small intestine or liver can be used for this purpose. Upon testing 13 already available monoclonal anti-TG2 antibodies [Korponay-Szabo et al. JPGN 2008] it was surprisingly found by the present inventors that one of these antibodies had an epitope binding site that overlapped with celiac disease antibody binding (Figure 15). The binding of Mab 885 was completely abolished when point mutant E153S was applied whereas mutations on the beta-sandwich domain and the N-terminal beta-barrel domain did not interfere with the binding. Moreover, Mab 885 could antagonize biological effects of celiac antibodies as demonstrated herein on a HUVEC cell culture. It is at hand of the skilled person to establish an assay with other type of cell or tissue cultures such as epithelial, endothelial, fibroblast, muscle or nerve cell or tissue cultures. Thus, the invention relates to a preferably two-partite test of a compound binding to a main celiac epitope on a test protein which is an active transglutaminase, wherein i) an activity of the transglutaminase is tested and/or ii) a cell or tissue culture test is performed to decide whether the compound can reverse any adverse effect of a celiac autoantibody. If activity of the transglutaminase essentially remains and the adverse effect is essentially reversed than the compound is considered as a therapeutic compound for the purpose of the present invention.

Thus, it is contemplated that an antibody or other therapeutic compound binding mainly or entirely on a part of the celiac epitope which is localized only either to the beta-sandwich domain or the core domain can be a good candidate for a therapeutic compound.

Monoclonal antibodies can be produced by e.g. the traditional hybridoma technique or by the phage display method. Latter methods are reviewed by Schmitz U et al. [Placenta 21 Suppl A: S106-12 (2000)]. There are a number of firms offering custom antibody services so a set of antibodies is easily available once the epitope is known. Such services can be ordered e.g. from GL Biochem Ltd. (Shanghai, CH), LC Sciences (Houston, TX, USA) or LAMPIRE Biological Laboratories (Pipersville, PA, USA), FusionAntibodies (Pembroke Loop Rd. BT17 0QL Northern Ireland).

Preparation of antibodies, antibody fragments and modified versions thereof are within the skills of a person skilled in the art and can be performed as described above.Further, therapeutic monoclonal antibodies can also be produced by immunizing the mice by homologues or mimotopes of the celiac epitope. A celiac epitope for this purpose can be built up on the surface of an originally non antigenic protein, preferably on a member of the TG superfamily, using molecular modelling and simulations [see references and programs cited herein]. Monoclonal antibodies produced against the mimotope of the celiac epitope are expected to bind to intact TG2 in the desired manner. Such antibodies also could further be selected by the panel of mutants and native TG2 by their differential binding.

### EXAMPLES

### EXAMPLE 1

### MATERIALS AND METHODS

### 1.1 Molecular modelling

Residues 1-14, 44-55 and 123-132 were missing in the crystal structure of human TG2 (PDB code: 1KV3) [Liu S et al, Proc Natl Acad Sci USA. 2002;99(5):2743-7.] however, the corresponding regions were visible in the TG3 structure (PDB code: 1VJJ,) [Ahvazi B et al., EMBO J. 2002;21(9):2055-67.], so this was used for modelling the full-length TG2. Homologous model was built by Modeller [Sali A, Blundell TL, J Mol Biol. 1993;234(3):779-815.] using the multiple template option of the program. Graphical analysis was made on Silicon Graphics Fuel workstation using Sybyl program package (Tripos, St. Louis, MO), and a study was made to search for amino acids that are located near enough to each other but belong to different domains. Particularly, the interface of the core domain with the N-teminal (I) domain and the interface of the core domain with the C-terminal (IV) domain were investigated. Preference was given to charged amino acids based on observations with Ca²⁺-binding mutants of TG2.

A set of amino acids was suggested by the present Inventors that might take part in the build-up of the celiac epitope, such as:
Arg¹⁹, His²², Glu¹⁵³, Glu¹⁵⁴, Arg¹⁵⁶, Arg⁴³³, Glu⁴³⁵, Met⁶⁵⁹, Leu⁶⁶¹.

It has also been observed that Arg¹⁹, Glu¹⁵³, Met⁶⁵⁹, are relatively close to each other
Glu¹⁵³-12.9 Å
Arg¹⁹ Met⁶⁵⁹-7.7 Å
Glu¹⁵³ Met⁶⁵⁹-16.8 Å

All these residues are located at the surface of the molecule and were assumed to possibly form a common conformational epitope. Position and effects of changes concerning further amino acids were evaluated as needed by experimental results.

### 1.2 Generating TG2 mutants

In order to obtain His-tagged human recombinant TG2, DNA construct encoding TG2 gene (pGEX-2T-TG2; Ambrus A et al, 2001) was used as a template in polymerase chain reaction, which was performed with specific primers (according to the Ek/LIC Cloning Kit, Novagen); oligonucleotide primer 1, 5'- gac gac gac aag atg aga att cag acc atg gcc gag gag ctg g - 3', and primer 2, 5'- gag gag aag ccc ggt tga att cgg tta ggc ggg gcc aat gat gac - 3'. The His-tagged TG2 was generated by subcloning of PCR-amplified DNA into pET-30 Ek/LIC Vector.

The TG2 mutants were generated according to the QuickChange Site-Directed Mutagenesis Kit (Stratagene) with the His-tagged TG2 construct in pET-30 Ek/LIC Vector (mentioned above) as a template. A pair of oligonucleotide primers containing the desired mutations was designed for the mutants. After the PCR reaction the parental strand was removed by DpnI digestion. Mutations were confirmed by DNA sequencing using the ABI PRISM® 3100-Avant Genetic Analyzer.

Serine was used for replacement instead of the more conventional Ala because all investigated positions were located at the surface of the molecule, and hydrophobic portion introduced by Ala-mutagenesis may cause folding problem.

The primers used for mutagenesis are listed in Table 1.

**Table 1**

| | |
|---|---|
| FORR19ShTG2 | 5'-gct gga gac caa tgg cag cga cca cca cac ggc cg-3' |
| REVR19ShTG2 | 5'-cgg ccg tgt ggt ggt cgc tgc cat tgg tct cca gc-3' |
| FORE153ShTG2 | 5'-gct gtg tac ctg gac tcg agc gag gag cgg cag g-3' |
| REVE153ShTG2 | 5'-cct gcc gct cct cgc tcg agt cca ggt aca cag c-3' |
| FORM659ShTG2 | 5' -cct gct gcc gct cca cag cgg cct cca caa gct gg- 3' |
| REVM659ShTG2 | 5' -cca gct tgt gga ggc cgc tgt gga gcg gca gca gg -3' |
| FORE158LhTG2 | 5'-gga aga gga gcg gca gct gta tgt cct cac cc- 3' |
| REVE158LhTG2 | 5'-ggg tga gga cat aca gct gcc gct cct ctt cc- 3' |
| FORE158QhTG2 | 5'-ggaagaggagcggcagcagtatgtcctcacc-3' |
| REVE158QhTG2 | 5'-ggtgaggacatactgctgccgctcctcttcc-3' |
| FORE154KhTG2 | 5'-cct gga ctc gga aaa gga gcg gca gg- 3' |
| REVE154KhTG2 | 5' -cct gcc gct cct ttt ccg agt cca gg- 3' |
| HTGCHZAPDXEEEXXE2 (Site 4) | 5'-ggg tga gga cat act gct gcc gct gct gct gcg agt tca ggt aca cag c-3' |
| HTGCHZAPDXEEEXXE1 (Site 4) | 5'- gct gtg tac ctg aac tcg cag cag cag cgg cag cag tat gtc ctc acc c -3' |

TG2 sequence used was as disclosed in UniProtKB/Swiss-Prot Database, ID: P21980 (TGM2_HUMAN) which is incorporated herein by reference.

Based on said sequence further primers can be prepared by the same method.

### 1.3 Expression and purification of TG2 proteins

Rosetta 2™ cells (Novagen) were transformed with the expression vectors and grown in LB at 37 °C to an OD₆₀₀ 0.6-0.8. To induce the expression of His-tagged proteins, the cultures were grown for 5h at 20 °C in the presence of 0,3 mM isopropyl P-D-thiogalactoside (IPTG) then the cells were harvested by centrifugation at 4 °C.

All purification steps were performed on ice. The cells were resuspended in lysis buffer [50 mM sodium phosphate (pH 7.4), 500 mM NaCl, 5 mM imidazole, 20 mM β-mercaptoethanol, 10% (v/v) glycerol, 1% (v/v) Triton X-100] with 1 mM phenylmethylsulfonyl fluoride (PMSF). Cell lysis was performed by sonication followed by centrifugation for 35 min at 20000 g. Supernatants were loaded onto a 2 ml Ni Sepharose High Performance column (GE Healthcare Bio-Sciences AB) in the presence of 20 mM imidazole. The column was washed with 80 mL wash1 buffer [50 mM, sodium phosphate (pH 7.4), 800 mM NaCl, 20 mM imidazole, 20 mM β-mercaptoethanol], then with 40 mL wash2 buffer [50 mM, sodium phosphate (pH 7.4), 500 mM NaCl, 30 mM imidazole, 20 mM β-mercaptoethanol]. The protein was eluted with 12 mL wash2 buffer containing 250 mM imidazole. The eluent was concentrated with Amicon Centricon-YM 50 MW (Millipore) and the buffer was exchanged three times to storing buffer [20 mM Tris-HCl (pH 7.2), 150 mM NaCl, 1 mM DTT, 1 mM EDTA, 10% (v/v) glycerol].

Domain deletion mutants were generated and expressed as described [Korponay-Szabo I et al., J Pediatr Gastroenterol Nutr. 2008;46:253-61.

### 1.4 Characterization of mutant proteins

### A) Western blotting

SDS/PAGE was performed according to standard techniques. TG2 proteins were separated by SDS/PAGE and transferred to Polyvinylidene Fluoride (PVDF) membrane (Millipore). The membrane was blocked with 1% bovine serum albumine (BSA) in 50 mM Tris-buffered saline containing 0,1% (v/v) Tween 20 (TTBS) for 1h at room temperature, followed by incubation with goat polyclonal anti-TG2 antibody (Upstate) diluted 1:20000 in TTBS or with mouse monoclonal anti-TG2 antibody TG100 (NeoMarkers) diluted 1:15000 for 1h at room temperature. After extensive washing with TTBS the membrane was incubated with anti-goat antibody or with anti-mouse antibody conjugated with horseradish peroxidase (HRP) (Sigma), 1:30000 in TTBS, for 1h at room temperature. The bands were revealed by Chemiluminescent ECL Detection System (Millipore).

All mutant TG2s could be expressed in bacteria successfully, and there were only slight differences in yield of the protein and in the intensity of the protein bands compared to the wild type (Wt) enzyme (Figure 1A). This phenomenon is probably due to the different properties of the mutants, which could affect the expression or purification efficiency. The Coomassie brillant blue-staining of the SDS gel showed purity greater than 80% for all expressed proteins.

### B) Transglutaminase activity

Transglutaminase activity was measured with microtiter plate assay based on the incorporation 5-(biotinamido) pentylamine into immobilised *N,N-*dimethylated casein (DMC) [Kiraly R et al, J Autoimmun. 2006;26(4):278-87.].

The wells were coated with 4 mg *N,N-*dimethylated casein (Sigma) in 100 mM Tris/HCl pH 8.0 overnight, 4°C. After washing, the wells were blocked with 0,5% milk powder solution for 30 min at room temperature. Then the reaction mixture was added (in a total volume of 200 µL 100 mM Tris/HCl pH 8.0) containing 10 mM dithiothreitol, 1 mM *N*-(5-aminopentyl)biotinamide (Molecular Probes, Eugene, OR), 5 mM CaCl₂ and 0.5 µg TG2. The reaction was performed at 37°C for 30 min. The plate was washed with 200 mM EDTA pH 8.5 and incubated with 0.42 µg/well streptavidine-alkaline phosphatase (Sigma). The enzyme reaction was revealed by adding 200 ml of 25mM p-nitrophenyl phosphate (Sigma) and measuring absorbance at 405 nm. Enzyme activity values were obtained from ΔA405/min of colour development between 10 and 30 min. Reaction blanks contained 10 mM EDTA and no added CaCl₂.

Mutant R showed Ca-dependent TGase activity, which was ∼30% higher than wild type (Wt) transglutaminase (Fig1B). All of the other mutants showed decreased, but measurable TGase activity (Figure 1B). Control mutant (D) in which domains I-III-IV were present, but the catalytic core (II) domain was missing, did not show TGase activity.

### C) GTPase activity assay

GTPase activity was determined by the charcoal method [Kiraly R et al, J Autoimmun. 2006;26(4):278-87.]. The 100 µL reaction mixture contained 2 µg of recombinant wild type or mutant TG2 in 50 mM Tris-HCl, pH 7.5, 4 mM MgCl2, 1 mM DTT, 1 mM EDTA, 10% (v/v) glycerol, 9.9 mM GTP and 0.1 mM [g-32P]GTP (3000 Ci/mmol, Institute of Isotopes Ltd., Budapest, Hungary). The reaction was performed at 37°C for 30 min and was stopped with 700 µL of 6% (w/v) activated charcoal in ice-cold 50 mM NaH₂PO₄ pH 7.5. The mixture was centrifuged and released [³²P] Pᵢ was determined by counting of 150 µL samples of the supernatant. Blank was determined without the enzyme.

The specific activity (cleaved pmol GTP/min/mg enzyme) of the Wt enzyme was set to 100%. The GTPase activities of mutant RM, EM and REM were more than two fold higher than the activity of the Wt (Figure 1B). The single and the other double mutants showed 24-50%.

### D) Fibronectin-binding capacity of the mutants (Fibronectin-TG2 ELISA)

Microtiter plates (ImmunoPlate Maxisorp, Nunc, Denmark) were coated with 0.3 µg human fibronectin (FBN) (Sigma) diluted in bicarbonate buffer for 1h at room temperature. The plates were washed 3 times with TTBS containing 10 mM EDTA (TTBS+EDTA) and were incubated with 0.8 µg TG2 in TBS containing 5 mM CaCl₂ and 0.1% (v/v) Tween 20 (Ca-TBS-Tween) for 1h at room temperature. Monoclonal antibody [TG100, 1:500 in TTBS+EDTA; (NeoMarkers, Fremont, CA)] was incubated for 1 h at room temperature. Plates were washed and incubated with HRP-conjugated anti-mouse IgG (1:4000, Sigma) for 1h at room temperature. The colour reaction was developed by adding 100 µL 3,3',5,5'-tetramethylbenzidine substrate (Sigma) and then stopped with 50 µL 1 N H₂SO₄. The absorbance was read at 450 nm. Standard curves were prepared from dilutions of TG100 monoclonal antibody for each mutant.

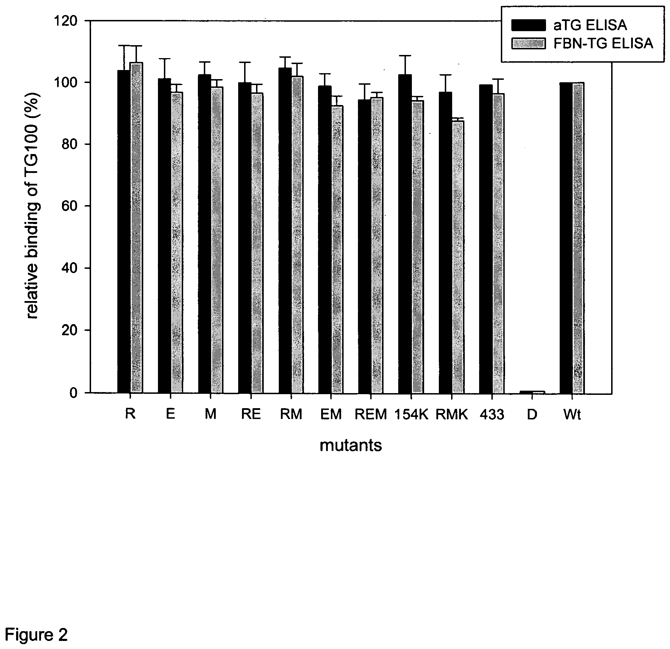

The mutants reacted with monoclonal mouse TG100 antibody in the same extent as Wt (Figure 2.) when bound to FBN. These data support that the mutants possessed proper conformation in order to be used for further measurements with celiac sera.

### 1.5 Patients

Unless different conditions specifically mentioned serum samples from altogether 216 celiac disease patients aged 0.9-78 years, of them 56 with selective humoral IgA deficiency (total serum IgA <0.05 g/l), were used; all had diagnosis by small bowel biopsy showing at least Marsh grade III villous atrophy. Samples were collected before the treatment and from 22 of them during a follow-up of up to 17 years. 11 subjects initially had preserved small bowel villous architecture but subsequently developed celiac type villous atrophy during prospective follow up (latent cases). Included non-celiac controls had normal small bowel villous architecture.

### 1.6 Antibodies and single chain variable fragments (ScFvs)

Mouse monoclonal antibody clone Mab885 (Cl. 885A) of Phadia has been deposited according to the Budapest Treaty by Phadia AB (Box 6460 751 37 UPPSALA, Sweden, Visiting address: Rapsgatan 7P 754 50 Uppsala) under Accession number DSM ACC3053 as indicated in the International form at DSMZ - Deutsche Sammlung von Miroorganismen un Zellkulturen GmbH, Inhoffenstr. 7 B, D-38124 Braunschweig, Germany. Fusion partner of Mab885 is SP2/0 (rat) cl. 321B.

Conditions for cultivation of Cl. 885A are as follows:
Culture Medium: F-DMEM + 4 mM L-glutamine + 5% foetal calf serum
Preferred Temperature: 37 °C
Gaseous Phase: 6% CO2
Optimal split ratio: about 1/10.

Long term storage is preferred in F-DMEM + 10% foetal calf serum + 7.5% DMSO, at -150 °C. 1.7 Anti-TG2 ELISA

The ELISA measurements were performed similar to the testing described previously [Sulkanen, S. et al., Gastroenterology 115, 1322-1328 (1998)]. Briefly, microtiter plates (ImmunoPlate Maxisorp, Nunc) were coated with 0.6 µg TG2 in 100 µL of TBS containing 5 mM CaCl2 (pH 7.4). The plates were washed 3 times with TTBS containing 10 mM EDTA (TTBS+EDTA). All antibodies were diluted in TTBS+EDTA. Serum samples (diluted 1:200) or monoclonal antibodies (TG100, 1:500; NeoMarkers) were incubated for 1 h at room temperature. Plates were washed and incubated either with HRP-conjugated rabbit anti-human IgA or IgG (1:5000; Dako) followed by HRP-conjugated anti-mouse IgG (1:5000; Sigma) for 1h at room temperature. The color reaction was developed by adding 100 µL 3,3',5,5'-tetramethylbenzidine substrate (Sigma) and then stopped with 50 µL 1 N H2SO4. The absorbance was read at 450 nm. Standard curves were prepared from dilutions of TG100 monoclonal antibody for each mutant and binding of other antibodies was calculated by 4-parameter fit if binding to Wt TG2 was 100%. All serum samples were examined in duplicates.

### 1.8 Competition ELISA assays

Microtiter plates were coated with 0.6 µg or less wild type (Wt) TG2 in 100 µL of TBS containing 5 mM CaCl2 (pH 7.4). Wells were incubated with celiac serum (1:800) and increasing amounts of purified total celiac IgG antibodies (dilution 1:200-1:50) or IgA deficient patient serum and binding of IgA and IgG antibodies were detected. In further competition assays celiac serum was added to the plate together with increasing amounts (up to 18 µg/well) of monoclonal mouse antibodies (885, CUB7402, H23) and bound IgA was measured.

### 1.9 Immunofluorescent studies

Unfixed frozen sections were incubated with anti-human IgA or IgG (DAKO) to detect in vivo-bound immunoglobulins alone or in combination with double labelling for TG2 carried out as described previously [Korponay-Szabo, I.R. et al. J. Pediatr. Gastroenterol. Nutr. 31, 520-527 (2000), Korponay-Szabo, I.R. et al. Gut 53, 641-648 (2004)]. Detecting competition with TG2-specific MAbs was done by adding MAbs to the tissue for 30 minutes in PBS, removing the incubation solutions and testing them for patient IgA and mouse antibodies. Binding of the MAbs was detected by Alexa-Fluor 594-conjugated or Rhodamine conjugated anti-mouse antibodies.

### 1.10 HUVEC preparation and cell culture experiments

Human umbilical vein endothelial cells (HUVECs) were prepared from fresh umbilical cords and cultured by standard techniques [Palatka K. et al. World J. Gastroenterol. 12, 1730-1738 (2006)]. For the analysis of antibody effects on cell differentiation, HUVECs were cultured on collagen I for 48 hours [Myrsky, E. et al. Clin. Exp. Immunol. 152, 111-119 (2008)] and length of endothelial tubules was analyzed by *Image J* software. Ten different pictures were taken from three wells with 50,000 cells/well.

### 1.11 Statistical analysis

Data from the ELISA measurements were analyzed using GraphPad Prism Software and STATISTICA. For comparison of antibody binding to mutant TG2, data were analyzed using repeated measures ANOVA followed with Dunnett's Multiple post test, one way ANOVA followed by Tukeys post test, or Kruskal-Wallis test followed by Dunn's multiple comparison test as appropriate. A p value < 0.05 was considered significant.

### EXAMPLE 2

### PRELIMINARY EXPERIMENTS TO LOCALIZE THE CELIAC EPITOPE

### Binding of celiac antibodies is related to a calcium binding site of TG2

During examination of Ca2⁺ binding of human TG2 we identified two negatively charged surface patches on the core domain that are near to each other and could serve as Ca²⁺ binding sites. Multiple mutations of acidic glutamate and aspartate residues to neutral glutamine and asparagine in Site4 (151DSEEERQE158 →151NSQQQRQQ158) or Site 5 (434DERED438→434NQRQN438) led to the decrease of the number of bound Ca²⁺ ions per TG2 molecule from 6 to 3 and also diminished the binding of celiac patient serum samples substantially (Site 4, residual binding compared to wild TG2 11.6±-8.5%) or moderately (Site 5, 51.3±16.0%) in enzyme-linked immunoassay (ELISA). These ELISA assays were performed with serum samples from 62 celiac disease patients (age at diagnosis 1-42 years) and 20 disease control subjects (age 1-17 years). The serum samples were collected at the time of initial clinical diagnosis, celiacs having anti-TG2 and endomysial antibodies in serum and having severe villous atrophy by small bowel biopsy, whereas controls having normal small bowel architecture and negative serology results, respectively..

For performing the ELISA, wild-type and mutant TG2 proteins were diluted in Ca-TBS-Tween and then coated to Maxisorp plate (0.6 µg/well) at room temperature for 1 hour. Then the ELISA was conducted as described in example 1, by adding patient antibodies at 1:200 dilutions in assay buffer and using rabbit anti-IgA polyclonal antibodies (DAKO, diluted 1:4000 in assay buffer) to detect the signal. The binding to wild-type TG2 was set to 100%, and binding of patient samples was calculated as proportional to this, using a TG100 monoclonal antibody reference to control that plates have comparable amounts of wild-type and mutant TG2 antigens.

In a liquid phase version of the assay microtiter plates (Maxisorp, Nunc, Denmark) were coated with 0,6 µg wild type (Wt) TG2. Celiac sera were preincubated with different amount of Wt or mutant TG2 in Ca-TBS-Tween for 10 min at room temperature. This mixture was added to the wells and incubated for 1h at room temperature. The coated Wt TG2-bound IgA antibodies were detected similarly as in anti-TG2 ELISA.

In search of the anchor residues that could form a celiac epitope in Site 4, we prepared mutant TG2 molecules bearing D151N, E153Q, E154Q, E155Q, E158Q and E158L mutations separately. From these, mutation of residues 153 or 158 significantly decreased (p<0.0001) the binding of celiac antibodies (Figure 3A) whereas the other changes did not have any effect. As E158 is not surface exposed, these results showed the importance of Glu¹⁵³, that could form an anchor point for the antibody binding. However, the mutation of Glu to Gln at this position was not sufficient to completely abolish patient antibody binding, or there might be also the possibility, that Glu¹⁵³ needed cooperativity of other surface parts to form a functional epitope.

Site-directed mutagenesis was performed also at Site 5. Analyzing surface properties, two candidate amino acids (Arg⁴³³ and Glu⁴³⁵) with charged side-chains in the middle of the putative epitope of Site5 were changed to serine (Mutant 433), because these were the nearest to Site 4. Mutant 433, however, did not show altered binding with celiac disease patient serum samples. In addition, monoclonal mouse anti-TG2 antibody H23 [Korponay-Szabo I et al. J Pediatr Gastroenterol Nutr. 2008;46:253-61.] which has its binding epitope at Site 5 did not interfere with celiac disease antibodies in their binding to wild-type TG2 when co-administered in excess together with celiac disease serum samples in ELISA.

We also prepared a transglutaminase active site mutant (C277S), but this protein bound celiac antibodies similarly well as wild type TG2.

### Calcium ions do not form part of the celiac epitope(s)

Site 4 is a Ca²⁺ binding site and previous clinical laboratory studies indicated that celiac antibodies recognize TG2 preferentially in the presence of Ca²⁺ [Sulkanen S et al, Gastroenterology 1998;115:1322-8.]. Since surface amino acids involved in the coordination of Ca²⁺ at Site 4 or Site 5 were not entirely responsible for celiac antibody binding, we explored whether calcium ions themselves also contribute to the epitope. For this purpose, we utilized TG2 preparations as antigen after exhaustive dialysis in ethylene-diamine-tetraacetate (EDTA) and we used EDTA in all buffer solution in ELISA. This procedure, however, only can remove all bound Ca²⁺ from the protein when its strong Ca²⁺ binding site (Site 1, amino acids 229-233, homologous to the strong Ca²⁺ binding site of TG3) is mutated. This Site 1 mutant where Site 4 and Site 5 aminoacids were intact was a good antigen for celiac antibodies and bound them equally well (≥100%) in ELISA both in the presence and absence of Ca²⁺. This result excludes the structural role of Ca ions in the potential celiac epitope(s) at Site 4 as well as at Site 5.

### Existence of epitope anchor points outside the core domain

As Glu¹⁵³ was near to interfaces between domains, we tried to establish which of the domains other than the core domain might harbour further epitopes or might cooperate with Glu¹⁵³ in antibody binding. Given that earlier results with truncated TG2 fragments were contradictory in the previous art, we expressed TG2 mutants each lacking one structural domain of TG2 as described [Korponay-Szabo et al, J Pediatr Gastroenterol Nutr. 2008;46:253-61.] When these domain mutant constructs were applied in equimolar concentrations in solid and liquid phase immunoassays, we found that celiac antibody binding proceeded normally even in the complete absence of domain III (aminoacids 472-584). In contrast, lack of either domain I or II resulted in proteins that did not bind celiac antibodies at all (Figure 3B), suggesting that both of these domains have direct or indirect role in antibody binding. Also the loss of domain IV (aminoacids 585-687) affected the binding to some extent (O.D. relative to wild TG2 0.8, p<0.0001). However, it was surprisingly also noted, that the construct consisting of domains I-III-IV but not containing domain II did not bind celiac antibodies, even though these domains are known to adopt their folded forms [Hang J et al, J Biol Chem. 2005;280(25):23675-83. ; Pinkas DM et al, PLoS Biol. 2007;5(12):e327.] also independently of the core domain. Thus these results indicated that in the celiac epitope(s) the presence of the core domain anchor residues is needed for efficient binding, and so in their absence the other epitope parts were not able to form functional binding sites.

### EXAMPLE 3

### EFFECTS OF MUTATIONS IN THE PUTATIVE CELIAC EPITOPE AND RELATED SURFACE AREAS

Based on the preliminary results and computational analysis, within a larger set of amino acids 6 amino acids (R19, D151, E153, E154, E155, M659) were identified that are on or adjacent to the surface of TG2 related to Site 4 and close enough to each other to potentially form composite epitopes. These residues were changed one by one (single mutants) or in combination (double and triple mutants) by site-directed mutagenesis to serine or in case of acidic residues to their neutral homologues.

We prepared the following point mutant TG2 molecules: E153S (E), R19S (R), M659S (M) or the respective mutations in combination (D151N/E154Q/E155Q, RE, EM, RM, REM, E154K/R19S/M659S [RKM]), and tested the proteins with a large set of consecutive patient serum samples (n=76). Relative amounts of bound antibodies were calculated by comparison to a calibrator curve constructed from the concentration dependent binding of mouse monoclonal anti-TG2 antibody TG100, which has a linear epitope at aminoacids 447-538.

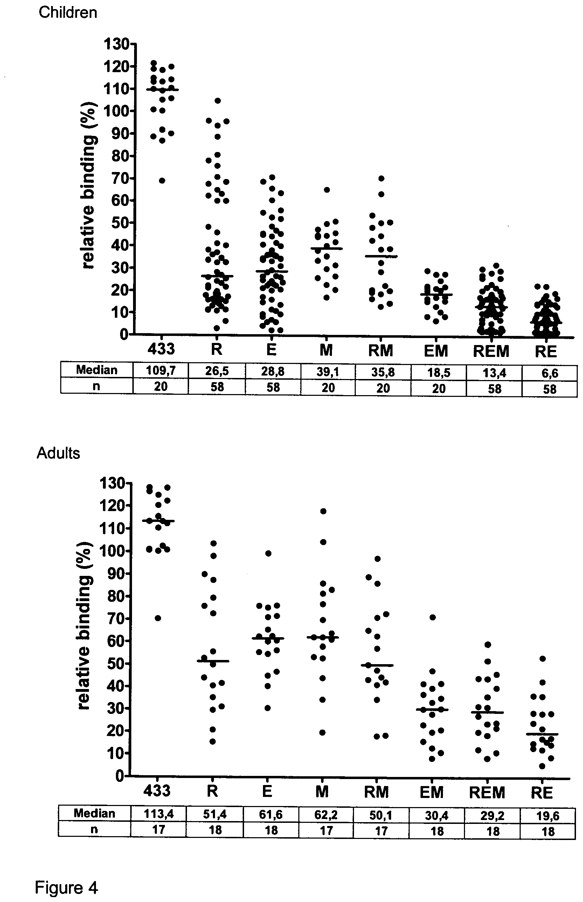

Each single mutation resulted in significant decrease in celiac antibody binding (26.5%, 28.8% and 39.1% remaining binding for R, E and M, respectively) and double and triple mutations caused proportionally changes. The D151N/E154Q/E155Q mutant also bound significantly less antibodies than its single point mutants (Figure 3A), but did not loose completely its binding ability. The RM mutant still retained 35.8% binding capacity, EM had 18.5% and REM and, surprisingly, RE were the lowest, giving 13.4% and 6.6% binding, respectively. The same binding pattern was seen both for consecutively diagnosed childhood and adult celiac patients (Figure 4), which results together pointed out the importance of the first alpha helix of the core domain in antibody binding.

Despite of the decreased binding of celiac antibodies, these mutant TG2 proteins bound normally a large set of mouse monoclonal anti-TG2 antibodies [Korponay-Szabo I et al, J Pediatr Gastroenterol Nutr. 2008;46:253-61.], showed functionality in fibronectin binding, and in either TG2-ase or GTPase assays, as shown in Example 1. Further, CD spectra analysis of selected mutants (R, S4, E¹⁵³ and E¹⁵⁸ mutants) showed that the proteins had a folded structure without significant increase (<5%) in the proportion of unordered segments.

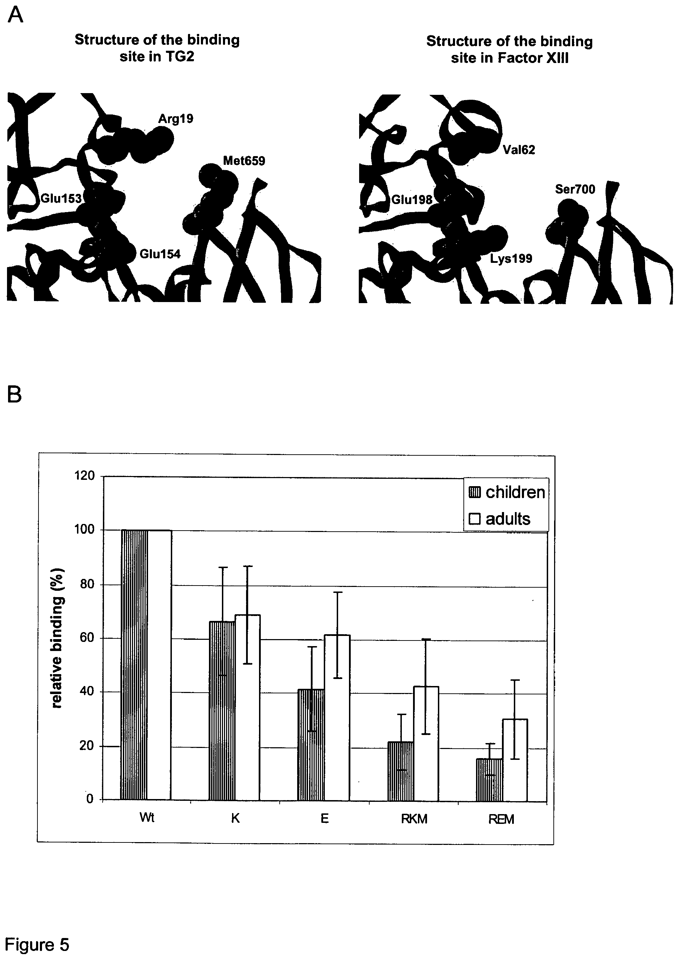

Direct binding of celiac antibodies to the surface around Glu¹⁵³ was further corroborated by modifying another core domain anchor point of the putative composite epitope. As shown above, changing Glu¹⁵⁴ to neutral Gln (E154Q) was not sufficient to alter celiac antibody binding, but change in TG2 (E154K) in combination with the R and M mutations (RKM) resulted in similarly dramatic reduction of celiac antibody binding (remaining binding 22.0%) as the REM mutation (Figure 5). This result is also supported by the fact that Factor XIII, one other member of transglutaminase family to which celiac antibodies do not bind [Sjöber et al, Autoimmunity 2002;35:357-64.] contains a positively charged lysine at the corresponding position within an otherwise very homologous surface. In contrast, library search of TG2 sequences from different species and human TGs showed that all animal TG2 proteins that are known to serve as highly sensitive antigens for the detection celiac disease antibodies in clinical laboratories by ELISA (guinea pig) or traditional immunofluorescent assays with tissue sections (monkey, rat, rabbit, mouse) do contain homologous negatively charged surfaces and anchor points at the corresponding positions (Table 2). A similar surface also can be found in human TG6.

To delineate the surface area that could contribute to the putative celiac epitope, we used the data obtained with Mutant 433 (Figure 4), which shows that the putative celiac epitope does not extend to these residues in direction of Site 5.

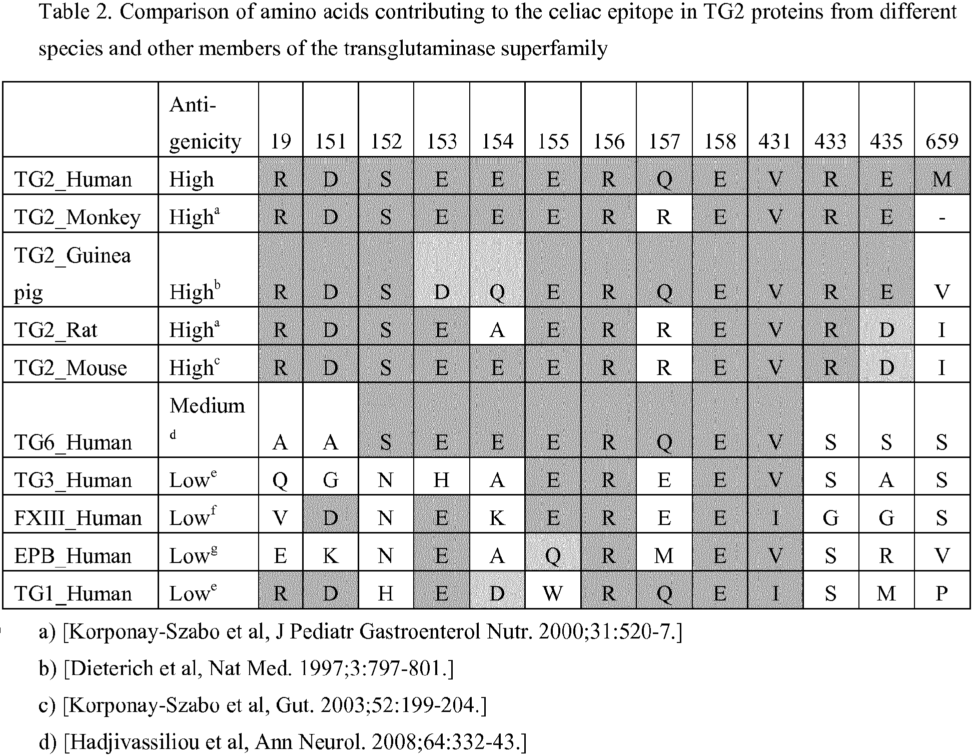

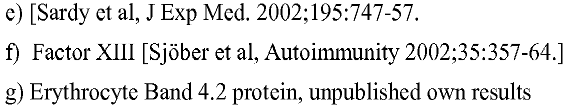

### Two anchor points sufficiently determine the celiac epitope

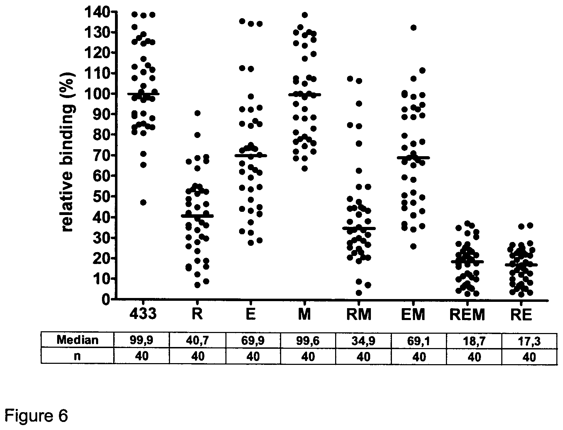

To establish the relative importance of and Met⁶⁵⁹ in celiac antibody binding, further studies were conducted with fibronectin-bound TG2 that mimics accessibility of epitopes in the extracellular matrix where antibodies primarily meet the autoantigen in patients. For this, 0.8 µg TG2/well was coated to fibronectin as described in Example 1, and bound IgA class celiac antibodies were detected by anti-IgA secondary rabbit antibodies (DAKO) diluted 1:4000 in assay buffer. Under these conditions, the mutations of Arg¹⁹ and Glu¹⁵⁴ had similar effects as when tested with TG2 directly bound to the ELISA plate. Surprisingly, the mutation of Met⁶⁵⁹ alone, however, did not alter celiac antibody binding in either children or adults (Figure 6), nor had additive effect if used combined with the other two mutations. Thus these results show that of Glu¹⁵⁴ and Arg¹⁹ together are enough for antibody binding, and thus celiac antibodies may bind to TG2 also in its catalytically active form when TG2 adopts an open-extended conformation [Pinkas DM et al, PLoS Biol. 2007;5(12):e327.] where domain IV with Met⁶⁵⁹ swings out. This is even more surprising as Arg¹⁹ is significantly closer in the closed conformation to Met⁶⁵⁹ (7.7 Ǻ) than to Glu¹⁵³ (12.9 Ǻ). On the other hand, measurements on the position of Arg¹⁹ and Glu¹⁵⁴ in the open form crystal structure (2Q3Z) did not show difference compared to the closed conformation (1KV3, 2,8-Ǻ) (Figure 7).

In liquid phase assay antibodies showed the same pattern as in solid phase ELISA supporting the finding that Glu¹⁵³ and Arg¹⁹ has major role in the antibody-binding.

### EXAMPLE 4

### OTHER MUTATIONS INDIRECTLY AFFECTING THE CELIAC EPITOPE

As shown in Figure 3A, a single point mutation on the core domain (E158Q or E158L) also severely reduced the binding of celiac autoantibodies to TG2 in ELISA. As this amino acid is not surface exposed, we conducted further in silico analysis and molecular modelling to explore the effect of this mutation. Glu¹⁵⁸ is located at the base of the first alpha helix of the core domain, and loss of the negative charge on its side-chain results in the disruption of hydrogen bonds and confers instability to the helix. This process may affect the relative position of the surface residues 153, 154 and 155 to each other or to the Arg¹⁹ on the N-terminal domain.

Evaluating also the position of the amino acids of Site 5, it was concluded that also these changes may affect the helix of Site 4 in indirect ways, but to a lesser degree.

Further, we evaluated whether changes other than the mutation of Arg¹⁹ in the N-terminal domain first alpha helix would have an effect on celiac antibody binding. We thus prepared mutant TG2 where the N-terminal first 14 amino acids of full length TG2 were deleted, that was supposed to affect also the said helix. Celiac antibodies showed a severely reduced binding to this mutant (8.4% residual binding compared to wild type TG2.

It is true for all these mutated amino acids that they do not form part of the molecular surface of the celiac epitope, however, a mutation indirectly effects said epitope.

### EXAMPLE 5

### DISEASE SPECIFICITY AND CLINICAL RELEVANCE OF THE COMPOSITE EPITOPE

### Disease specificity

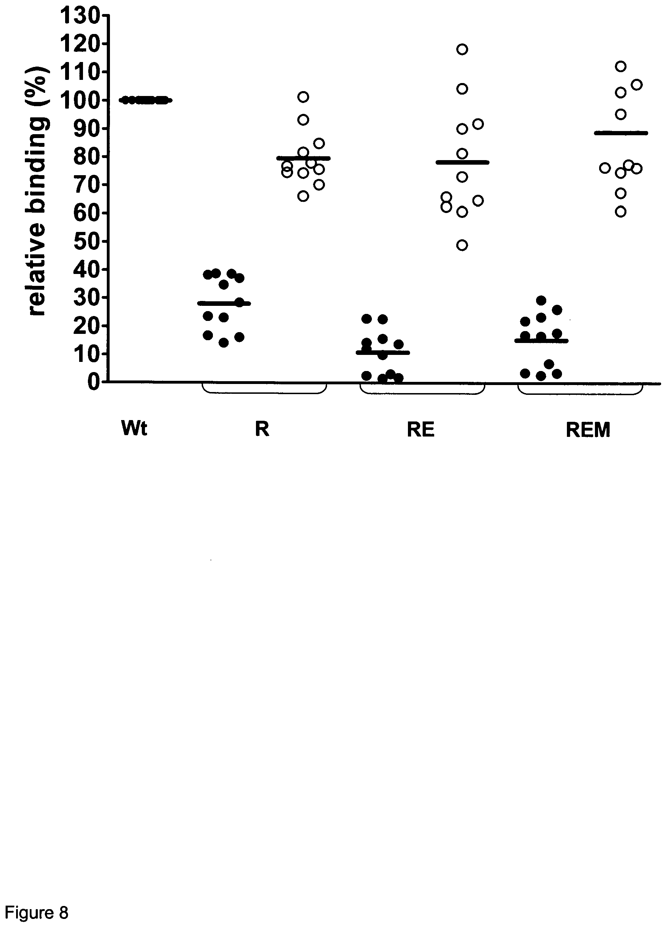

We evaluated whether the identified composite epitope is characteristic only for celiac autoimmunity. Therefore we compared the binding pattern of 11 celiac disease samples which were positive for both endomysial and anti-TG2 antibodies with that of serum samples from 11 patients with anti-TG2 antibodies in serum due to other autoimmune diseases (SLE, Sjögren's syndrome, rheumatoid arthritis). The binding pattern of the non-celiac group was clearly different and irrelated to the celiac epitope (Figure 8), and these subjects were also negative for antibodies against endomysium and deamidated gliadins. The autoimmune patients did not have malabsorption or other intestinal symptoms, and when a small bowel histology was performed (in 1 case), the small bowel was normal.

### Antibodies of different celiac patients recognize parts of the same epitope

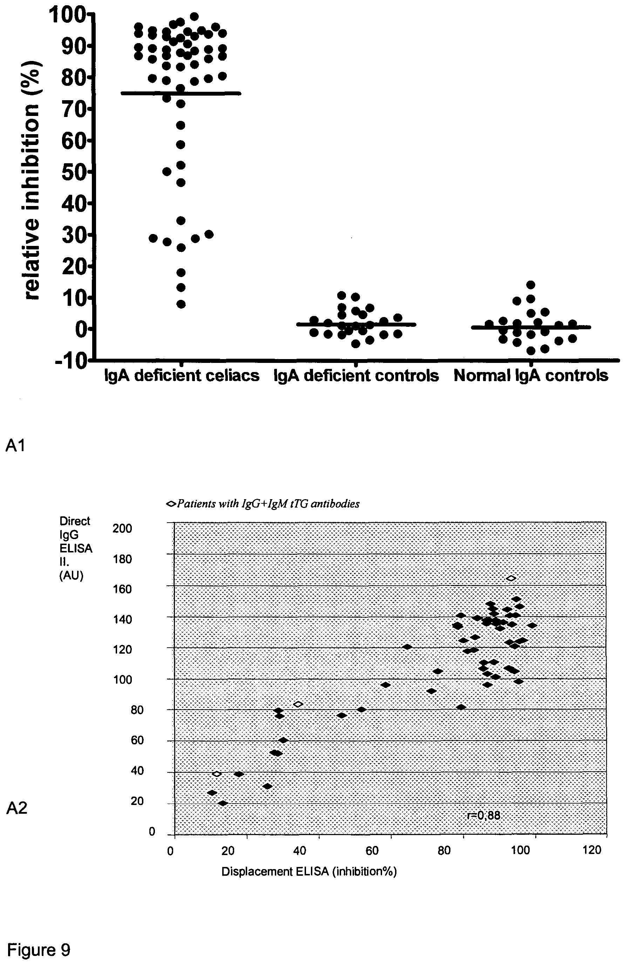

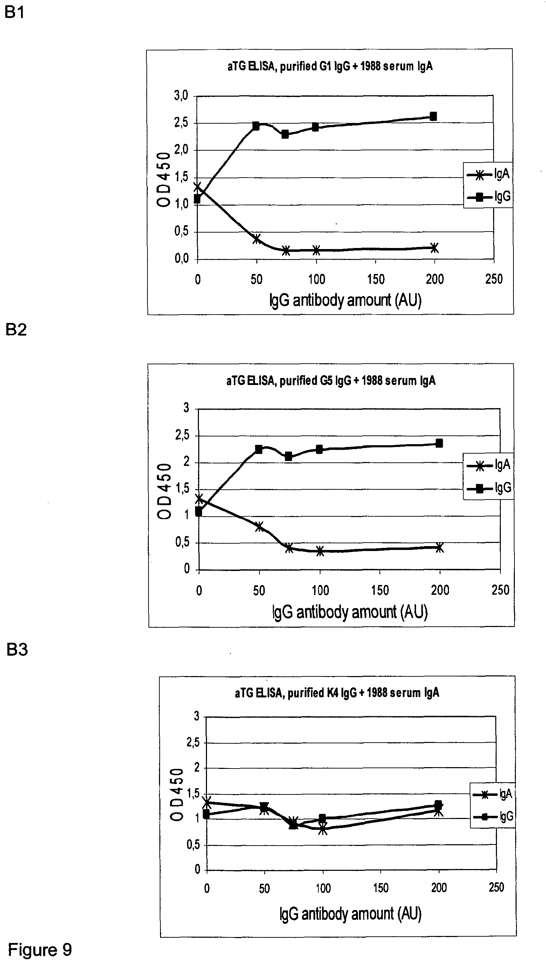

Since natural patient antibodies are polyclonal and may contain a mixture of antibodies with different epitope specificity, we next investigated whether the newly identified conformational epitope is determining antibody response in all celiac cases. Therefore we conducted competition studies with serum samples and purified IgG and IgA patient immunoglobulins. Serum samples from all investigated 56 IgA deficient celiac patients (aged 0.9-78 years) with IgG or IgM class anti-TG2 antibodies were able to displace the same IgA anti-TG2 celiac antibody from the same IgA competent celiac patient (Figure 9) while sera of 23 non-celiac IgA deficient control subjects without anti-TG2 antibodies were ineffective. The displacement effect was proportional to the concentration of IgG and IgM class anti-TG2 antibodies in the sera and was thus suitable to measure unknown non-IgA celiac antibodies in the clinical laboratory.

Although all celiac patient serum samples displayed a severely reduced reaction with REM, RKM triple and RE double mutants, their reaction showed some variability with the R point mutant where only the N-terminal anchor point (Arg¹⁹) had been changed as shown in Figure 4. However, when we performed the competition studies with purified IgG antibodies from a celiac patient whose antibodies did react with the R, we got the same competition effect for the same IgA celiac antibody as when we used other IgG celiac antibody that did not react with R (Figure 9).

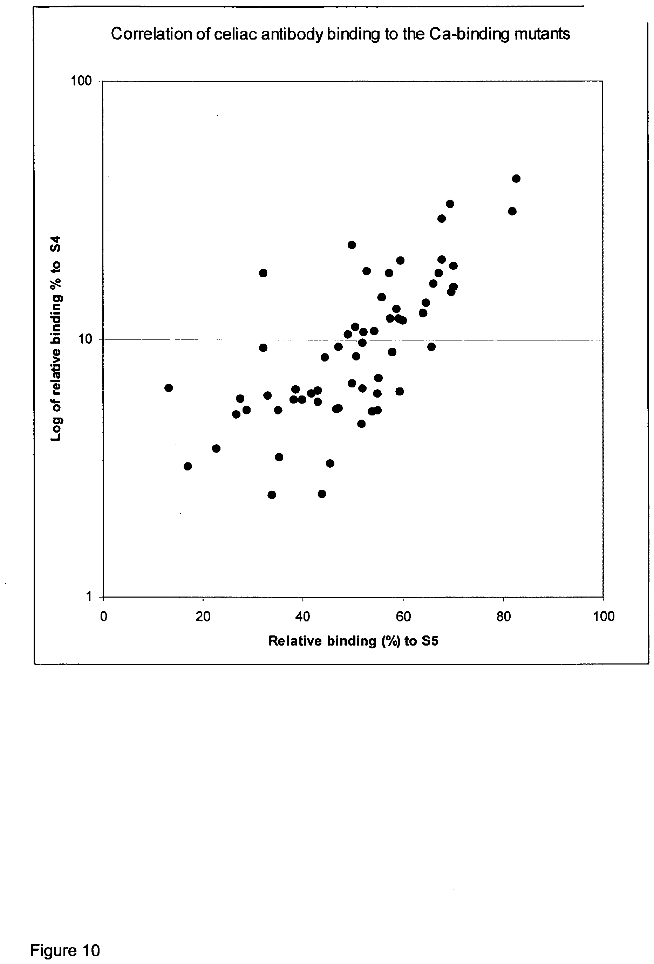

Further, when we compared the antibody binding results to the Site 4 and Site 5 Ca²⁺-binding mutants obtained with the originally investigated 62 celiac serum samples, the binding to Site 5 correlated with the logaritmic values of the binding to Site 4 for the individual samples (Figure 10). This result indicates that the binding was primarily determined by the Site 4 helix but also changes at the nearby Site 5, that is at the margin of the Site 4 surface area and can influence the position of the helix of Site 4, had proportional effect pointing to the existence of a single main binding site for all patients. This series contained also adults, for whom similar results were observed.

### Stability and evolution of the epitope binding pattern during disease development

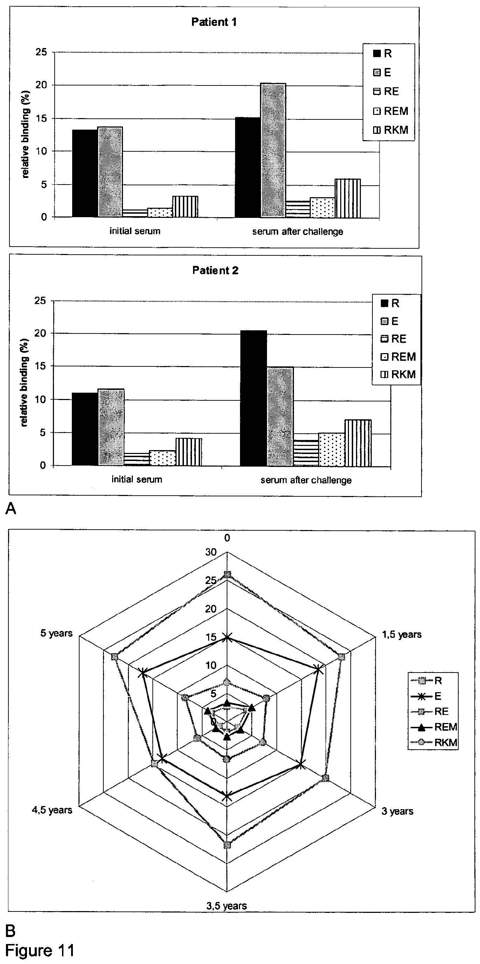

Celiac disease is a strictly gluten-dependent clinical entity and autoantibody production to TG2 is dependent on gluten ingestion, disappears and reappears with gluten elimination and reintroduction. Therefore, we investigated the epitope specificity of circulating TG2 autoantibodies of individual patients from the initially obtained and subsequent serum samples. In a series of 7 patients, in whom antibodies disappeared on diet, but gluten was reintroduced at a later time (after 1-4 years) for diagnostic gluten challenge, as used to be clinical practice in earlier times, the patients responded with antibodies of the same epitope specificity on gluten challenge. Similarly, in cases without diet compliance and constantly positive for TG2 antibodies in serum over a long time from childhood into adulthood, stability of the epitope binding pattern was observed for 3.5-14 years (Figure 11).

### Similar epitope specificity of circulating antibodies and of those in vivo- bound to patient tissues

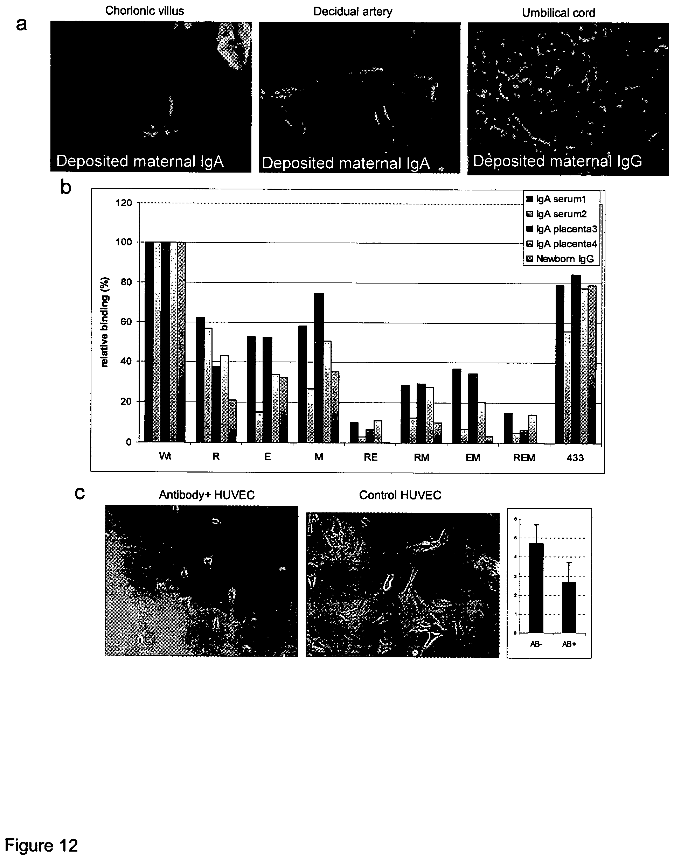

Next we evaluated whether antibodies to the composite epitope are also relevant for the clinical disease manifestations. There are several clinical observations that high avidity antibodies might be trapped in the tissues and do not circulate, explaining seronegative celiac cases in the minority of patients [Salmi TT et al, Gut. 2006;55(12):1746-53.]. The celiac antibodies deposited in tissues were present all diseased organs organs [Korponay-Szabo IR et al, Gut. 2004;53(5):641-8.] and were shown to be functional as they bound externally added recombinant TG2 [Salmi TT et al, Gut. 2006;55(12): 1746-53.], thus they might be more important for disease pathogenesis that blood antibodies. Therefore, we aimed to test whether epitope specificity of tissue-bound TG2 antibodies is similar to blood antibodies. We eluted patient IgA antibodies deposited in patient tissues from tissue sections and tested them with the panel of relevant mutant TG2 proteins after purification. In order to obtain only the functional patient antibodies that bound to the TG2 antigen and to avoid contamination of the IgA fraction by nonspecific IgA contained within plasma cells or epithelial cells that does not necessarily take part in the disease process, we chose not to use gut biopsy samples but an extraintestinal organ without local production of IgA. Such a patient tissue was easily available in sufficent amounts for biochemical studies without performing invasive procedures when we tested placenta samples from two celiac mothers who gave birth while having serologically active disease. In fact, we observed that placenta in celiac disease contains high amounts of TG2-bound maternal antibodies both in the decidual parts and on the surface of the chorionic villous structures (Figure 12). This IgA was eluted from the placenta tissues with chloroacetic acid as previously described [Korponay-Szabo I et al, Gut. 2004;53(5):641-8.9], and showed identical epitope targeting pattern to the typical pattern observed with celiac serum samples.

### Celiac antibodies targeted against the identified composite epitope cause disease in passive transfer

To assess whether the antibodies targeting the celiac epitope we identified are involved in disease phenomena, we sought a disease model where humoral and cellular components of the immune reaction could separately be investigated. Unfortunately, celiac disease is a strictly human disorder and there aren't relevant animal models where TG2-specific antibodies would be operative. Thus we explored whether natural transfer of maternal antibodies occurs in human newborns and whether this process is accompained by pathological features. These offsprings are often underweight and have more often obstetrical complications than children of normal mothers [Hadziselimovic F,et al. Fetal Pediatr Pathol. 2007;26:125-34]. Therefore, we investigated placenta, umbilical cord and serum specimens at 8 deliveries where the mother had celiac disease and human umbilical cord endothelial cells (HUVEC) were prepared. Three of these mothers had active disease with circulating TG2 antibodies with the typical specificity as assessed by ELISA with their serum samples. One of these mothers was IgA deficient with high serum levels of IgG class antibodies. IgG class anti-TG2 antibodies appeared in all three infants' serum. These antibodies had similar epitope specificity as the IgG antibodies of their mothers (Figure 12). The IgG antibodies were detected also in the umbilical cord tissue of the newborn from the IgA deficient mother, and this baby was underweight and had liver damage for which no other clinical cause was found and which resolved in paralel to the recession of serum antibody levels in blood. Jejunal biopsy was not possible in this infant by ethical reasons. HUVEC cells prepared from another baby exposed to maternal antibodies before birth displayed reduced cell survival, abnormalities in their shape and spreading on surfaces compared to cells from babies with antibody-negative celiac mothers.

### EXAMPLE 6

### DESIGN OF DIAGNOSTIC TRANSGLUTAMINASE MUTANTS OTHER THAN TG2

Using molecular modelling, we evaluated the structure of transglutaminase family member proteins and their surfaces corresponding to the composite celiac epitope identified in TG2. As shown in Table 2, the first N-terminal helix of the core domain is similar also in Factor XIII and TG3, which, however differ in some of the anchor points of the celiac epitope and typically do not cross-react with the classic celiac autoantibodies. Given the similarities in the the overall structure framework of these molecules, we expect that changing the amino acids corresponding to the main anchors points of the celiac epitope will enhance their binding properties. Based on results from molecular modelling and our experimental data with the E154K mutation in TG2, change of XIII factor Lys¹⁹⁹ to Glu is predicted to increase celiac antigenicity of Factor XIII (Figure 5). This engineered TG antigen could be used as reference protein together with original Factor XIII as test protein for measuring patient samples in a celiac epitope-specific way in ELISA or other immunoassay. In this embodiment, signal with the engineered protein but absence of the signal with original Factor XIII would indicate a celiac-type antibody binding. Similarly, change of Gly¹⁴⁶ and/or His¹⁴⁸ in TG3 or corresponding amino acids in other human or eukaryote TG family members could have a similar effect.

### EXAMPLE 7

### DEVELOPMENT OF DIAGNOSTIC KITS

### Diagnostic kit based on mutant TG2

A diagnostic kit can be developed based on the specific celiac antibody binding site of TG2 and the assay conditions described in examples 1-3. This kit is based on ELISA method, other immunoassays, or label-free binding assays. In ELISA setting, wild type TG2 and one or more mutant TG2s with an altered celiac epitope are bound to the surface of the plate and the binding pattern of the investigated sample would distinguish between false-positive and "real" celiac sera. The TG2 protein with the intact celiac epitope will be the reference and the decrease of antibody binding with celiac epitope-specific mutants indicates the celiac-type binding pattern. If a binding is detected to the reference protein but without at least a 30% change in the binding of the test protein, the result is interpreted as non-celiac type TG2 antibody result and no further clinical investigations are performed. In this way, unnecessary invasive investigations, like upper endoscopy and small intestinal biopsy can be avoided. If the results with both the reference and the test protein come out as lower than a predermined cut-off level, the sample does not contain TG2 antibodies and thus invasive investigations can be avoided as well. A suitable cut-off for declaring antibodies as reactive with the reference TG2 protein are established by a receiver operating characteristic curve (ROC) performed with known celiac and non-celiac samples. A cut-off of 5 % of the binding obtained with a sample from patient with untreated celiac disease is suggested based on results in example 5.

In addition to full-length wild type TG2, our following recombinant proteins showed a binding similar to wild-type TG2 and are suitable to be used as reference proteins in the above assay: D151N, E154Q, E155Q, C277S, Mutant 433, the domain mutants B (domains I-II-IV) and A (domains I-II-III) and the Site1 Ca²⁺-binding mutant with or without Ca²⁺ ions. The latter is useful in assay settings were addition of calcium would be undesirable. Further, a reference protein could be an engineered protein with a built-up celiac epitope on a TG family framework that is non-antigenic per se for celiac antibodies. For a test protein, mutants R, E, E158Q, E158L, but more preferably combined mutants RE, REM, RKM, Site 4 mutant, or the homologous TG protein without a celiac binding epitope representing the counterpart of the engineered protein are suggested.

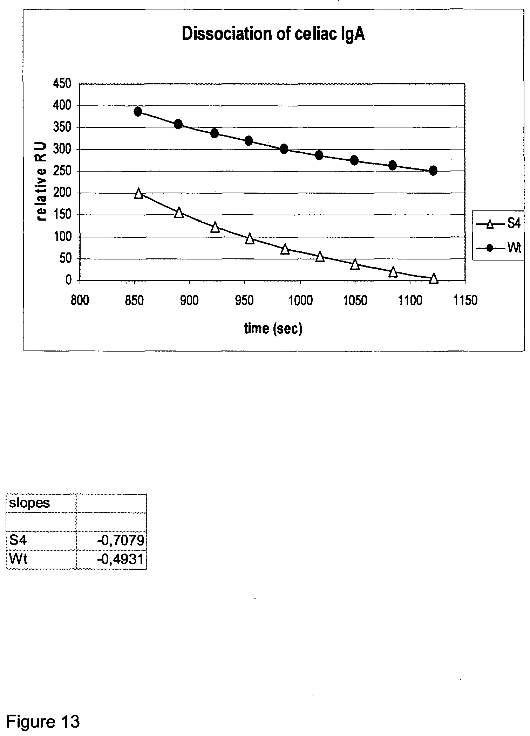

Figure 13 shows an example for label-free measurement of celiac antibody binding with Biacore method, where both binding and dissociation is altered of celiac antibodies to Site 4 mutant compared to wild TG2.

### Diagnostic kit based on antibody displacement

It was found that natural IgA and IgG antibodies purified from sera of celiac patients and recognizing R19S differently also competed with each other but not with non-celiac human IgG (Fig. 9 B1 to B3). Based on this finding we developed a diagnostic ELISA with 98% sensitivity and 96% specificity for measuring IgG class celiac antibodies in subjects with selective humoral IgA deficiency by their concentration-dependent displacement effect (r=0.88) on a known celiac IgA tracer antibody (Fig.9 A1 to A2).

In this exemplary assay we measured serum IgG class anti-TG2 antibodies in IgA deficient patients by displacement of an IgA celiac patient antibody. Serum samples from 56 untreated celiac disease patients aged 0.9-73 years (median 10.5) with selective IgA deficiency (total serum IgA <0.05 g/l), from 23 non-celiac IgA deficient and 22 normal serum IgA controls with normal small bowel architecture (age of the controls 1-18 years, median 5.5) were measured in ELISA with wild-type TG2 antigen. While the majority of the patients had IgG class patient antibodies, three of them also had IgM class anti-TG2. Investigated serum samples were diluted 1:100 in assay buffer containing a celiac IgA tracer antibody diluted 1:500 and the binding of the IgA antibody was measured by anti-IgA peroxidase-conjugated secondary antibodies. The optical density values (OD) obtained with blank were defined as 100% inhibition and the signal with the IgA tracer antibody without added IgA deficient samples was defined as 0% inhibition. The inhibition obtained with the investigated samples was calculated as 100-(ODtracer-ODsample /ODtracer). Intraassay variability was 5.9% and interassay variability was 9.2%. The optimal cut-off as a predetermined threshold calculated from receiver operated characteristics curves was 11% inhibition where the displacement assay had 98.2% sensitivity (95% confidence interval: 94.5-100) and 95.6% specificity (95% confidence interval: 89.9-100) for the diagnosis of celiac disease in IgA deficient persons. (Fig 9.A1) Correlation of the displacement effect with the serum concentration of IgG anti-TG2 antibodies measured by direct recognition of human IgG by monoclonal mouse anti-human IgG (Phadia) and anti-mouse secondary HRP-conjugated antibodies; r=0.88. (Fig 9.A2) In the present experimental setting the most preferred displacement ratio was observed at IgG dilution 1:100 and IgA dilution 1:500.

### EXAMPLE 8

### PREDICTION OF FORTHCOMING CLINICAL DISEASE BY THE EPITOPE SPECIFICITY OF PATIENT ANTIBODIES

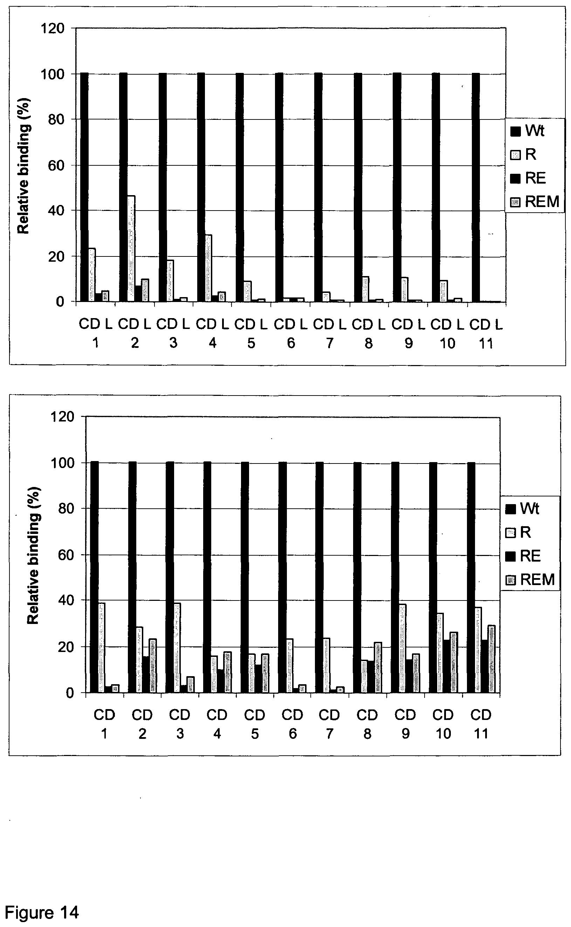

In order to see whether the reaction to the identified composite celiac epitope is already seen at the early preclinical stage of the disease and has also predictive value for diagnosis of celiac disease n later life, we tested 11 serum samples from patients positive for endomysial and TG2 antibodies in whom at the time of the serum collection signs of intestinal disease were not present (normal villous architecture) and thus the diagnosis of celiac disease could not immediately be made. Six of these subjects were symptom-free family members of known celiac disease patients and were picked by family screening. During a follow-up of 0.8-3 years, seven of these subjects developed severe villous atrophy in the small bowel with local antibody deposition and further two had antibody deposition with other minor lesions. The serum samples collected at the latent stage of the disease showed in all 11 cases similar epitope recognition pattern as the serum samples of celiac patients with overt clinical disease (Figure 14), and the difference between the reaction to the wild type TG2 and mutants RE and REM was even bigger.

Further cases giving diagnostic challenge are subjects in whom TG2 antibodies do not circulate. In such cases, the antibodies may be trapped within the tissues, and be available for diagnosis in the form of eluates. As these cases often present with extraintestinal symptoms that can be induced also by other diseases, epitope specificity will help to establish whether the observed pathology is gluten-related or not.

### EXAMPLE 9

### TREATMENT OPTION OF CELIAC DISEASE WITH MONOCLONAL ANTIBODY 885

### Monoclonal antibody targeting Glu¹⁵³ interferes with celiac antibody binding in vitro

Using our mutant TG2 proteins we characterized the binding properties of 13 already available monoclonal anti-TG2 antibodies [Korponay-Szabo IR et al, J Pediatr Gastroenterol Nutr. 2008;46(3):253-61.]. These antibodies had been generated in mice with purified full length wild-typeTG2 proteins of natural or recombinant origin, and antibody clones reacting with TG2 were selected in a random way. Surpisingly, one of these antibodies was found to have an epitope binding site that overlapped with celiac disease antibody binding (Figure 15). The binding of Mab 885 was completely abolished when point mutant E was applied and also when any of the double or triple mutants containing E were used. However, 885 bound well to both R and M mutants. Thus the binding site of Mab 885 was localized to Glu¹⁵³, so it was partially overlapping with the celiac epitope. We thus next tested if 885 antibodies would interfere with the binding of celiac antibodies to TG2 in ELISA and indirect immunofluorescent studies. Recombinant wild type full length TG2 was incubated with celiac patient IgA antibodies in the presence of excess (18 ug/well) MAb 885 and bound IgA was measured. The optimal ratio of the antigen in relation to celiac patient serum had previously been established to obtain a signal around O.D. 0.8-1.0 to which the decrease could be compared. Values for the bound amounts of celiac IgA were calculated from a standard curve obtained with IgA alone, without competing 885 antibodies. This experiment showed that in the presence of MAb 885 only 6.7% of natural polyclonal celiac IgA bound to TG2 whereas comparable amounts of anti-TG2 MAb CUB7402 or TG100 (NeoMarkers, Fremont, USA) having an irrelevant epitope did not have significant effect. Similar results were obtained with two further patient serum samples in independent experiments, which result further proves that the identified composite celiac epitope is relevant for all celiac patients and indicates the possibility of therapeutic exploitation. Similarly, in contrast to CUB7402 which produces a reproducible double staining signal when co-administered with celiac IgA on normal tissue sections often used in clinical diagnostics for the classical endomysial (or reticulin) antibody test [Korponay-Szabo I et el, J Pediatr Gastroenterol Nutr. 2000;31(5):520-7], previous binding of MAb885 to monkey esophagus or normal human jejunum tissue sections resulted in the loss of visible IgA binding, thus celiac IgA did not bind in the presence of MAb885.

### Monoclonal antibody targeting Glu¹⁵³ displaces and releases celiac antibodies from patient tissues

When Mab885 antibodies were added to celiac patient tissue sections which contained deposited IgA *in vivo*-bound to intrinsic TG2 of the patient, it was observed that 885 bound extensively to extracellular TG2 and could be recognized by secondary anti-mouse antibodies labelled with Alexa-Fluor red fluorescent dye, but surprisingly, at the same time the signal for human IgA almost disappeared (Figure 15C). This experiment was performed with the placenta tissue of celiac mothers with active disease, as described above. In order to evaluate whether 885 caused the lack of IgA staining by simply interfering with the binding of secondary anti-IgA green fluorescent dye or by displacing celiac IgA and removing it from the binding to TG2, the drop containing 885 antibodies was carefully aspirated from the tissue sections after the incubation time and was further tested. 885 antibodies were removed from this solution by magnetic beads conjugated with protein G (Dynabeads, Invitrogen). This treatment aimed to eliminate competing 885 antibodies in the detection ELISA. Indeed, the solution treated in this way contained TG2-specific IgA antibodies binding to recombinant human TG2 on the ELISA plate (Figure 15D). Drops containing only buffer were also aspirated after the same length of incubation time from the tissues for comparison, but these were negative for TG2-specific antibodies and showed that celiac IgA was not released spontaneously from the tissue simply by incubation with buffer, only in the presence of competing mouse 885 antibodies.

### Monoclonal antibodies targeting Glu¹⁵³ do not have same biological effects as celiac patient antibodies

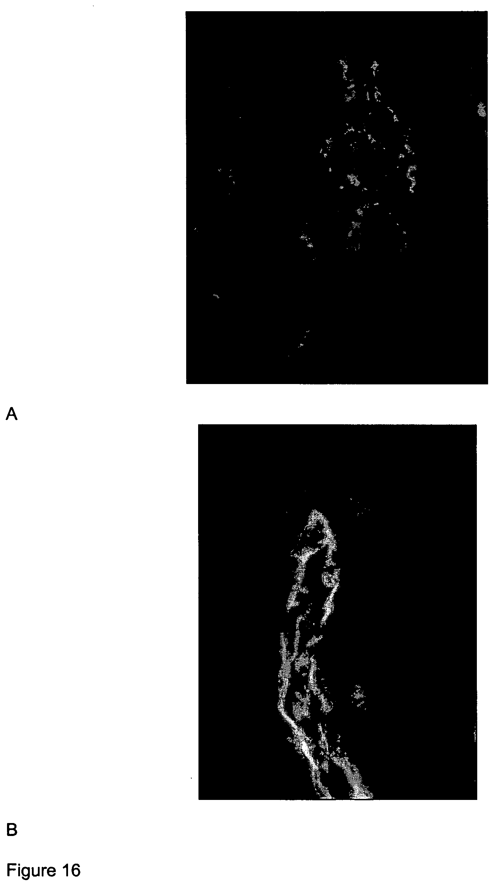

Effective competition of 885 antibodies with the celiac IgA raised the possibility that 885 antibodies might have a therapeutic potential in celiac patients by interfering with celiac IgA binding and the downstream pathogenic effects of this binding. Therefore we aimed to evaluate whether 885 antibodies themselves would have the same biological effects as patient antibodies that would preclude their medical use. Deleterious effect of patient antibodies is suggested recently by Caja et al. [Scandinavian Journal of Gastroenterology, 2010; Early Online, 1-7] As patient antibodies were shown to act both by modulating the protein cross-linking activity of TG2 and take part in protein-protein interactions, we addressed this issue in both biochemical measurements and cell experiments. In the previously established setting for measurement of transamidating activity of TG2 [Kiraly R et al, J Autoimmun. 2006;26(4):278-87.] where celiac antibodies caused an increment, while MAb CUB7402 and TG100 blocked catalytic activity, MAb 885 did not have any modulating effect (Figures 15E and 16A)..

In separate experiments we added 885 antibodies to commercial HUVECs in culture in the presence of the purified IgA fraction of celiac serum samples [Myrsky E et al, Clin Exp Immunol. 2008;152(1):111-9.]. Celiac IgA alone caused a decrease in cell length and adhesion properties which was reversed in the presence of MAb 885 (Figure 15F). These results indicate that MAb 885 can antagonize biological effects of celiac antibodies, too.

### EXAMPLE 10

### PREPARING THERAPEUTIC MONOCLONAL ANTIBODIES

To prepare further epitope hiding compounds in the forms of monoclonal antibodies, standard techniques can be complemented with a specific selection to obtain the desired targeting of the celiac epitope. First, mice are immunized with human recombinant TG2 to produce antibodies against TG2. After a few weeks of the immunization, blood samples are taken and screened for antibodies against TG2 with ELISA. When the antibody titer is high enough, mice are euthanized and spleens are removed to isolate antibody-producing spleen cells. To make these cells immortal, cell fusion is performed with tumor myeloma cells resulting in hybridomas, which are able to grow unlimitedly and secrete antibodies. Fusion is made by co-centrifugation of spleen and myeloma cells in polyethylene glycol, which allows membrane fusions. Then cells are distributed to 96 well plates covered with feeder cells that can supply growth factors to them. Hybridoma cells can be distributed to the wells with limiting dilution to ensure that each well contain a single clone and these clones can be grown in cell cultures. After establishment of the hybridoma lines, monoclonal antibodies are constantly secreted into the medium. In order to select to right clones, epitope specificity of the individual monoclonal antbodies is measured by ELISA using a TG2 antigen with intact celiac epitope and a mutant TG2 where the celiac epitope is altered. Then clones which secrete antibodies that bind to the intact protein but not to the mutant are selected for antibody production and purification. Using different mutants, the whole celiac epitope or parts thereof can be targeted. Preferebly, an antibody that only partially overlaps with the celiac antibody binding will be produced, and for this, mutant E or RE could be suitable. Candidate antibodies are also tested for avidity and for competition ability with patient antibodies in ELISA or related immunoassays and in the cell culture model described in Example 9.

### EXAMPLE 11

### PREPARING THERAPEUTIC MONOCLONAL ANTIBODIES BY THE PHAGE DISPLAY METHOD

A further tool to produce monoclonal antibodies that target the celiac epitope in the manner that is desired for use in therapy is creating a library from the variable regions of human antibodies by phage display technique. During this method, single chain variable fragments (scFv) are cloned from human samples, produced and selected for a given antigen [Marzari, R. et al. J. Immunol. 166, 4170-4176 (2001)]. There is a chance to find a TG2 specific antibody having the same binding site but opposite biological effect than the celiac antibodies from symptomatic patients, if we use a library from a symptom free celiac disease patient with silent disease or a celiac family member. This scFv could be tested for therapeutic aspects as described above for the 885 antibody.

The first step in the process of total antibody library preparation is total RNA isolation by Trizol from intestinal lymphocytes. Next, first-strand cDNA is synthesized using the isolated RNA as template and the variable region of H, κ and λ chains of IgA antibodies are amplified with specific primers by PCR technique. After the addition of a linker region, the amplified variable regions of heavy (VH+linker) and light chains (Vκ+linker and Vλ+linker) are assembled by another PCR. For the library production the assembled VH-Vκ and VH-Vλ are digested with restriction enzymes and ligated to the pDAN5 vector digested with the same enzymes. After this step the vector contains the assembled scFv as an insert and it can be transformed to DH5ΔF' electrocompetent *E. coli* cells by electroporation. The bacteria which contain the antibody library are grown overnight on selective media and next day, the library is harvested and stored at -80 C. The library should contain ∼10⁷ clones to maintain variability comparable with variability of antibodies in the human body.

Selection of the library for a given antigen is performed by phage display technique. For this, the bacteria containing the library are grown in selective medium and infected with helper phage. The helper phage provides enzymes and other proteins for the assembly of new, recombinant phages, which contain the scFv fused to its coating protein p3. After the production, the recombinant phages reacting with TG2 are selected with TG2 protein antigen coated immunotubes that have a Maxisorp (DAKO) surface. This antigen contains an intact celiac epitope. The uncoated surface is blocked with buffer containing bovine serum albumin, and the phages are added to the immunotube with the same blocking solution. Phages, which contain TG2-specific scFv on their surface, will bind to the coated antigen. These phages are then eluted from the immunotube with DH5ΔF' bacteria, grown overnight in plated bacteria, and harvested the next day as the 1^{st} output of selection. Second and more selection rounds are performed (growing bacteria from the previous output and with the same steps as in the first round) to enhance specificity of the clones, using TG2 proteins - preferably our E or RE mutants - were the celiac epitope is altered, and then growing only the bacteria which contain inserts specific for the intact TG2 but not for the TG2 protein with the altered celiac epitope. In this way, clones containing antibodies targeted against the desired TG2 epitope are specifically selected and grown further on selective media, while clones reacting to other epitopes of TG2 are discarded. It follows that if the aim is to select a therapeutic antibody which binds to only a part of the epitope, such as E153 or an equivalent amino acid thereto, but does not bind to Arg19, it is advisable also to screen for binding to Arg19. For this purpose an R mutant can be used wherein if binding is shown to R mutant but not to the E mutant a potential therapeutic antibody is obtained. The epitope specificity of the clones is verified by ELISA using the produced antibodies, characterized by fingerprinting PCR and digestion with restriction enzymes. ELISA plates are covered with full length human recombinant TG2 and mutant TG2 proteins and then incubated with the phages. The binding is detected with anti-phage M13 antibody conjugated with peroxidase (GE Healthcare), revealed with tetramethylbenzidine and the reaction is stopped with sulphuric acid. The absorbance values are read at OD450 by spectrophotometer. Clones producing celiac epitope-specific ScFv antibodies having opposite effect on the activity of TG2 enzyme than natural celiac patient antibodies and not eliciting the same biological effect in cell culture described in Example 9 are picked for further application.

### EXAMPLE 12

### COMPETITION ELISA FOR EVALUATING DIAGNOSTIC PATIENT SAMPLES FOR EPITOPE SPECIFICITY

Antibodies against TG2 in patient samples may cause diagnostic difficulties because either the detection of their antibody class might be difficult (like IgM) or their epitope specificity is uncertain. To obtain a disease-specific antibody result, we designed two kinds of competition ELISA where the binding of patient antibodies to a TG2 antigen with intact celiac epitope was evaluated in the presence and absence of a test antibody interfering with the patient antibody-celiac antigen binding at the level of the epitope. For the ELISA application, the isotype of the test antibody was different than the antibody to be measured.
a) In the first type of ELISA, the monoclonal antibody 885 having a partially overlapping epitope as patient antibodies was used as the competitor test antibody in excess and the remaining binding of the celiac IgA patient antibodies to wild-type TG2 was measured as described in Example 9. Before adding 885 plus patient antibodies together, an optimising experiment was performed. The amount of TG2 coated on the plate was minimized to still obtain with the given dilution of the patient antibody a signal around O.D. 0.8-1.0 to which the decrease could be compared. We showed that under these conditions the displacement effect was dose dependently 90.6%, 85%, 64% with decreasing amounts of the 885 and in relation to the same patient sample, thus in the presence of 885 only 9.3%, 15% and 36% of patient antibodies bound. This ELISA in one embodiment may also show that if the patient antibody is mostly displaced by 885 or by a similar test antibody, the epitope specificity of the investigated patient antibody is such that it targets the main composite celiac epitope we identified.
b) If the ELISA is performed in the way that the amount of the test antibody is kept constant and the bound amounts of the test antibody are measured by the HRP-conjugate, a decrease in the signal proves the presence of competing TG2-specific antibodies in the sample having the celiac-type epitope specificity. Thereby e.g. unknown patient antbodies with IgG, IgM or IgA framework can be measured at the same time simultaneously by using the 885 test antibody in high dilutions, whereas patients' antibodies are in excess. Using appropriately diluted 885 antibodies, we could achieve that in immunofluorescent test with normal tissue samples patient IgA bound to the TG2 antigen in the endomysium while signal for the binding of Mab 885 has decreased (Figure 16). This type of ELISA could also be performed using a known IgA class celiac patient antibody and measuring IgG class celiac-specific antibodies of IgA deficient patients as described in Example 5. However, competition between two IgA class celiac antibodies also could be shown if the test antibody is labelled (e.g biotinylated) and the label is specifically recognized.

## Claims

1. A method for diagnosis of a gluten-induced autoimmune disease in a subject comprising the steps of
i) providing a biological sample taken from a subject, said sample containing autoantibodies of said subject, and optionally isolating autoantibodies from said sample,
ii) contacting the autoantibodies of said sample with
- a reference protein belonging to the transglutaminase family and having
a) a part of the molecular surface of a TG family protein to which Mab885 is capable of binding, said epitope including a surface area within 7 Å from the area covered by Mab885 when bound, or
b) a part of the molecular surface of a TG family protein, to which a celiac autoantibody of a subject with celiac disease is capable of binding, in a manner that said autoantibody can be displaced in a competitive assay by Mab 885, or
c) a part of the molecular surface of a TG family protein which may be an autoantigen in celiac disease, at least partially formed by amino acid residue corresponding to or equivalent to Glu153 and/or Arg19 numbered according to the amino acid numbering of the full length human TG2 based on multiple sequence alignment, or
d) a part of a molecular surface of a TG family protein, which is capable of binding a surface recognition molecule, wherein said surface recognition molecule is capable of binding to a part of the molecular surface as defined in a) to c),
said part forming an intact main celiac epitope,
- at least one test protein belonging to the transglutaminase family, in which the side chain and/or spatial position of at least one surface amino acid residue contributing to the main celiac epitope is altered as compared to the reference protein in which the main celiac epitope is intact,
wherein said at least one surface amino acid residue the side chain and/or spatial position of which is altered is selected from
an amino acid residue corresponding to or equivalent to Glu153 numbered according to the amino acid numbering of the full length human TG2 based on amino acid sequence alignment, and/or
an amino acid residue corresponding to or equivalent to Arg19 numbered according to the amino acid numbering of the full length human TG2 based on amino acid sequence alignment
and wherein the core domain has a folded three dimensional structure,
iii) assessing a binding property of the autoantibodies to the reference protein and to the at least one test protein,
wherein if the binding of autoantibodies to the test protein is impaired as compared to the reference protein, this fact is considered as indicative of a gluten-induced autoimmune disease in said subject.

2. The diagnostic method for the diagnosis of a gluten-induced autoimmune disease in a subject according to claim 1, wherein in step iii) the binding is assessed by measuring binding level of the autoantibodies to the test protein and to the reference protein,
wherein if the mean binding level of the autoantibodies to the reference protein exceeds a predetermined threshold value and if the mean binding level of the autoantibodies to the test protein is significantly lower, preferably reduced by at least 30% as compared to the reference protein, this fact is considered as indicative of a gluten-induced autoimmune disease in said subject.

3. The diagnostic method according to any of claims 1 or 2, wherein any one of the test protein and the reference protein is a wild type protein or is prepared by protein engineering from an appropriate scaffold which is selected from an animal TG1, TG2, TG3, TG4, TG5, TG6, TG7, factor XIII, or EBP42,
wherein preferably
a) the test protein is a mutant transglutaminase (TG), preferably a mutant TG2, TG3 or TG6, and
b) the reference protein is a wild type TG, preferably a wild type TG2, TG3 or TG6.

4. The diagnostic method according to any of claims 1 to 3 wherein in said test protein belonging to the transglutaminase family the side chain or spatial position of at least one further amino acid is altered as compared to the reference protein in which the main celiac epitope is intact.

5. The diagnostic method according to claim 4, wherein said further amino acid is selected from the group of amino acid residues corresponding to or equivalent to Arg 151, Glu 153, Glu 154, Arg156, Arg 19, His22, Val431, Arg433, Glu435, Met659 and Leu661 numbered according to the amino acid numbering of the full length human TG2 based on multiple sequence alignment.

6. A use of a test protein as defined in any of claims 1 to 5 in a method for diagnosis of celiac disease, said test protein belonging to the transglutaminase family, in which the side chain and/or spatial position of at least one surface amino acid residue contributing to the main celiac epitope as defined in claim 1 is altered as compared to the reference protein in which the main celiac epitope is intact,
wherein the said at least one surface amino acid residue of the main celiac epitope comprises or is selected from
an amino acid residue corresponding to or equivalent to Glu153 numbered according to the amino acid numbering of the full length human TG2 based on amino acid sequence alignment, and/or
which is an amino acid residue corresponding to or equivalent to Arg19 numbered according to the amino acid numbering of the full length human TG2 based on amino acid sequence alignment
and wherein the core domain has a folded three dimensional structure.

7. A diagnostic method for the diagnosis of a gluten-induced autoimmune disease in a subject comprising the steps of
i) providing a biological sample taken from a subject, said sample containing autoantibodies of said subject and optionally isolating autoantibodies from said sample,
ii) contacting autoantibodies of said sample with the reference protein belonging to the transglutaminase family as defined in claim 1, said protein having an intact main celiac epitope as defined in claim 1, and
iii) assessing the level of binding of the autoantibodies to the reference protein both in the absence of and in the presence of a test compound, said test compound being a test antibody or fragment, variant, derivative or analogue thereof known to be capable of binding to the main celiac epitope;
wherein if the mean binding level of the autoantibodies in the absence of the test compound exceeds a predetermined threshold value and if the mean binding level of the autoantibodies to the reference protein in the presence of the test compound is significantly lower, preferably reduced by at least 50% as compared to the binding level of the autoantibodies in the absence of said test antibody, this fact is considered as indicative of a gluten-induced autoimmune disease in said subject.

8. A diagnostic method for the diagnosis of a gluten-induced autoimmune disease in a subject comprising the steps of
i) providing a biological sample taken from a subject,
ii) contacting a test compound with a reference protein belonging to the transglutaminase family as defined in claim 1, said protein having an intact main celiac epitope as defined in claim 1 and said test compound being a test antibody or fragment, variant, derivative or analogue known to be capable of binding to the main celiac epitope,
iii) assessing the level of binding of the test compound to the reference protein both in the absence of and in the presence of said biological sample,
wherein if the mean binding level of the test compound to the reference protein in the presence of the sample is lower than the binding level in the absence of the sample, this fact is indicative of the presence of autoantibodies capable of binding to the main celiac epitope and of a gluten-induced autoimmune disease in said subject.

9. Use of a test compound known to be capable of binding to the main celiac epitope of a reference protein belonging to the transglutaminase family as defined in claim 1 in a diagnostic method for the diagnosis of a gluten-induced autoimmune disease in a subject, said test compound being a test antibody or antibody fragment, variant or analogue known to be capable of binding to the main celiac epitope said reference protein being an autoantigen in celiac disease.

10. The diagnostic method of claim 7 or 8 or the use of claim 9 wherein said test compound being Mab885 or a fragment or derivative thereof capable of binding to the same epitope region.

11. A diagnostic kit for the diagnosis of a gluten-induced autoimmune disease in a subject, said kit comprising
- a reference protein belonging to the transglutaminase family and having
a) a part of the molecular surface of a TG family protein to which Mab885 is capable of binding, said epitope including a surface area within 7 Å from the area covered by Mab885 when bound, or
b) a part of the molecular surface of a TG family protein, to which a celiac autoantibody of a subject with celiac disease is capable of binding, in a manner that said autoantibody can be displaced in a competitive assay by Mab 885, or
c) a part of the molecular surface of a TG family protein which may be an autoantigen in celiac disease, at least partially formed by amino acid residue corresponding to or equivalent to Glu153 and/or Arg19 numbered according to the amino acid numbering of the full length human TG2 based on multiple sequence alignment, or
d) a part of a molecular surface of a TG family protein, which is capable of binding a surface recognition molecule, wherein said surface recognition molecule is capable of binding to a part of the molecular surface as defined in a) to c),
said part forming an intact main celiac epitope,
- a test protein belonging to the transglutaminase family
in which the side chain and/or spatial position of at least one surface amino acid residue contributing to the main celiac epitope is altered as compared to the reference protein in which the main celiac epitope is intact,
wherein said at least one surface amino acid residue the side chain and/or spatial position of which is altered is selected from
an amino acid residue corresponding to or equivalent to Glu153 numbered according to the amino acid numbering of the full length human TG2 based on amino acid sequence alignment, and/or
an amino acid residue corresponding to or equivalent to Arg19 numbered according to the amino acid numbering of the full length human TG2 based on amino acid sequence alignment,
and wherein the core domain has a folded three dimensional structure, and
means for taking or processing a biological sample from a subject, said sample containing autoantibodies of said subject and/or
means for isolating autoantibodies from said sample, and/or
means for assessing the level of binding and/or kinetics of the autoantibodies to the proteins.

12. A diagnostic kit for the diagnosis of a gluten-induced autoimmune disease in a subject comprising
- a reference protein belonging to the transglutaminase family and having
a) a part of the molecular surface of a TG family protein to which Mab885 is capable of binding, said epitope including a surface area within 7 Å from the area covered by Mab885 when bound, or
b) a part of the molecular surface of a TG family protein, to which a celiac autoantibody of a subject with celiac disease is capable of binding, in a manner that said autoantibody can be displaced in a competitive assay by Mab 885, or
c) a part of the molecular surface of a TG family protein which may be an autoantigen in celiac disease, at least partially formed by amino acid residue corresponding to or equivalent to Glu153 and/or Arg19 numbered according to the amino acid numbering of the full length human TG2 based on multiple sequence alignment, or
d) a part of a molecular surface of a TG family protein, which is capable of binding a surface recognition molecule, wherein said surface recognition molecule is capable of binding to a part of the molecular surface as defined in a) to c),
said part forming an intact main celiac epitope, , wherein the core domain of said reference protein has a folded three dimensional structure , and
- a test compound known to be capable of binding to the main celiac epitope of said reference protein belonging to the transglutaminase family, said main celiac epitope
wherein
either the test compound is labelled or
the kit also comprises a labelled compound specifically binding to the test compound
to detect binding of the test compound to the reference protein,
and optionally
means for taking or processing a biological sample from a subject, said sample containing autoantibodies of said subject and/or
means for isolating autoantibodies from said sample.

13. A therapeutic compound which is a monoclonal antibody or any fragment thereof comprising a binding region, e.g. single chain variable fragment (scFv) or an Fab fragment, said compound being a specific inhibitor of the binding of a celiac antibody to a celiac epitope present on a protein belonging to the transglutaminase family, for use in the treatment of or preventing the onset of a gluten induced autoimmune disease,
said compound being capable of binding
to a part of the main celiac epitope which is at least partially formed by
an amino acid residue corresponding to or equivalent to Glu153 numbered according to the amino acid numbering of the full length human TG2 based on amino acid sequence alignment and
said part being a part of the molecular surface of a TG family protein to which or to a part of which Mab 885 is capable of binding, including a surface area within 7 Å from the area covered by Mab885 when bound,
with a sufficiently high avidity of binding affinity to displace a celiac autoantibody,
said compound being capable of reversing or antagonizing an adverse effect of celiac autoantibodies on cells of an epithelial cell or tissue culture and
wherein said compound does not affect or alter by more than ±50% the transglutaminase activity of said active protein and/or said compound does not affect or alter by more than ±50% the GTPase activity of said protein.

14. The compound of claim 13,
wherein said compound is capable of antagonizing any deleterious effect of celiac antibodies on HUVEC cells, once added to the HUVEC cells.

15. The compound of claim 13 or 14, said compound being a monoclonal antibody, whereby if contacted with protein belonging to the transglutaminase family, it is capable of displacing the celiac autoantibody and thereby to antagonize its effect.

16. The compound of claim 14, said compound being Mab885 or a fragment or derivative thereof capable of binding to the same epitope region.

## Patentansprüche

1. Verfahren zur Diagnose einer gluten-induzierten Autoimmunkrankheit in einem Subjekt, umfassend die Schritte
i) Bereitstellen einer aus einem Subjekt entnommenen biologischen Probe, wobei die Probe Autoantikörper des Subjekts enthält, und wahlweise Isolieren von Autoantikörpern aus der Probe,
ii) Inkontaktbringen der Autoantikörper der Probe mit
- einem Referenzprotein aus der Transglutaminase-Familie mit
a) einem Teil der Moleküloberfläche von einem Protein aus der TG Familie, an das Mab885 anbinden kann, wobei das Epitop einen Oberflächenbereich in der Weite von 7 Å von dem durch Mab885 abgedeckten Bereich umfasst, wenn Mab885 gebunden ist, oder
b) einem Teil der Moleküloberfläche von einem Protein aus der TG Familie, an das ein Zöliakie-Autoantikörper des Subjekts mit Zöliakie anbinden kann, so dass der Autoantikörper in einem kompetitiven Assay durch Mab885 verlagerbar ist, oder
c) einem Teil der Moleküloberfläche von einem Protein aus der TG Familie, das ein Autoantikörper in Zöliakie sein kann, wobei das Teil wenigstens teilweise durch ein zur/zum Glu153 und/oder Arg19 entsprechender oder äquivalenter Aminosäurerest gebildet ist, wobei Glu153 und/oder Arg19 gemäß der Nummerierung der Aminosäure von der vollständigen menschlichen TG2 auf Grund des multiplen Sequenzalignments nummeriert sind/ist, oder
d) einem Teil der Moleküloberfläche von einem Protein aus der TG Familie, das ein Oberfläche-Erkennungsmolekül anbinden kann, wobei das Oberfläche-Erkennungsmolekül an ein Teil der Moleküloberfläche wie unter a) bis c) definiert, anbinden kann,
wobei das Teil ein intaktes Zöliakie-Hauptepitop bildet,
- wenigstens ein Testprotein aus der Transglutaminase-Familie, in dem die Seitenkette und/oder die räumliche Position von mindestens eine zum Zöliakie-Hauptepitop beitragende Oberfläche-Aminosäurerest im Vergleich zum Referenzprotein, bei dem das Zöliakie-Hauptepitop intakt ist, verändert ist,
wobei der mindestens eine Oberfläche-Aminosäurerest, dessen Seitenkette und/oder räumliche Position verändert sind/ist, ausgewählt ist aus
einem zum Glu153 entsprechenden oder äquivalenten Aminosäurerest, das gemäß der Nummerierung der Aminosäure von der vollständigen menschlichen TG2 auf Grund des Aminosäure-Sequenzalignments nummeriert ist, und/oder einem zum Arg19 entsprechenden oder äquivalenten Aminosäurerest, das gemäß der Nummerierung der Aminosäure von der vollständigen menschlichen TG2 auf Grund des Aminosäure-Sequenzalignments nummeriert ist,
und wobei die Kerndomäne eine gefaltete dreidimensionale Struktur hat,
iii) Bewertung einer Bindungseigenschaft des Autoantikörpers zu dem Referenzprotein und zu dem mindestens einen Testprotein,
wobei wenn die Bindung der Autoantikörper zu dem Testprotein im Vergleich zu dem Referenzprotein beeinträchtigt ist, dieser Fakt als Hinweise auf eine gluten-induzierte Autoimmunkrankheit in dem Subjekt betrachtet ist.

2. Diagnoseverfahren zur Diagnose einer gluten-induzierten Autoimmunkrankheit in einem Subjekt nach Anspruch 1, wobei in Schritt iii) die Bindung durch Messen des Bindungsniveaus der Autoantikörper zu dem Testprotein und zu dem Referenzprotein bewertet ist,
wobei wenn das mittlere Bindungsniveau der Autoantikörper zu dem Referenzprotein einen vorbestimmten Schwellenwert überschreitet und wenn das mittlere Bindungsniveau der Autoantikörper zu dem Testprotein im Vergleich zu dem Referenzprotein signifikant niedriger ist, vorzugsweise um zumindest 30% verringert ist, dieser Fakt als Hinweise auf eine gluten-induzierte Autoimmunkrankheit in dem Subjekt betrachtet ist.

3. Diagnoseverfahren nach Anspruch 1 oder 2, wobei eines dem Testprotein und dem Referenzprotein ein Wildtyp-Protein ist, oder durch Protein-Engineering aus einem geeigneten Gerüst, das aus tierische TG1, TG2, TG3, TG4, TG5, TG6, TG7, Faktor XIII oder EBP42 ausgewählt ist, hergestellt wird,
wobei vorzugsweise
a) das Testprotein eine mutierte Transglutaminase (TG), vorzugsweise eine mutierte TG2, TG3 oder TG6, ist,
und
b) das Referenzprotein eine Wildtyp-TG, vorzugsweise eine Wildtyp-TG2, -TG3 oder -TG6, ist.

4. Diagnoseverfahren nach einem der Ansprüche 1 bis 3, wobei in dem Testprotein aus der Transglutaminase-Familie die Seitenkette oder die räumliche Position von mindestens einer weiteren Aminosäure im Vergleich zum Referenzprotein, bei dem das Zöliakie-Epitop intakt ist, verändert ist.

5. Diagnoseverfahren nach Anspruch 4, wobei die weitere Aminosäure aus der Gruppe von Aminosäurereste, die entsprechend oder äquivalent zur Arg151, Glu153, Glu154, Arg156, Arg19, His22, Val431, Arg433, Glu435, Met659 und Leu661 ist, die gemäß der Nummerierung der Aminosäure von der vollständigen menschlichen TG2 auf Grund des multiplen Sequenzalignments nummeriert sind, ausgewählt ist.

6. Verwendung eines Testproteins, wie definiert in einem der Ansprüche 1 bis 5, in einem Verfahren zur Diagnose von Zöliakie, wobei das Testprotein aus der Transglutaminase-Familie, in dem die Seitenkette und/oder die räumliche Position von mindestens einer zum Zöliakie-Hauptepitop beitragenden Oberfläche-Aminosäurerest, wie definiert in Anspruch 1, im Vergleich zum Referenzprotein, bei dem das Zöliakie-Hauptepitop intakt ist, verändert ist,
wobei der mindestens eine Oberfläche-Aminosäurerest des Zöliakie-Hauptepitops die folgende Aminosäurereste umfasst oder ausgewählt ist aus
einem zum Glu153 entsprechenden oder äquivalenten Aminosäurerest, das gemäß der Nummerierung der Aminosäure von der vollständigen menschlichen TG2 auf Grund des Aminosäure-Sequenzalignments nummeriert ist, und/oder
einem zum Arg19 entsprechenden oder äquivalenten Aminosäurerest, das gemäß der Nummerierung der Aminosäure von der vollständigen menschlichen TG2 auf Grund des Aminosäure-Sequenzalignments nummeriert ist,
und wobei die Kerndomäne eine gefaltete dreidimensionale Struktur hat.

7. Diagnoseverfahren zur Diagnose einer gluten-induzierten Autoimmunkrankheit in einem Subjekt, umfassend die Schritte
i) Bereitstellen einer aus einem Subjekt entnommenen biologischen Probe, wobei die Probe Autoantikörper des Subjekts enthält, und wahlweise Isolieren von Autoantikörpern aus der Probe,
ii) Inkontaktbringen der Autoantikörper der Probe mit dem Referenzprotein aus der Transglutaminase-Familie wie definiert in Anspruch 1, wobei das Protein ein intakt Zöliakie-Hauptepitop wie definiert in Anspruch 1 hat, und
iii) Bewertung des Bindungsniveaus der Autoantikörper zu dem Referenzprotein sowohl in der Abwesenheit als auch in der Gegenwart einer Testverbindung, wobei die Testverbindung ein Testantikörper, ein Fragment, eine Variante, ein Derivat oder ein Analogon davon, die oder das bekanntermaßen an das Zöliakie-Hauptepitop anbinden kann, ist;
wobei wenn das mittlere Bindungsniveau der Autoantikörper in der Abwesenheit der Testverbindung einen vorbestimmten Schwellenwert überschreitet und wenn das mittlere Bindungsniveau der Autoantikörper zu dem Referenzprotein in der Gegenwart der Testverbindung im Vergleich zu dem Bindungsniveau der Autoantikörper in der Abwesenheit des Testantikörpers signifikant niedriger ist, vorzugsweise um zumindest 50% verringert ist, dieser Fakt als Hinweise auf eine gluten-induzierte Autoimmunkrankheit in dem Subjekt betrachtet ist.

8. Diagnoseverfahren zur Diagnose einer gluten-induzierten Autoimmunkrankheit in einem Subjekt, umfassend die Schritte
i) Bereitstellen einer aus einem Subjekt entnommenen biologischen Probe,
ii) Inkontaktbringen einer Testverbindung mit einem Referenzprotein aus der Transglutaminase-Familie wie definiert in Anspruch 1, wobei das Protein ein intakt Zöliakie-Hauptepitop wie definiert in Anspruch 1 hat, und wobei die Testverbindung ein Testantikörper oder ein Fragment, eine Variante oder ein Derivat, die oder das bekanntermaßen an das Zöliakie-Hauptepitop anbinden kann, ist,
iii) Bewertung des Bindungsniveau der Testverbindung zu dem Referenzprotein sowohl in der Abwesenheit als auch in der Gegenwart der biologischen Probe,
wobei wenn das mittlere Bindungsniveau der Testverbindung zu dem Referenzprotein in der Gegenwart der Probe niedriger als das Bindungsniveau in der Abwesenheit der Probe ist, dieser Fakt als Hinweise auf die Gegenwart der Autoantikörper bindungsfähig zu dem Zöliakie-Hauptepitop und auf eine gluten-induzierte Autoimmunkrankheit in dem Subjekt gilt.

9. Verwendung einer Testverbindung, die bekanntermaßen zu dem Zöliakie-Hauptepitop eines Referenzproteins aus der Transglutaminase-Familie wie definiert in Anspruch 1, in einem Diagnoseverfahren zur Diagnose einer gluten-induzierten Autoimmunkrankheit in einem Subjekt anbinden kann, wobei die Testverbindung ein Testantikörper oder ein Testantikörper-Fragment, eine Variante oder ein Analogon , die oder das bekanntermaßen zu dem Zöliakie-Hauptepitop anbinden kann, ist, wobei das Referenzprotein ein Autoantigen in Zöliakie ist.

10. Diagnoseverfahren nach Anspruch 7 oder 8 oder Verwendung nach Anspruch 9, wobei die Testverbindung Mab885, oder ein Fragment oder Derivat davon, das an dieselben Epitop-Region anbinden kann, ist.

11. Diagnose-Kit zur Diagnose von einer gluten-induzierten Autoimmunkrankheit in einem Subjekt, umfassend
- ein Referenzprotein aus der Transglutaminase-Familie mit
a) einem Teil der Moleküloberfläche von einem Protein aus der TG Familie, an das Mab885 anbinden kann, wobei das Epitop einen Oberflächenbereich in der Weite von 7 Å von dem durch Mab885 abgedeckten Bereich umfasst, wenn Mab885 gebunden ist, oder
b) einem Teil der Moleküloberfläche von einem Protein aus der TG Familie, an das ein Zöliakie-Autoantikörper des Subjekts mit Zöliakie anbinden kann, so dass der Autoantikörper in einem kompetitiven Assay durch Mab885 verlagerbar ist, oder
c) einem Teil der Moleküloberfläche von einem Protein aus der TG Familie, das ein Autoantikörper in Zöliakie sein kann, wobei das Teil wenigstens teilweise durch ein zur/zum Glu153 und/oder Arg19 entsprechender oder äquivalenter Aminosäurerest gebildet ist, wobei Glu153 und/oder Arg19 gemäß der Nummerierung der Aminosäure von der vollständigen menschlichen TG2 auf Grund des multiplen Sequenzalignments nummeriert sind/ist, oder
d) einem Teil der Moleküloberfläche von einem Protein aus der TG Familie, das ein Oberfläche-Erkennungsmolekül anbinden kann, wobei das Oberfläche-Erkennungsmolekül an ein Teil der Moleküloberfläche wie unter a) bis c) definiert, anbinden kann,
wobei das Teil ein intaktes Zöliakie-Hauptepitop bildet,
- wobei ein Testprotein zu der Transglutaminase-Familie gehört, in dem die Seitenkette und/oder die räumliche Position von mindestens eine Oberfläche-Aminosäurerest im Vergleich zum Referenzprotein, bei dem das Zöliakie-Epitop intakt ist, verändert wird,
wobei der mindestens eine Oberfläche-Aminosäurerest, dessen Seitenkette und/oder räumliche Position verändert sind/ist, ausgewählt ist aus
einem zum Glu153 entsprechenden oder äquivalenten Aminosäurerest, wobei Glu153 gemäß der Nummerierung der Aminosäure von der vollständigen menschlichen TG2 auf Grund des Aminosäure-Sequenzalignments nummeriert ist, und/oder
einem zum Arg19 entsprechenden oder äquivalenten Aminosäurerest, wobei Arg19 gemäß der Nummerierung der Aminosäure von der vollständigen menschlichen TG2 auf Grund des Aminosäure-Sequenzalignments nummeriert ist,
und wobei die Kerndomäne eine gefaltete dreidimensionale Struktur hat, und
Mittel zur Entnahme oder zur Verarbeitung einer biologischen Probe von einem Subjekt, wobei die Probe Autoantikörper des Subjekts enthält, und/oder
Mittel zum Isolieren von Autoantikörpern aus der Probe, und/oder
Mittel zum Bewertung des Bindungsniveaus und/oder der Kinetik der Autoantikörper zu der Proteine.

12. Diagnose-Kit zur Diagnose von einer gluten-induzierten Autoimmunkrankheit in einem Subjekt, umfassend
- ein Referenzprotein aus der Transglutaminase-Familie mit
a) einem Teil der Moleküloberfläche von einem Protein aus der TG Familie, an das Mab885 anbinden kann, wobei das Epitop einen Oberflächenbereich in der Weite von 7 Å von dem durch Mab885 abgedeckten Bereich umfasst, wenn Mab885 gebunden ist, oder
b) einem Teil der Moleküloberfläche von einem Protein aus der TG Familie, an das ein Zöliakie-Autoantikörper des Subjekts mit Zöliakie anbinden kann, so dass der Autoantikörper in einem kompetitiven Assay durch Mab885 verlagerbar ist, oder
c) einem Teil der Moleküloberfläche von einem Protein aus der TG Familie, das ein Autoantikörper in Zöliakie sein kann, wobei das Teil wenigstens teilweise durch ein zur/zum Glu153 und/oder Arg19 entsprechender oder äquivalenter Aminosäurerest gebildet ist, wobei Glu153 und/oder Arg19 gemäß der Nummerierung der Aminosäure von der vollständigen menschlichen TG2 auf Grund des multiplen Sequenzalignments nummeriert sind/ist, oder
d) einem Teil der Moleküloberfläche von einem Protein aus der TG Familie, das ein Oberfläche-Erkennungsmolekül anbinden kann, wobei das Oberfläche-Erkennungsmolekül an ein Teil der Moleküloberfläche wie unter a) bis c) definiert, anbinden kann,
wobei das Teil ein intaktes Zöliakie-Hauptepitop bildet, wobei die Kerndomäne des Referenzproteins eine gefaltete dreidimensionale Struktur hat, und
- eine Testverbindung, die bekanntermaßen an den Zöliakie-Hauptepitop des Referenzproteins aus der Transglutaminase-familie anbinden kann,
wobei
entweder die Testverbindung markiert ist, oder
der Kit auch eine markierte Verbindung umfasst, die spezifisch an die Testverbindung bindet um
die Bindung der Testverbindung zu dem Referenzprotein nachzuweisen und wahlweise
Mittel zur Entnahme oder zur Verarbeitung einer biologischen Probe von einem Subjekt, wobei die Probe Autoantikörper des Subjekts enthält, und/oder
Mittel zum Isolieren von Autoantikörpern aus der Probe.

13. Therapeutische Verbindung, die ein monoklonaler Antikörper oder ein Fragment davon ist, die eine Bindungsregion z. B. ein einkettiges variables Fragment (scFv) oder ein Fab Fragment enthält, wobei die Verbindung ein spezifischer Inhibitor der Bindung eines Zöliakie-Antikörpers zu einem Zöliakie-Epitop an einem Protein aus der Transglutaminase-Familie, zur Verwendung bei der Behandlung von oder bei der Vorbeugung vom Einsatz einer gluten-induzierten Autoimmunkrankheit, ist
wobei die Verbindung
an einen Teil des Zöliakie-Hauptepitops anbinden kann, das wenigstens teilweise durch
einen zum Glu153 entsprechenden oder äquivalenten Aminosäurerest gebildet ist, wobei Glu153 gemäß der Nummerierung der Aminosäure von der vollständigen menschlichen TG2 auf Grund des Aminosäure-Sequenzalignments nummeriert ist,
wobei das Teil ein Teil der Moleküloberfläche von einem Protein aus der TG Familie ist, an das oder an ein Teil davon Mab885 anbinden kann, und einen Oberflächenbereich in der Weite von 7 Å von dem durch Mab885 abgedeckten Bereich umfasst, wenn Mab885 gebunden ist, oder
mit einer ausreichenden hohen Avidität oder Bindungsaffinität um ein Zöliakie-Autoantikörper zu verlagern,
wobei die Verbindung eine nachteilige Auswirkung der Zöliakie-Autoantikörper auf Zellen einer Epithelzellkultur oder einer Gewebekultur umkehren oder antagonisieren kann, und
wobei die Verbindung höchstens um ±50% der Transglutaminaseaktivität des aktiven Proteins nicht beeinflusst oder verändert, und/oder die Verbindung höchstens um ±50% der GTPase-Aktivität des Proteins nicht beeinflusst oder verändert.

14. Verbindung nach Anspruch 13, wobei die Verbindung jede schädliche Wirkung des Zöliakie-Antikörpers auf HUVEC Zellen antagonisieren kann, sobald es zu den HUVEC Zellen hinzugefügt ist.

15. Verbindung nach Anspruch 13 oder 14, wobei die Verbindung ein monoklonaler Antikörper ist, wodurch wenn die mit einem Protein aus der Tranglutaminase-Familie inkontaktgebracht ist, die den Zöliakie-Autoantikörper verlagern kann und damit dessen Wirkung antagonisieren kann.

16. Verbindung nach Anspruch 14, wobei die Verbindung Mab885, oder ein Fragment oder ein Derivat davon, das an dieselben Epitop-Region binden kann, ist.

## Revendications

1. Un procédé pour le diagnostic d'une maladie auto-immune induite par le gluten chez un sujet comprenant les étapes de
i) fournir un échantillon biologique prélevé sur un sujet, ledit échantillon contenant des autoanticorps dudit sujet, et éventuellement isoler des autoanticorps dudit échantillon,
ii) contacter les autoanticorps dudit échantillon avec
- une protéine de référence appartenant à la famille des transglutaminases et ayant
a) une partie de la surface moléculaire d'une protéine de la famille TG à laquelle Mab885 est capable de se lier, ledit épitope comprenant une surface comprise dans un rayon de 7 Å autour de la zone couverte par Mab885 lors de sa liaison ou
b) une partie de la surface moléculaire d'une protéine de la famille TG, à laquelle un autoanticorps coeliaque d'un sujet atteint d'une maladie coeliaque est capable de se lier, d'une manière telle que ledit autoanticorps peut être déplacé dans un essai compétitif par Mab 885 ou
c) une partie de la surface moléculaire d'une protéine de la famille TG qui peut être un auto antigène dans la maladie coeliaque, formée au moins partiellement par un résidu d'acide aminé correspondant à ou équivalent à Glu153 et / ou Arg19, numérotée(s) selon la numérotation des acides aminés de TG2 humaine de pleine longueur basée sur l'alignement de séquences multiples
d) une partie d'une surface moléculaire d'une protéine de la famille TG, capable de se lier à une molécule de reconnaissance de surface, où ladite molécule de reconnaissance de surface est capable de se lier à une partie de la surface moléculaire telle que définie dans a) à c),
ladite partie formant un épitope coeliaque principal intact,
- au moins une protéine d'essai appartenant à la famille des transglutaminases, dans laquelle la chaîne latérale et / ou la position spatiale d'au moins un résidu d'acide aminé de surface contribuant à l'épitope coeliaque principal est modifiée par rapport à la protéine de référence dans laquelle l'épitope coeliaque principal est intact,
où ledit au moins un résidu d'acide aminé de surface dont la chaîne latérale et / ou la position spatiale est modifiée est choisi parmi
un résidu d'acide aminé correspondant à ou équivalent à Glu153 numérotée selon la numérotation des acides aminés de TG2 humaine de pleine longueur basée sur l'alignement de séquences d'acides aminés, et / ou
un résidu d'acide aminé correspondant à ou équivalent à Arg19 numérotée selon la numérotation des acides aminés de TG2 humaine de pleine longueur basé sur l'alignement de séquences d'acides aminés,
et où le domaine central a une structure tridimensionnelle pliée,
iii) l'évaluation d'une propriété de liaison des autoanticorps à la protéine de référence et à la au moins une protéine d'essai,
où, si la liaison des autoanticorps à la protéine d'essai est détériorée par rapport à la protéine de référence, ce fait est considéré comme indicatif d'une maladie auto-immune induite par le gluten dans ledit sujet.

2. Le procédé de diagnostic pour le diagnostic d'une maladie auto-immune induite par le gluten chez un sujet selon la revendication 1, dans lequel, dans l'étape iii), la liaison est évaluée en mesurant le taux de liaison des autoanticorps à la protéine d'essai et à la protéine de référence,
où, si le taux moyen de liaison des autoanticorps à la protéine de référence dépasse une valeur de seuil prédéterminée et si le taux moyen de liaison des autoanticorps à la protéine d'essai est significativement inférieur, de préférence réduit d'au moins 30% par rapport à la protéine de référence, ce fait est considéré comme indicatif d'une maladie auto-immune induite par le gluten dans ledit sujet.

3. Le procédé de diagnostic selon l'une quelconque des revendications 1 ou 2, dans lequel l'une quelconque des protéines d'essai et la protéine de référence est une protéine de type sauvage ou est préparée par ingénierie des protéines à partir d'un échafaudage approprié qui est choisi parmi un animal TG1, TG2, TG3, TG4, TG5, TG6, TG7, facteur XIII ou EBP42,
où, de préférence,
a) la protéine d'essai est une transglutaminase (TG) mutante, de préférence un TG2, TG3 ou TG6 mutante, et
b) la protéine de référence est un TG de type sauvage, de préférence un TG2, TG3 ou TG6 de type sauvage.

4. Le procédé de diagnostic selon l'une quelconque des revendications 1 à 3, dans lequel, dans ladite protéine d'essai appartenant à la famille des transglutaminases, la chaîne latérale ou la position spatiale d'au moins un autre acide aminé est modifiée par rapport à la protéine de référence dans laquelle l'épitope coeliaque principal est intact.

5. Le procédé de diagnostic selon la revendication 4, dans lequel ledit autre acide aminé est choisi parmi le groupe des résidus d'acides aminés correspondant à ou étant équivalent à Arg 151, Glu 153, Glu 154, Arg156, Arg 19, His22, Val431, Arg433, Glu435, Met659 Et Leu661, numérotées selon la numérotation des acides aminés de TG2 humaine de pleine longueur basée sur l'alignement de séquences multiples.

6. Utilisation d'une protéine d'essai telle que définie dans l'une quelconque des revendications 1 à 5 dans un procédé de diagnostic de la maladie coeliaque,
ladite protéine d'essai appartenant à la famille des transglutaminases, dans laquelle la chaîne latérale et / ou la position spatiale d'au moins un résidu d'acide aminé de surface contribuant à l'épitope coeliaque principal tel que défini dans la revendication 1 est modifiée par rapport à la protéine de référence dans laquelle l'épitope coeliaque principal est intact,
où ledit au moins un résidu d'acide aminé de surface de l'épitope coeliaque principal comprend ou est choisi parmi
un résidu d'acide aminé correspondant à ou équivalent à Glu153 numérotée selon la numérotation des acides aminés de TG2 humaine de pleine longueur basée sur l'alignement de séquences d'acides aminés, et / ou
un résidu d'acide aminé correspondant à ou équivalent à Arg19 numérotée selon la numérotation des acides aminés de TG2 humaine de pleine longueur basée sur l'alignement de séquences d'acides aminés,
et où le domaine central a une structure tridimensionnelle pliée.

7. Un procédé de diagnostic pour le diagnostic d'une maladie auto-immune induite par le gluten chez un sujet comprenant les étapes de
i) fournir un échantillon biologique prélevé sur un sujet, ledit échantillon contenant des autoanticorps dudit sujet et éventuellement isoler des autoanticorps dudit échantillon,
ii) mettre en contact des autoanticorps dudit échantillon avec la protéine de référence appartenant à la famille des transglutaminases telle que définie dans la revendication 1, ladite protéine ayant un épitope coeliaque principal intact tel que défini dans la revendication 1, et
iii) évaluer le taux de liaison des autoanticorps à la protéine de référence à la fois en l'absence et en présence d'un composé d'essai, ledit composé d'essai étant un anticorps d'essai ou un fragment, un variant, un dérivé ou un analogue de celui-ci connu pour être capable de se lier à l'épitope coeliaque principal;
où, si le taux de liaison moyen des autoanticorps en l'absence du composé d'essai dépasse une valeur de seuil prédéterminée et si le taux de liaison moyen des autoanticorps à la protéine de référence en présence du composé d'essai est significativement inférieur, de préférence réduit d'au moins 50% par rapport au taux de liaison des autoanticorps en l'absence dudit anticorps d'essai, ce fait est considéré comme indicatif d'une maladie auto-immune induite par le gluten dans ledit sujet.

8. Un procédé de diagnostic pour le diagnostic d'une maladie auto-immune induite par le gluten chez un sujet comprenant les étapes de
i) fournir un échantillon biologique prélevé sur un sujet,
ii) mettre en contact un composé d'essai avec une protéine de référence appartenant à la famille des transglutaminases telle que définie dans la revendication 1, ladite protéine ayant un épitope coeliaque principal intact tel que défini dans la revendication 1 et ledit composé d'essai étant un anticorps d'essai ou un fragment, un variant, un dérivé ou un analogue connu pour être capable de se lier à l'épitope coeliaque principal,
iii) évaluer le niveau de liaison du composé d'essai à la protéine de référence à la fois en l'absence et en présence dudit échantillon biologique,
où, si le taux de liaison moyen du composé d'essai à la protéine de référence en présence de l'échantillon est inférieur au taux de liaison en l'absence de l'échantillon, ce fait est indicatif de la présence des autoanticorps capables de se lier à l'épitope coeliaque principal et d'une maladie auto-immune induite par le gluten dans ledit sujet.

9. Utilisation d'un composé d'essai connu pour être capable de se lier à l'épitope coeliaque principal d'une protéine de référence appartenant à la famille des transglutaminases telle que définie dans la revendication 1 dans une méthode de diagnostic pour le diagnostic d'une maladie auto-immune induite par le gluten chez un sujet, ledit composé d'essai étant un anticorps d'essai ou un fragment, un variant ou un analogue d'anticorps connu pour être capable de se lier à l'épitope coeliaque principal, ladite protéine de référence étant un auto antigène dans une maladie coeliaque.

10. Le procédé de diagnostic selon la revendication 7 ou 8 ou l'utilisation selon la revendication 9, dans lequel ledit composé d'essai est Mab885 ou un fragment ou dérivé de celui-ci capable de se lier à la même région d'épitope.

11. Un kit de diagnostic pour le diagnostic d'une maladie auto-immune induite par le gluten chez un sujet, ledit kit comprenant
- une protéine de référence appartenant à la famille des transglutaminases et ayant
a) une partie de la surface moléculaire d'une protéine de la famille TG à laquelle Mab885 est capable de se lier, ledit épitope comprenant une surface comprise dans un rayon de 7 Å autour de la zone couverte par Mab885 lors de sa liaison ou
b) une partie de la surface moléculaire d'une protéine de la famille TG, à laquelle un autoanticorps coeliaque d'un sujet atteint d'une maladie coeliaque est capable de se lier, d'une manière telle que ledit autoanticorps peut être déplacé dans un essai compétitif par Mab 885 ou
c) une partie de la surface moléculaire d'une protéine de la famille TG qui peut être un auto antigène dans la maladie coeliaque, formée au moins partiellement par un résidu d'acide aminé correspondant à ou équivalent à Glu153 et / ou Arg19, numérotée(s) selon la numérotation des acides aminés de TG2 humaine de pleine longueur basée sur l'alignement de séquences multiples
d) une partie d'une surface moléculaire d'une protéine de la famille TG, capable de se lier à une molécule de reconnaissance de surface, où ladite molécule de reconnaissance de surface est capable de se lier à une partie de la surface moléculaire telle que définie dans a) à c), ladite partie formant un épitope coeliaque principal intact,
- une protéine d'essai appartenant à la famille des transglutaminases,
dans laquelle la chaîne latérale et / ou la position spatiale d'au moins un résidu d'acide aminé de surface contribuant à l'épitope coeliaque principal est modifiée par rapport à la protéine de référence dans laquelle l'épitope coeliaque principal est intact,
où ledit au moins un résidu d'acide aminé de surface dont la chaîne latérale et / ou la position spatiale est modifiée est choisi parmi
un résidu d'acide aminé correspondant à ou équivalent à Glu153 numérotée selon la numérotation des acides aminés de TG2 humaine de pleine longueur basée sur l'alignement de séquences d'acides aminés, et / ou
un résidu d'acide aminé correspondant à ou équivalent à Arg19 numérotée selon la numérotation des acides aminés de TG2 humaine de pleine longueur basé sur l'alignement de séquences d'acides aminés,
et où le domaine central a une structure tridimensionnelle pliée, et
des moyens pour prendre ou traiter un échantillon biologique d'un sujet, ledit échantillon contenant des autoanticorps dudit sujet et / ou
des moyens pour isoler des autoanticorps à partir dudit échantillon, et / ou
des moyens pour évaluer le taux de liaison et / ou la cinétique des autoanticorps aux protéines.

12. Un kit de diagnostic pour le diagnostic d'une maladie auto-immune induite par le gluten dans un sujet comprenant
- une protéine de référence appartenant à la famille des transglutaminases et ayant
a) une partie de la surface moléculaire d'une protéine de la famille TG à laquelle Mab885 est capable de se lier, ledit épitope comprenant une surface comprise dans un rayon de 7 Å autour de la zone couverte par Mab885 lors de sa liaison ou
b) une partie de la surface moléculaire d'une protéine de la famille TG, à laquelle un autoanticorps coeliaque d'un sujet atteint d'une maladie coeliaque est capable de se lier, d'une manière telle que ledit autoanticorps peut être déplacé dans un essai compétitif par Mab 885 ou
c) une partie de la surface moléculaire d'une protéine de la famille TG qui peut être un auto antigène dans la maladie coeliaque, formée au moins partiellement par un résidu d'acide aminé correspondant à ou équivalent à Glu153 et / ou Arg19, numérotée(s) selon la numérotation des acides aminés de TG2 humaine de pleine longueur basée sur l'alignement de séquences multiples
d) une partie d'une surface moléculaire d'une protéine de la famille TG, capable de se lier à une molécule de reconnaissance de surface, où ladite molécule de reconnaissance de surface est capable de se lier à une partie de la surface moléculaire telle que définie dans a) à c), ladite partie formant un épitope coeliaque principal intact, dans lequel le domaine central de ladite protéine de référence a une structure tridimensionnelle pliée, et
- un composé d'essai connu pour être capable de se lier à l'épitope coeliaque principal de ladite protéine de référence appartenant à la famille des transglutaminases,
où
soit le composé d'essai est étiqueté ou
le kit comprend également un composé étiqueté liant spécifiquement au composé d'essai
pour détecter la liaison du composé d'essai à la protéine de référence, et éventuellement
des moyens pour prendre ou traiter un échantillon biologique d'un sujet, ledit échantillon contenant des autoanticorps dudit sujet et / ou
des moyens pour isoler des autoanticorps dudit échantillon.

13. Un composé thérapeutique qui est un anticorps monoclonal ou tout fragment de celui-ci comprenant une région de liaison, par example un fragment variable à chaîne unique (scFv) ou un fragment Fab, ledit composé étant un inhibiteur spécifique de la liaison d'un anticorps coeliaque à un épitope coeliaque présent sur une protéine appartenant à la famille des transglutaminases, destiné à être utilisé dans le traitement ou la prévention du début d'une maladie auto-immune induite par le gluten,
ledit composé étant capable de se lier
à une partie de l'épitope coeliaque principal qui est au moins partiellement formée par
un résidu d'acide aminé correspondant à ou équivalent à Glu153 numérotée selon la numérotation des acides aminés de TG2 humaine de pleine longueur basée sur l'alignement de séquences d'acides aminés, et
ladite partie étant une partie de la surface moléculaire d'une protéine de la famille TG à laquelle ou à une partie de laquelle Mab 885 est capable de se lier, comprenant une surface comprise dans un rayon de 7 Å autour de la zone couverte par Mab885 lors de sa liaison,
avec une avidité ou affinité de liaison suffisamment élevée pour déplacer un autoanticorps coeliaque, ledit composé étant capable d'inverser ou d'antagoniser un effet néfaste des autoanticorps coeliaques sur des cellules d'une culture de cellules ou de tissus épithéliales et
dans lequel ledit composé n'affecte ou ne modifie pas par plus de ± 50% l'activité transglutaminase de ladite protéine active et / ou dudit composé n'affecte pas ou ne modifie pas par plus de ± 50% de l'activité GTPase de ladite protéine.

14. Le composé selon la revendication 13, dans lequel ledit composé est capable d'antagoniser tout effet délétère des anticorps coeliaques sur des cellules HUVEC, une fois ajouté aux cellules HUVEC.

15. Le composé selon la revendication 13 ou 14, ledit composé étant un anticorps monoclonal, de sorte que s'il est mis en contact avec une protéine appartenant à la famille des transglutaminases, il est capable de déplacer l'autoanticorps coeliaque et ainsi d'antagoniser son effet.

16. Le composé selon la revendication 14, ledit composé étant Mab885 ou un fragment ou dérivé de celui-ci capable de se lier à la même région d'épitope.
